(19) Europäisches Patentamt
European Patent Office
Office européen des brevets

(11) EP 1 397 123 B1

(12) EUROPEAN PATENT SPECIFICATION

(45) Date of publication and mention of the grant of the patent:
**30.12.2009 Bulletin 2009/53**

(21) Application number: **02743969.4**

(22) Date of filing: **21.06.2002**

(51) Int Cl.:
***A61K 9/127*** *(2006.01)*

(86) International application number:
**PCT/NL2002/000412**

(87) International publication number:
**WO 2003/000233 (03.01.2003 Gazette 2003/01)**

# (54) DELIVERY OF SMALL HYDROPHILIC MOLECULES PACKAGED INTO LIPID VESICLES

VERABREICHUNG VON IN LIPIDVESIKELN VERPACKTEN KLEINEN HYDROPHILEN MOLEKÜLEN

LIBERATION DE PETITES MOLECULES HYDROPHILES COMPRISES DANS DES VESICULES LIPIDIQUES

(84) Designated Contracting States:
**AT BE CH CY DE DK ES FI FR GB GR IE IT LI LU MC NL PT SE TR**

(30) Priority: **21.06.2001 EP 01202401**

(43) Date of publication of application:
**17.03.2004 Bulletin 2004/12**

(73) Proprietor: **Applied NanoSystems B.V.**
**9700 BC Groningen (NL)**

(72) Inventors:
- **FRIESEN, Robert, Heinz, Edward**
  **NL-3994 NG Houten (NL)**
- **POOLMAN, Berend**
  **NL-9751 AC Haren (NL)**
- **FERINGA, Bernard Lucas**
  **NL-9765 EP Paterswolde (NL)**
- **ENGBERTS, Jan, Bernard, Frederik, Nicolaas**
  **NL-9742 EC Groningen (NL)**

(74) Representative: **Prins, Adrianus Willem et al**
**Vereenigde**
**P.O.Box 87930**
**2508 DH Den Haag (NL)**

(56) References cited:
**WO-A-99/20252 US-A- 5 820 879**

- **SIMOES S ET AL.: "Enhancement of cationic liposome-mediated gene delivery by transferrin and fusogenic peptides" PROCEEDINGS OF THE INTERNATIONAL SYMPOSIUM ON CONTROLLED RELEASE OF BIOACTIVE MATERIALS, vol. 24, 15 - 19 June 1997, pages 659-660, XP002098090 ISSN: 1022-0178**
- **HÄSE CC ET AL.: "Purification and functional reconstitution of the recombinant large mechanosensitive ion channel (MscL) of Escherichia coli." JOURNAL OF BIOLOGICAL CHEMISTRY, vol. 270, no. 31, 1995, pages 18329-18334, XP002131093 ISSN: 0021-9258**
- **HÄSE CC ET AL.: "Molecular dissection of the large mechanosensitive ion channel (MscL) of E. coli: Mutants with altered channel gating and pressure sensitivity." JOURNAL OF MEMBRANE BIOLOGY, vol. 157, no. 1, 1997, pages 17-25, XP002183679 ISSN: 0022-2631**
- **MARTINAC B: "Mechanosensitive channels in prokaryotes." CELLULAR PHYSIOLOGY AND BIOCHEMISTRY, vol. 11, no. 2, 2001, pages 61-76, XP002183680 ISSN: 1015-8987**

EP 1 397 123 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The invention relates to the field of medicine. More in particular the invention relates to the field of pharmacology.

Liposomes are typically spherical lipid bilayers from 50 nm to 1000 nm in diameter, and serve as convenient delivery vehicles for biologically active molecules. Lipid/drug aggregates are easy to form and vulnerable to structural manipulations, allowing for the adjustment of their properties for particular purposes. In selected cases, the application of liposomes in pharmacological therapy improves drug pharmacokinetics compared to its free form. The major advantages of the liposome application are: the protection of active compounds from degradation; the increase in circulation time and the possibility to achieve partial or total tissue or cell selectivity. Selectivity alone improves drug potency, eliminates side effects and allows for dosage reduction.

Although liposome mediated delivery of biologically active molecules is a very promising approach, there are also limitations associated with the current forms. A particular problem is the hydrophobic nature of the lipid bilayer. Hydrophilic drugs are typically not easily released from liposomes. In order to effectively release hydrophilic compounds, the integrity of the bilayer needs to be disrupted. Very often this is not possible in a controlled fashion.

Vesicles for targeted delivery are disclosed in WO 99/20252 A and in US 5,820,879 A.

In a more basic form the invention provides a solution to this problem by providing a mechanosensitive proteinaceous channel that in the open state allows passage of the biologically active molecule Incorporation of such a channel into the lipid vesicles allows passage of the biologically active molecule to the exterior of the lipid vesicle and thereby releasing the biologically active molecule from the vesicle. Concrete embodiments of the invention are defined in the claims. In one aspect the invention therefore provides a method for producing a lipid vesicle for modulating the bio-availability of a small hydrophilic molecule upon administration of said small hydrophilic molecule comprising producing a lipid vesicle comprising said small hydrophilic molecule and a proteinaceous channel, wherein the open state of said proteinaceous channel allows passage of said small hydrophilic molecule to the exterior of said vesicle. For use in a biological system the produced lipid vesicle can be tested for it biocompatibility and/or for the capability of the small molecule to pass through the proteinaceous channel. Often biocompatibility and capability of passage can be predicted from the properties of the various components. The lipid vesicle is preferably administered to a biological system. In this preferred embodiment, said small hydrophilic molecule comprises a molecule that is biologically active in said biological system. Vesicles produced according to the invention can be used to modulate bio-availability whether the proteinaceous channel is in the open state or in the closed state after production. The open state can for instance be used to load the vesicle with the small hydrophilic molecule. For producing the vesicle the proteinaceous channel is preferably used in the closed state or closed upon loading of the vesicle with the small hydrophilic molecule.

The invention further provides a method for making a small hydrophilic molecule bio-available comprising providing a biological system with a lipid vesicle and/or a composition according to the invention. The method is capable of providing an altered release profile for the small hydrophilic molecule compared to the absence of said proteinaceous channel. Bio-availability can further be controlled, or modulated by providing a signal for activating said proteinaceous channel to said biological system. The signal can be provided to the biological system or to a part thereof. In a preferred embodiment the signal is provided to a part of said biological system. In this way it is possible to (further) restrict or at least in part limit bio-availability of the small hydrophilic molecule to one or more parts of the biological system as a whole. Preferred signals for activating (opening) of the proteinaceous channel will be discussed elsewhere in the application.

In another embodiment the invention provides a method for obtaining controlled release of hydrophilic drugs from liposomes. To this end, the present invention in one aspect provides a method for delivering a small hydrophilic molecule to a cell, comprising loading a lipid vesicle with said small molecule and administering said vesicle to fluid that is in contact with said cell, said vesicle further comprising a proteinaceous channel, said channel in the open state allowing passage of said small molecule to the exterior of said vesicle in the vicinity of said cell. Said vesicles can be administered to culture medium of cells growing in vitro. However, preferably, said vesicle is administered to an animal or a human. More preferably, said vesicle is administered to a human. The proteinaceous channel comprises a mechanosensitive channel. Preferably, a mechanosensitive channel of large conductance (MscL) or a functional equivalent thereof.

The invention further provides a method for delivering a small hydrophilic molecule to a cell, comprising loading a lipid vesicle with said small molecule and administering said vesicle to an individual comprising said cell, said vesicle further comprising a mechanosensitive channel of large conductance (MscL) or a functional equivalent thereof, said channel in the open state allowing passage of said small molecule to the exterior of said vesicle.

In nature MscL allows bacteria to rapidly adapt to a sudden change in environmental conditions such as osmolarity. The MscL channel opens in response to increases in membrane tension, which allows for the efflux of cytoplasmic constituents. By allowing passage of said constituents to the outside of the prokaryote, it is able to reduce the damage that the sudden change in environmental conditions would have otherwise inflicted. The genes encoding MscL homologues from various prokaryotes are cloned [8]. Nucleic acid and amino acid sequences are available and

have been used to obtain heterologous (over)-expression of several MscL [8]. In the present invention lipid vesicles, preferably liposomes, comprising MscL or a functional equivalent thereof are loaded with small hydrophilic molecules whereupon loaded small molecules can be released from said vesicles under activation or opening of the channel. Loading of the lipid vesicle can be accomplished in many ways as long as the small molecules are dissolved in a hydrophilic solvent which is separated from the surrounding hydrophilic solvent by a lipid bilayer.

An advantage of a method of the invention is that the lipid vesicle can be formulated to allow preferential opening of said channel near cells of a selected tissue. Activation of MscL has been found to be controllable. It is possible to tune the type and relative amount of lipids in the vesicle such that the amount of membrane tension required to activate the channel is altered. Thus depending on the circumstances near cells of selected tissue, the lipid vesicle can be tuned to allow preferential activation of the channel and thus preferential release of said small molecule in the vicinity of said cells of said tissue.

Release near a cell or in the vicinity of cells is obtained when release is in fluid that is in contact with said cell. For instance, release into culture medium cells is for the present intended to mean release near or in the vicinity of said cells. The terms "near" or "vicinity" as used herein typically refer to a functional distance rather than a physical distance. For instance, release of a small molecule from a lipid vesicle of the invention in a capillary vessel that feeds target cells is release "near" or in the "vicinity" of said target cells. In contrast, release of said small molecule in blood-vessels that carry blood away from said target cells can be as close physically as said in the feeding vessels but are for the present invention not considered to be release near or in the vicinity of said target cells, because in this case the released small molecule is carried away from the target cells. An exception is made for lymphe and similar fluid, although these types of fluid are carried away from target cells, the contact with the surrounding cells is so intense that said small molecule can still exert effect upon release. If a physical distance is used to define the terms "near" or "in the vicinity of" said physical distance is typically not more than 100 times and preferably not more than 20 times the radius of a target cell, more preferably, said distance is not more than 10 times the radius of a target cell.

Compositions comprising lipid vesicles have been used in vivo, for instance to enable delivery of nucleic acid or anti-tumour drugs to cells. It has been observed that blood stream administration of such vesicles often leads to uptake of vesicles by cells. Uptake by cells seems to correlate with the charge of the lipid in the vesicle. Uptake is particularly a problem with negatively charged lipid vesicles, these vesicles are very quickly removed from the blood stream by the mononuclear phagocytic system (MPS) in the liver and the spleen. Although the present invention may be used to facilitate uptake of small molecules by cells, it is preferred that the small molecules are delivered to the outside of cells. In the present invention it has been found that MscL is also active in lipid vesicles that consist of positively and/or neutrally charged lipids. Lipid vesicles comprising said positively and/or neutrally charged lipids are more resistant to uptake by cells of the MPS. Lipid vesicles of the invention therefore preferably comprise positively and/or neutrally charged lipids. Such vesicles exhibit improved half-lifes in the bloodstream. Such vesicles demonstrate improved targeting to non-MPS cells. The lipid part of a lipid vesicle of the invention directed toward the exterior preferably consists predominantly of positively and/or neutrally charged lipids, thereby nearly completely avoiding cellular uptake through negatively charged lipid and thereby further increasing the bloodstream half life of lipid vesicles of the invention. Apart from increasing the half life of the vesicle in the blood stream, positively and/or neutrally charged lipids can also be used to alter the amount of added pressure needed to activate the channel in the vesicle. Vesicles of the invention wherein the outwardly directed lipid part of a lipid vesicle consists predominantly of positively and/or neutrally charged lipids, postpone the rapid cellular uptake as seen for vesicles wherein the outwardly directed part consists of negatively charged lipid. Postponed uptake through the MPS system leads to increased circulation times. Apart from this, positively and/or negatively charged lipids can also be used to alter the amount of membrane tension needed to activate the channel.

The signal or event leading to activation of a channel of the invention can also be changed by altering the MscL in the vesicle. Besides the pH sensitive mutants, other mutants are available that have a higher open probability as compared to the wild type MscL from *Escherichia coli* [11, 14] This property can be used to tune the activation potential of the channel in a method or composition of the invention. For instance, it is known that in tumours the pH is very often considerably lower than in the normal tissue surrounding the tumour. Other areas in the body that have a lowered pH are the liver, area's of inflammation and ischemic area's. A lower pH can be used as a trigger for activation of the MscL in a vesicle of the invention. Mutant MscL are available that activate (open) in response to a pH that is frequent encountered in tumours. One non-limiting example of such a pH-sensitive mutant is the G22H mutant. This mutant exhibits a higher open probability a (low) pH values that are frequently encountered in tumours, as compared to normal pH values of circulating blood [9]. Thus in a preferred embodiment of a method or composition of the invention, said mutant allows preferred release of said small molecule in said target tissue.

A small molecule can be any hydrophilic molecule small enough to pass through the pore of a channel of the invention. Preferably said small molecule comprises a diameter of no more than 6 nm (60 Å), more preferably no more than 5 nm (50 Å) and still more preferably no more than 4 nm (40 Å.) Particularly peptides are preferred for the present invention. Peptides typically have very poor pharmacodynamic properties when injected into the bloodstream. With the present invention it is possible to significantly increase the half life of peptides in the circulation. Moreover, by enabling

controlled release of a small molecule with a vesicle of the invention it is also possible to have locally a relatively high bio-availability of the peptide, whereas systemically the bio-availability is low or even absent. This also allows for the therapeutic use of molecules that are otherwise too toxic when bio-available systemically.

Controlled and/or localised release of a small molecule can be achieved in many ways. By, for instance, tuning of the composition of the lipid vesicle and/or the use of a mutant MscL it is possible to control how and where release of the small molecule will occur. In a preferred embodiment, activation of said channel is triggered upon the availability of a signal. In a preferred embodiment said signal comprises light, pH, a chemical compound or temperature. Various chemical compounds can be used for this purpose as long as they induce opening of the proteinaceous channel. In a preferred embodiment opening is induced by providing the chemical to a part of the biological system. Non-limiting examples of suitable chemical compounds are for instance, compounds that influence the pH of the environment. Another, non-limiting example is a compound capable of interacting or reacting with the proteinaceous channel leading to an altered open-probability. Preferably, but not necessarily changing the gating properties to a property wherein the channel is open under the conditions that the channel is experiencing. In one embodiment said chemical compound comprises an MTS molecule. Preferably, said chemical compound comprises reduced glutathione.

The signal for activation can for instance be exposure of the vesicle to a certain pH, to light or to a certain temperature. Exposure to the signal can directly or indirectly (through an intermediary signal) lead to the activation of the channel. Preferably, said signal comprises light. There are hydrophobic compounds such as azobenzene phospholipids and related compounds available [10], that mix with the lipids in the vesicle, and that upon exposure to light undergo a structural change such that the gating of the MscL channel can be controlled. It is also possible to insert a photosenstive mutant MscL in the lipid vesicle. Upon exposure to light, a photoreactive molecule conjugated to a specific site of the MscL protein alters conformation thereby controlling the gating of the MscL channel. Activation through light is just one example of an embodiment wherein opening/activation of the channel can be induced by another signal than membrane tension. An alteration in the redox-potential is another non-limiting example. MscL can be made sensitive to the local redox-potential after conjugation of a redox-sensitive molecule, such as a nicotinamide adenine dinucleotide derivative, to a specific site of the MscL protein. Such a redox-sensitive MscL can be (de)activated by changing the redox-potential of the environment. In yet another embodiment said signal comprises an altered pH, preferably said pH is equal or less then 6.5. Recognition of only the open conformation of MscL by an antibody is another non-limiting example of an embodiment that gating of the channel can be induced by another signal than membrane tension. Such an antibody can for instance be used to preferentially increase the open probability of the channel near target cells. A bi-specific antibody comprising the above mentioned specificity for the open state and a specificity for a target cell can be used to accumulate open vesicles near target cells. Another example of a signal that triggers activation of a MscL is local anaesthetics [13]. Local anaesthetics most probably work to activate the channel through their incorporation in the lipid bilayer, which changes the bilayer properties.

There are very likely many substances that can cause activation of the channels. One example to note is this context is [2-(trimethylammonium)ethyl]methanethiosulfonate bromide (MTSES). This compound is capable of associating with MscL mutant G22C. Whereas a hydrophobic moiety at this position makes the channel harder to open, a hydrophilic addition at this position helps to overcome the mechanical work required to open the channel. The compound MTSES helps to lower the amount of signal required to activate the channel (16). Various polar and nonpolar variants of MTSES exist that can be used depending on whether the channel should be easier or more difficult to activate. It is also possible and for some applications even preferred to change the signal needed for activation of the channel from membrane pressure to another signal. Signals such as light, local anaesthetics, pH, temperature, etc., can be used to facilitate the local delivery of an incorporated small molecule. For instance, through local illumination a circulating lipid vesicle can be triggered to release incorporated molecules only in the illuminated area of the body. This is an important additional advantage of having another signal or an intermediate signal than pressure for activation of the channel. In a preferred embodiment a vesicle of the invention comprises an asymmetrical bilayer. An asymmetrical bilayer is yet another example of a method to tune the lipid vesicle such that the activation of the channel is altered. It seems that the force gating MscL is from the lipid bilayer and amphipaths probably generate this force by differential insertion into the two leaflets [13]. In a preferred embodiment, a signal required for activation is provided through an intermediate. The intermediate is here capable of transforming the given signal into a pressure signal thereby allowing, if sufficient, the opening of the channel

Vesicles and/or composition of the invention containing pH-sensitive proteinaceous channels, may be used for pH-induced drug release. In tumors and sites of inflammation, the pH of the interstitial fluid is reduced whereas the blood flow is increased and the vasculature 'leaky'. pH-sensitive liposomes have been developed for these purposes [Shi, J Contr. Release 2002;80:309, Drummond, Biochem. Biophys 2000;1463:383]. The advantage of our pH-sensitive proteinaceous channels is the instant drug release (within seconds) that can be achieved. In the lung, the pH of the airway surface liquid is reduced in inherited and acquired diseases such as cystic fibrosis and asthma as a result of lung obstruction, infection and inflammation [Coakley, J. Pancreas 2001;2:294]. Since not all lobs of the lung are affected at the same time, pH-sensitive drug release may improve the therapeutic index of a drug adminstered by inhalation. To

benefit from (patho)physiological changes of the ASL pH to improve inhalation therapy has not been exploited before.

Since cellular uptake of liposomes generally follows an endocytotic pathway, pH-sensitivity might have a potential application in the delivery of drugs and genes from the endosomes into the cytosol [Straubinger, Methods Enzymol 1993;221:361].

pH-sensitive formulations of the invention may also be used for release of orally taken drugs in the gastrointestinal tract. The highly acid (less than pH 2) content of the stomach is neutralized in the first segment of the small intestine by the pancreatic fluid. Subsequently, along the small and large intestines, the pH drops to pH 6.4 in the caecum and rises again to neutral pH till the end. To delay the release of drugs in the gastrointestinal tract, dosage forms have been designed which dissolve at pH 7 or above [Friend, Aliment Pharmacol Ther 1998;12:591]. However, for the treatment of active colitis, characterized by a low pH at the site of inflammation, a drug should pass the acid environment of the stomach inactive with a subsequent activation at the site with lowest pH. For this purpose, our liposomes with pH-sensitive channels may be coated with a coating that is resistant in the stomach but effectively degraded by the enzymes in the small intestines (like several disaccharides).

Liposomes containing osmo-sensitive protein channels, may be used for osmo-induced drug release. We use of osmotic sensitive liposomes to release drugs in the small intestine from stomach resistant capsules.

Liposomes containing light-sensitive protein channels, may be used for light-induced drug release. Light induced drug release is useful for patient-controlled drug therapy such as analgesia for pain treatment and insulin for treatment of diabetes. At the moment, only invasive patient-controlled systems are available for these purposes.

In one aspect the invention provides a lipid vesicle obtainable by a method of the invention. Preferably, said vesicle comprises a biologically active molecule. The invention further provides a composition for making a small hydrophilic molecule biologically available comprising a lipid vesicle obtainable by a method of the invention. Preferably, said composition is formulated and prepared for human use. The invention further provides a composition comprising a lipid vesicle comprising a proteinaceous channel and a small hydrophilic molecule, wherein said lipid vesicle and/or said proteinaceous channel is formulated such that said proteinaceous channel is in the open state in the vicinity of a target cell. The open state may be arrived at by supplying the channel in open state, by enabling, through said formulation, the opening of said channel in the vicinity of the target cell and/or by providing a signal enabling opening of said channel. Preferably, said proteinaceous channel comprises an MscL or functional part, derivative and/or analogue thereof. In a preferred aspect the invention provides a composition comprising a lipid vesicle comprising an MscL or functional part, derivative and/or analogue thereof, wherein said composition is formulated and prepared for use in a human. Preferably said lipid vesicle comprises a small hydrophilic molecule capable of passing through an activated MscL. Preferably, said composition is used in the preparation of a medicament. Preferably said small molecule is intended to be delivered to the outside of a cell in said tissue. A composition as described is of course ideally suited to be used in a method of the invention. Preferably, said MscL is a mutant MscL or a functional part, derivative and/or analogue thereof. A functional part of MscL comprises at least the region that in *E.coli* comprises residue 4 to 110 [11]. It is possible to generate MscL proteins that comprise amino-acid substitution(s), insertion(s) and/or deletion(s) compared to the protein found in bacteria. Such derivatives can of course also be used for the present invention provided that the derivative is functional, i.e. comprises the channel activity in kind, not necessarily in amount. The channel activity may, as will be apparent from the description, be triggerably by means other than pressure. With activity in kind is therefor not meant the type of triggering but the channeling activity, the capability of the protein to allow passage of a hydrophilic substance from one side of the lipid obstruction to the other. The amount of activity, both in the amount of small molecules that may pass per time unit, or the size of the pore through which the small molecule can pass may differ. A derivative of MscL is also an MscL that comprise more or less or different (post-translational) modifications as compared to the native protein. Other options with mutant or derivative channels would be using MscL with genetically engineered changes in the outside loop, like receptor recognising domains (e.g. RGD) that upon binding with the receptor undergo conformation changes that induce opening of the channel. One may also add (part of) an antibody to that loop that induces channel opening after ligand binding. An MscL analogue is a molecule comprising the same activity in kind to allow passage of hydrophilic molecules through a lipid obstruction than MscL itself, not necessarily in amount.

In another aspect the invention provides a method for generating a vehicle for delivery of a small hydrophilic molecule to a cell, said method comprising generating in an aqueous fluid, a lipid vesicle comprising a proteinaceous channel, said vehicle formulated such that said proteinaceous channel is in the open state in the vicinity of said cell. Preferably, said proteinaceous channel assumes said open state upon entering the vicinity of said cell. More preferably, said lipid vesicle further comprises said small molecule. A method for the generation of a vehicle as described above can be advantageously used to generate a composition of the invention.

In a preferred embodiment a lipid vesicle of the invention further comprises a non-channel protein. Preferably, said non-channel protein is a binding molecule capable of binding to a binding partner in said tissue thereby enabling at least a prolonged stay of said vesicle in said tissue and/or near a target cell.

In another aspect the invention provides the use of a lipid vesicle comprising an MscL for controlling delivery of a small hydrophilic molecule to a target tissue in a body.

A lipid vesicle of the invention may be used to deliver a small molecule to any part of the body. However, preferably it is used to deliver to tissue with permeable endothelium such as the liver, the spleen area's of inflammation or tumour bearing tissues.

A lipid vesicle can comprise lipid but may also comprise other molecules. Glycolipids or lipids modified in other ways that maintain the classical bipolarity of a lipid molecule in kind, not necessarily in amount are also called lipids in the present invention. In a preferred embodiment of the invention said lipid vesicle comprises a liposome, more preferably a long circulating liposome. Long circulating liposomes are typically small (150 nm or smaller), neutral and have a specific composition (cholesterol-containing with either phosphatidylcholine and PEG or sphingomyelin etc).

Considering that MscL is typically a foreign protein it is conceivable that upon repeated administration an immune response is mounted by the host. To allow at least a partly evasion of the immune system of the host so called masking groups can be attached to the outside of the lipid vesicle. Preferably, said masking groups comprise PEG.

In yet another embodiment the invention provides a use of a lipid vesicle or a composition according to the invention for delivering a small hydrophilic molecule to a biological system for a non-medical purpose. The invention further provides the use of a lipid vesicle or a composition of the invention for the preparation of a medicament. Further provided is the use of a mechanosensitive channel for modulating the bio-availability of a small hydrophilic molecule packaged in a lipid vesicle.

Non-limiting examples of small molecules that may advantageously be used in a lipid vesicle of the invention are :

- *Interleukins*: peptides and proteins that modulate the immune response
- *Diphteria toxin (fragment):* potent inhibitor of protein synthesis in human cells
- *Muramyl dipeptide*: activator of immune system; macrophage-mediated destruction of tumor cells
- *Cis-4-hydroxyproline:* potential treatment for lung fibrosis
- *Cisplatin (derivatives)*: cancer treatment
- *Cytosine arabinose*: cancer treatment
- *Phosphonopeptides*: antibacterial agent
- β-*Glucuronidase*: activator of prodrugs (e.g., epirubicin-glucuronide) -*Cytostatic drugs (doxorubicin,ciplatin etc.)*
- *Small therapeutic proteins/peptides (interleukins, insuline, growth factors, chemokines)*

Bio-availability of a small molecule can be achieved in various ways. Typically the molecule is provided to the biological system where it is to be made available. However, the reverse may also be true, in that the molecule is first provided. In this situation the biological system is provided later. The purpose of the latter situation can for instance be to at least in part prevent further development of the biological system, as for example in a decontamination setting.

As used herein, altering the open-probability of a proteinaceous channel refers to the shifting of the equilibrium of the open/closed state of the proteinaceous channel such that the equilibrium lies more to the open state or more to the closed state at the conditions used.

Examples

**Example 1**

MATERIAL AND METHODS

MscL Expression and Purification

*E.coli* PB104 cells containing the plasmid pB104 carrying the MscL-6His construct was grown to mid-logarithmic phase in Luria Bertani medium (10L fermentor) and induced for 4 h with 0.8 mM IPTG [3]. Cells were French-pressed and membranes were isolated by differential centrifugation, as previously described [1]. The membrane pellet (5-8 g wet weight) was solubilized in 100 mL of buffer A (50 mM $Na_2HPO_4.NaH_2PO_4$, 300 mM NaCl, 10 mM imidazole) containing 3% n-octyl β-glucoside. The extract was cleared by centrifugation at 120 000 x g for 35 min, mixed with 4 mL (bed volume) $Ni^{2+}$-NTA agarose beads (Qiagen, Chatworth, CA) equilibrated with buffer A and gently rotated for 15 min (batch loading). The column material was poured into a Bio-Spin column (Bio-Rad) and washed with 10 column volumes of buffer B (as buffer A, except 1% n-octyl β-glucoside) followed by 5 column volumes of the buffer B but with 100 mM imidazole. The protein was eluted with buffer B but with 300 mM imidazole. Eluted protein samples were analysed by fractionation on a SDS-15 % polyacrylamide gel followed by staining with Coomassie Blue or transferring the fractionated proteins to PVDF membranes by semi-dry electrophoretic blotting for immunodetection with a anti-His antibody (Amerham Pharmacia Biotech). Immunodetection was performed with an alkaline phosphatase conjugated secondary antibody as recommended by the manufacturer (Sigma).

Electrospray Ionization Mass Spectrometry of Detergent Solubilized MscL proteins.

Purified detergent solubilized G22C-MscL-6His was heated to 60 ˚C for 15' and precipitated protein was spun down at 14 000 rpm in a tabletop centrifuge (Eppendorf) for 5 min. The pellet was dissolved in 50% formic acid and 50% acetonitril just before electrospray ionization mass spectrometry (ESI-MS) analysis. The average molecular masses of the proteins were calculated from the m/z peaks in the charge distribution profiles of the multiple charged ions. Spectral deconvolution was performed on the peaks over the mass range from 800 to 1700 using the computer program MacSpec (Sciex). All molecular masses quoted in this paper are average, chemical atomic masses.

2-Sulfonatoethyl methanethiosulfonate Labeling of G22C-MscL-6His.

The single cysteine mutant, G22C-MscL-6His, was labeled with (2-sulfonatoethyl)methanethiosulfonate (MTSES). A suspension of 20-30 μM of G22C-MscL-6His in buffer B with 300 mM imidazole ( 0.5 mL final volume), was incubated with 0.6 mM MTSES at 4 ˚C for 30 min. Conjugation was monitored employing ESI-MS.

Membrane Reconstitution of 6His-MscL

Dry lipid mixtures were prepared by co-dissolving lipids (Avanti Polar Lipids, Alabaster, AL) in chloroform, in weight-fractions as indicated in the experiments, and removing the chloroform by evaporation under vacuum for 4 h. All acyl chains of the synthetic lipids were of the type, dioleoyl, unless indicated otherwise.The dried lipid film was dissolved (20 mg/mL) in 50 mM potassium phosphate, pH 7.0, followed by three freeze/thaw cycles. An aliquot, 200 μL of the rehydrated liposomes and 5% n-octyl p-glucoside, was added to 200 μL purified 6His-MscL. Final protein-to-lipid molar ratio as indicated in the experiments. Subsequent membrane reconstitution was achieved by exhaustive dialysis into a buffer containing 0.1 mM $Na_2HPO_4.NaH_2PO_4$ pH 6.8 containing detergent-absorbing Bio-Beads SM-2 (Bio-Rad, Inc.).

Sucrose Gradient Centrifugation.

Discontinuous sucrose gradients were employed to analyze membrane reconstituted 6His-MscL as described elsewere [6].

Freeze-Fracture Electron Microscopy.

Freeze-fracture electron microscopy of membrane-reconstituted 6His-MscL were performed as described elsewere [7].

Electrophysiologic Characterization of Membrane-Reconstituted MscL.

MscL was reconstituted into liposomes of different lipid composition and aliquots of 200 μL were centrifuged at 48 000 rpm in a tabletop ultracentrifuge (Beckmann). Pelleted proteoliposomes were resuspended into 40 μL buffer C (10 mM 4-morpholinepropanesulfonic acid (MOPS)-buffer, 5% ethylene glycol, pH 7.2), and 20 μL droplets were subjected to dehydration-rehydration cycle on glass slides [2]. Rehydrated proteoliposomes were analysed employing patch-clamp experiments as described previously [3].

In vitro Release Profiles of a Model Drug from Proteoliposomes

The percentage release of a fluorescent model drug, calcein, from MscL-containing liposomes was calculated from the dequenching of calcein fluorescence according to the following equation:

$$\% \text{ Release} = \frac{F_x - F_0}{F_t - F_0} \times 100$$

Where $F_0$ is the fluorescence intensity at zero time incubation, $F_x$ is the fluorescence at the given incubation time-points and $F_t$ is the total fluorescence, obtained after Triton X-100 lysis. Fluoresence was monitored with a SLM 500 spectrofluorimeter in a thermostatted cuvette (1 mL) at 37 ˚C, under constant stirring. Excitation and emission wavelengths were, respectively, 490 (slit 2 nm) and 520 nm (slit 4 nm). The experiments were performed at lipid concentrations of approximately 50 μM. Control, and MscL-containing liposomes were prepared as described above followed by mixing with an equal volume of 200 mM calcein in PBS buffer. Then a freeze-thaw cycle has been repeated three times followed by extrusion through a 100 nm polycarbonate membrane [12]. The liposomes were separated from free calcein by using Sephadex 50 column chromatography equilibrated with PBS (160 mM NaCl, 3.2 mM KCl, 1.8 mM $KH_2PO_4$, 0.12 mM $Na_2HPO_4$, 1.2 mM EGTA, pH 8.0), which was isotonic to the calcein-containing buffer.

Results and discussion

Overexpression and purification of the MscL channel protein.

Since the expression level of His-MscL in *E. coli* was relatively low, based on the absence of a significant IPTG-inducible band on a SDS-PAGE, attention was focused on obtaining a high biomass during fermentation and a high yield after protein purification.

The His-tagged MscL could be purified to apparent homogeneity in a single step using nickel chelate affinity chromatography as shown by SDS-PAGE (Fig. 1, lane B). The yield of this eluted His-tagged MscL was ± 2 mg per Liter of cell-culture with an estimated purity of >98% based on analysis using SDS-PAGE and Coomassie Brilliant Blue staining.

The rate of excretion via MscL of small molecules is > 10 000 nmol/sec. x mg of cell protein, i.e. when the protein is in the open state. Since the expression level of mscL in wild-type bacteria is 4-10 functional units per cell and the MscL channel is a homopentamer of 15 000 Da, it can be concluded that the flux via a functional MscL channel is > $10^6$ x $s^{-1}$. This activity of MscL is such that on average 5 molecules of pentameric MscL per liposome with a diameter of 400 nm should suffice. Such a liposome contains approximately 1.67 x $10^6$ molecules of lipid; the molar ratio of lipid over MscL will thus be 0.67 x $10^5$. Consequently, 2 mg of MscL will yield 6 g of proteoliposomes.

Electrospray Ionization Mass Spectrometry of Detergent Solubilized MscL proteins.

ESI-MS is an accurate and effective method to verify primary sequences of the 6His-MscL protein and the stoichiometry of conjugation reactions. Figure 2 shows the ESI-MS spectra of the G22C-MscL-6His and the MTSES conjugated G22C-MscL-6His samples.

Based on the deduced amino acids, the average molecular weight of G22C-MscL-6His is 15 826 Da. ESI-MS analysis of G22C-MscL-6His resulted in a molecular weight of 15 697 Da, which corresponds to the deduced molecular weight minus a methionine. This observation would be consistent with an excision of the N-terminal methionine as reported for many proteins expressed in *E. coli* [5]. ESI-MS analysis of the MTSES conjugated G22C-MscL-6His resulted in a molecular weight of 15 837 Da, which corresponds exactly with the calculated mass increase of the MTSES conjugation. ESI-MS analysis is routinely used to verify the average masses of MscL mutants and the products of conjugation reactions.

Membrane reconstitution into liposomes of different lipid compositions.

Purified detergent-solubilized MscL was reconstituted into preformed liposomes, which were titrated with low amounts of detergent. After removal of the detergent by adsorption onto polystyrene beads, proteoliposomes were formed. The proteoliposomes were characterized by equilibrium sedimentation on a sucrose gradient as shown in figure 3. All 6His-MscL protein detected by the Western blot (inset in Fig. 3) was associated with the lipid bilayer as detected by octadecylrhodamine-β-chloride ($R_{18}$) fluorescence.

Association of the 6His-MscL protein with the liposomes does not necessarily mean the protein is inserted correctly into the lipd bilayer. Correctly inserted MscL protein should be trans-membrane, and show up as an intra-membrane vesicle (IMP) in a freeze-fracture image as shown in the white boxed area of figure 4.

The equilibrium sedimentation and freeze-fracture electron microscopy experiments provided structural evidence for the correct reconstitution of the 6His-MscL protein into lipid bilayers.

Electrophysiologic characterization of membrane-reconstituted MscL activity.

The purified protein reconstituted into phospholipid liposomes forms functional mechanosensitive channels, as seen from the traces in Fig. 5 at different pipette pressures (mechanical activation). The MscL open probability plotted against pressure can be fitted with a Boltzmann distribution (Fig. 6).

Interestingly, reconstituted MscL is active in the absence of negatively charged lipid headgroups (Fig. 5, PC:

PE 70:30)). This is a very important finding since negatively charged headgroups prevent targeting to most target sites in the human body. Additionally, these experiments have shown for the first time that the pressure treshold is significantly effected by the lipid composition of the membrane reconstituted MscL channels (Fig. 6). This allows tailor making of the drug release profiles to the specific neads.

We have constructed several 6His-MscL mutants with altered gating properties, e.i.; mutants that are hypersensitive to membrane tension, and mutants with increased open probability at lower pH-values.

In vitro release profiles of a model drug.

The fluorescence efflux-assay was developed to monitor the MscL-mediated release profiles.. Liposomes (DOPC:Chol, 60:40, m/m) with and without MscL-6His were subjected to an osmotic downshock, thereby effectively increasing the membrane tension, to monitor the calcein release. As shown in Fig. 7, less calcein remained in the liposomes containing MscL (closed circles) relative to the liposomes without MscL (closed squares) when change in osmolality was larger than 200 mOsm. These data demonstrate that, upon osmotic downshock, liposomes containing reconstituted MscL exhibit a greater efflux of calcein than liposomes without MscL. This MscL-mediated efflux is consistent with the electro-physiologic analysis, showing that the MscL is reconstituted into membranes of synthetic lipids while retaining its functional properties.

For controlled release of drugs at the target site, membrane tension may not be the most promising stimulus since little is known about osmotic differences in the human body. Several alternatives to activate the MscL channel at the target site are described in this patent. Introducing a charge through conjugation of MTSES to cysteine at position 22 serves as an example for channel activation under iso-osmotic conditions. ESI-MS analysis of the MTSES conjugated G22C-MscL-6His protein showed that all MscL monomers were conjugated to a stoichiometry of 1:1. The conjugated G22C-MscL-6His samples was subsequently reconstituted into liposomes as described above and calcein release was measured as shown in Fig. 8. These data demonstrate that, in the absence of an increased membrane tension, MscL exhibits drug release from drug laden synthetic liposomes. This patent includes MscL conjugates that will release drugs at the target site as a function of pH, light activation and specific interactions with target associated molecules.

The integrity of liposomes consisting of phosphatidylcholine is seriously affected at first contact with a biological milieu following intravenous injection [15]. The integrity of liposomes (PC:Chol:DGPE-PEG, 55:40:5, m/m/m) was studied as a function of the molecular mass of the PEG group attached to the DGPE lipid in the presence of rat and human plasma at 37 ˚C. Calcein release from only the liposomes without DGPE-PEG and with 5 ml% DGPE-PEG(2000) are shown in Fig. 9 (not being part of the invention). These data show that addition of 5 ml% of DGPE-PEG(2000) significantly increase liposomal integrity in rat and human plasma up to several hours and will serve to prevent drug leakage during the traveling time to the target cells.

## Example 2

Title: Delivery of a substance from liposomes through a charge induced channel opening

There are very likely many substances that can cause activation of the MscL channel. One example in this context is a group of compounds that are capable of associating with MscL mutant G22C (16). Attachment of these reagents that are positively charged ([2-(Trimethylammonium)ethyl] methanethiosulfonate) (MTSET) and (2-aminoethyl methanethiosulfonate) (MTSEA) or negatively charged (sodium (2-sulfonatoethyl) methane thiosulfonate) (MTSES) to the cysteine under patch clamp causes MscL to gate spontaneously, even when no tension is applied (16). These results indicate that chemically charging the pore constriction at amino acid position 22 opens the MscL channel.

In literature, experiments were performed on spheroplasts containing the MscL mutant G22C in its natural environment, including a wide variety of lipidic and proteinaceous molecules. It is relevant to show that the methanethiosulfonate compounds attach specifically to the MscL mutant and this charged induced gating occurs in artificial lipid membranes without the involvement of other cellular or membrane components.

## Example 2A

Example 1, Fig. 2, shows that a methanethiosulfonate compound covalently attaches to the MscL mutant G22C in a one-to-one stoichiometry. In this example we show the effect of MTSET attachment to MscL mutant G22C on the pressure sensitivity of the channel and the change in preference for specific conductance states under patch clamp conditions.

Materials and Methods

MscL mutant G22C containing six C-terminal histidine residues was constructed using standard molecular biology techniques. Expression, purification, membrane reconstitution, and patch clamp analysis were performed as described in example 1 or as described below.

MscL Expression and Purification. *E.coli* PB104 cells containing the plasmid pB104 carrying the MscL-6His construct was grown to early-logarithmic phase in Enriched medium (Yeast extract 150 g/l, Bactotrypton 100 g/l, NaCl 50 g/l, $K_2HPO_4$ 25 g/l, $KH_2PO_4$ 25 g/l, Antifoam A 2 ml/l, after sterilization add 1.5 g Amp 10 ml 1000x $Fe^{+2}$ stock ($Fe^{+2}$ stock: 0.278 gr $FeSO_4$. $7H_2O$ in 100 ml 1N HCl) and 10 ml 1000x spore-elements stock (Spore-elements per 100 ml: EDTA 1 gr, $ZnSO_4$. $7H_2O$ 29 mg, $MnCl_2$ . $4H_2O$ 98 mg, $CoCl_2$ . $6H_2O$ 254 mg, $CuCl_2$ 13.4 mg, $CaCl_2$ 147 mg, pH 4 with NaOH) (15 L fermentor) and induced for 4 h with 1.0 mM IPTG [3]. Cells were French-pressed and membranes were isolated by differential centrifugation, as previously described [1]. The membrane pellet (2.4 g wet weight) was solubilized in 30 ml of buffer A (50 mM $K_2HPO_4$.$KH_2PO_4$ pH 8.0, 300 mM NaCl, 35 mM imidazole, pH 8.0, 3% n-octyl β-glucoside). The extract was cleared by centrifugation at 120,000 x g for 35 min., mixed with 4 ml (bed volume) $Ni^{2+}$-NTA agarose beads (Qiagen, Chatworth, CA) equilibrated with wash buffer (300 mM NaCl, 50 mM $K_2HPO_4$.$KH_2PO_4$ pH 8.0, 35 mM imidazole pH 8.0, 1% n-octyl β-glucoside) and gently rotated for 30 min. at 4 ˚C (batch loading). The column material was poured into a Bio-Spin column (Bio-Rad) and washed with 25 ml of wash buffer, with 0.5 mL/min. flow rate. The protein was eluted with wash buffer containing 235 mM imidazole. Eluted protein samples were analyzed by fractionation on a SDS-15 % polyacrylamide gel followed by staining with Coomassie Brilliant Blue or transferring the fractionated proteins to PVDF membranes by semi-dry electrophoretic blotting for immunodetection with a anti-His antibody (Amerham Pharmacia Biotech). Immunodetection was performed with an alkaline phosphatase conjugated secondary antibody as recommended by the manufacturer (Sigma).

Electrophysiologic characterization of membrane-reconstituted MscL. MscL was reconstituted into liposomes of different lipid composition and aliquots of 200 μL were centrifuged at 70,000 rpm in a tabletop ultracentrifuge (Beckmann). Pelleted proteoliposomes were resuspended into 30 μL buffer C (10 mM 4-morpholinepropanesulfonic acid (MOPS)-buffer, 5% ethylene glycol, pH 7.2), and 15 μL droplets were subjected to dehydration-rehydration cycle on glass slides [2]. Rehydrated proteoliposomes were analysed employing patch-clamp experiments as described previously [3].

Giant spheroplasts were prepared as explained before (Blount, P. et al., Methods Enzymol. Vol. 294:458-482, 1999).

Results and Discussion

The electrophysiologic characterization of the MscL mutant G22C as shown in Fig. 10A and C resulted in similar channel properties as described in literature with respect to pressure sensitivity and a conductance of 3.42 nS. The other observed conductances are from MscS (1.6 nS), another mechanosensitive channel in spheroplast, and most probably a simultaneous opening of the MscL mutant G22C and MscS (5.0 nS). However, when MTSET is attached to MscL mutant G22C, channel properties change significantly as shown in Fig. 10B and D. Firstly, pressure sensitivity decreases significantly resulting in spontaneous gating of the channel. Secondly, When the MscL channel opens, it closes much faster, smaller dwell times. It appears as if the introduced charge at amino acid position 22 destabilizes both the closed and open state. As a consequence, the channel rapidly switches from the closed to open state resulting in this "flickery" appearance even in the absence of membrane tension.

**Example 2B**

Example 1 and 2A showed that specific attachment of MTSET to MscL mutant G22C in spheroplasts results in spontaneous gating of the channel. Patch clamp also showed that the channel opening is exhibiting much smaller dwell times compared to unlabeled channel proteins. In this example we show that after MscL purification and membrane reconstitution into an artificial lipid membrane, attachment of MTSET, MTSEA, or MTSES to MscL mutant G22C results in spontaneous gating. Additionally, we show that the charge induced channel opening, can result in the release of a membrane impermeable hydrophilic molecule from artificial liposomes containing MscL mutant G22C upon the introduction of a charge by means of a MTS compound.

Materials and Methods

Samples were prepared, and calcein efflux was monitored as described in example 1 and 2A.

Results and Discussion

Increase in MscL mediated calcein release upon attachment of MTSET, MTSEA or MTSES to MscL mutant G22C is shown in Fig. 11. MscL mutant G22C reconstituted into DOPC:DOPS (90:10, mol/mol) liposomes showed no calcein release at the time scale of this experiment as indicated by the stable fluorescence in the first 85 sec. of the experiment. At 85 sec., 1 mM MTSET was added to the sample and calcein was rapidly released. In control liposomes, same lipid composition but without MscL, no calcein release was observed (data not shown). This experiment shows that when formulated as described, 80 percent of the encapsulated calcein is effectively released from these liposomes, and half of the MscL mediated efflux occurs within 7 seconds. This experiment was repeated using another positively charged compounds MTSEA, and a negatively charged compound MTSES. Calcein is released with both these compounds, however, the kinetics of release are significantly slower. The release of calcein upon the addition of these compounds is a composite of the reactivity of the MTS compounds with cysteine at position 22, opening of the MscL channel in response to the introduced charge and the efflux of calcein through the MscL channel. The difference in calcein release can be best explained by the difference in hydrophobicity between the MTS compounds, with MTSET being the most hydrophilic and therefore resulting in the fastest cysteine reactivity leading to the fastest calcein release.

These results establish the correlation between the gating properties as determined with patch clamp and the release profile of a hydrophilic molecule from liposomes. It can be concluded that even when a channel exhibits short dwell times, hydrophilic molecules of approximately 600 Da can be effectively and rapidly released under control of charging amino acid at position 22 of the MscL channel.

These results show that liposomes with MscL mutant G22C can be used in a two-component system. In this, the first component, liposomes with MscL mutant G22C with encapsulated drug, are administered. After these liposomes have accumulated at a target site, a second component is administered. This second component, MTS compound or similar in that it attaches specifically to the cysteine at position 22, will then effectively cause the release of the encapsulated drugs. By using second components of different hydrophobicities allows tailor making of the drug release profiles as is shown in Fig. 11.

Example 3

Title: Formulation of liposomes containing MscL channel proteins with an encapsulated substance.

Employing MscL channel proteins for sustained and controlled release of substances is shown in examples 1, 2, 4, 6, and 8. For its application, however, it is important to establish a relative simple procedure to formulate the delivery vehicles. This example shows that mixing synthetic lipids, detergent, MscL channel protein, and the substance that needs to be delivered, followed by detergent removal results in a functional controllable drug delivery vehicle. Additionally, depending on the clinical application, specific lipid compositions of the liposomal drug delivery vehicle are required. This example shows that the MscL mediated controlled release of a substance can be achieved in liposomes composed of different lipid compositions.

**Materials and Methods**

MscL mutant G22C was overexpressed, and purified as described in example 2. Membrane reconstitution was started by mixing 200μL of 20 mg/mL of preformed liposomes (DOPC:Cholesterol, 80:20, mol/mol), 500 μL of 0.4 mg/mL purified MscL mutant G22C, 12 mg n-octyl β-glucoside, and 700 μL of a calcein loading buffer, containing 200 mM calcein, 300 mM sucrose, 25 mM Tris, and 1 mM EDTA, pH 8.0. Incubate 30 min. at room temperature and add 40 mg of detergent-absorbing Bio-Beads SM-2 (Bio-Rad, Inc.) and incubate at 4 ˚C for 30 min. Add 400 mg Bio-Beads and incubate overnight at 4 ˚C. All steps are performed under mild agitation. Samples were prepared for calcein efflux assay as described in example 1.

**Results and Discussion**

The MscL-mediated release from liposomes was examined by monitoring the increase in fluoresence of the self-quenching fluorescent dye, calcein. Upon addition of MTSET to liposomes, indicated by the arrow, with and without MscL mutant G22C, only the liposomes with the channel protein exhibited effective release of calcein (Fig. 12). Therefore, it can be concluded that mixing all necessary components including the detergent, followed by detergent removal results in a controllable drug delivery vehicle. Additionally, this result shows that the MscL mutant G22C can be used for controlled delivery of a substance in DOPC:Cholesterol (80:20, mol/mol) as well as in DOPC:DOPS (90: 10, mol/mol) as is shown in example 2 and 1.

**Example 4**

Title: pH and light responsive drug delivery, mediated by chemically modified MscL channel proteins.

It was previously shown that substitution of a residue that resides within the channel pore constriction, Gly-22, with all other 19 amino acids affects channel gating according to the hydrophobicity of the substitution (9). One mutant was of particular interest for clinical applications since the MscL mutant G22H exhibited a significantly higher open probability at pH 6.0 compared to pH 7.5. This MscL mutant G22H would be an interesting candidate to deliver drugs at target sites with a lowered pH value, such as in solid tumors or at sites of inflammation. When overexpressing this MscL mutant G22H it became apparent that directly after induction with IPTG, cells would stop growing and the amount of expressed protein was very low, only detectable by western blot analysis (data not shown). Changing growth conditions with respect to medium pH or osmolality did not improve the expression of the MscL mutant G22H. Therefore, this potentially interesting MscL mutant is not usable in a drug delivery application.

Example 1, 2, and 3 showed that MscL mutant G22C can be overexpressed and purified to high enough yield to be applicable in a drug delivery vehicle. Additionally, this mutant allows the specific attachment of a MTS compound, thereby introducing a charge and consequently releasing the substance from the liposomes (Fig. 11 and Fig. 12). Supposedly, a decrease of the pH from 7.5 to 6.0 shifts the equilibrium from the unprotoned to the protonated state of the imidazole side chain of the MscL mutant G22H. This protonation results in the introduction of a charge at amino acid position 22 and thereby affects the opening of the MscL channel. This example will show the chemical synthesis of compounds, reactive specifically with cysteine at amino acid position 22, which introduce chemical groups responsive to pH or light, thereby effecting the local hydrophobicity at the pore constriction and thus affect the gating of the channel protein.

**Example 4A**

This example will show the chemical synthesis of a compound that is reactive specifically with cysteine at amino acid position 22 and contains an imidazole group, effectively mimicking the MscL mutant G22H and circumventing the low production yield of the channel protein.

Materials and Methods

MscL mutant G22C was overexpressed, purified and membrane reconstituted as described in example 2. For labeling of MscL mutant G22C, protein is isolated as described in example 2, but before elution, column is washed with 10 ml of the wash buffer without imidazole. The label is dissolved to a 1 mg/ml final concentration in the same buffer. The wash buffer in the column is allowed to equilibrate over the column matrix. An equal volume of the buffer containing the label is applied to the column matrix. The top of the column is closed after equilibration with nitrogen gas. Column is incubated at 4 ˚C for three days and then the elution procedure is followed as described in example 2.

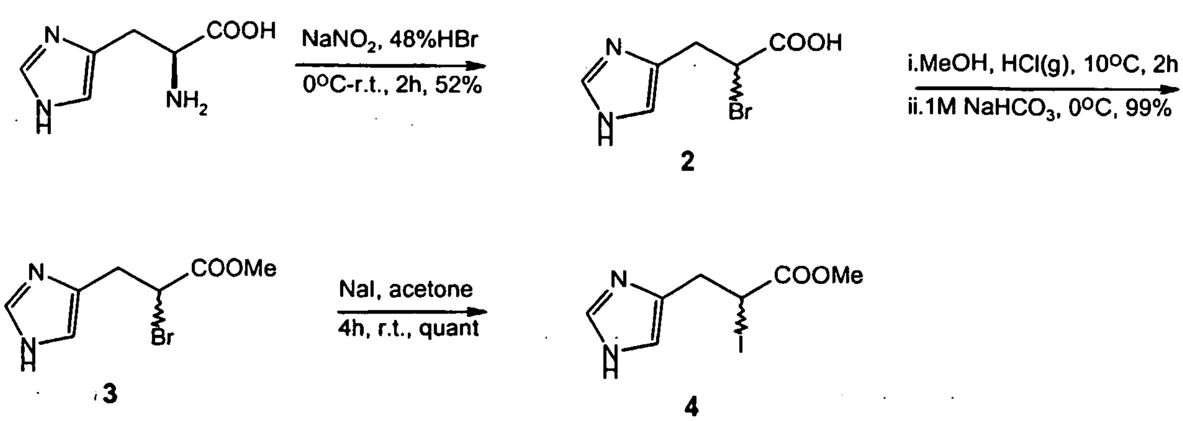

2
3
4

<u>2-bromo-3-(5-imidazolyl)propionic acid monohydrate ( **2** )</u>[2]

To a vigorously stirred suspension of L-histidine (7.76 g, 50 mmol) in HBr (48%, 110 ml) kept at -5 to 0˚C was

dropwise added solution of $NaNO_2$ (10.4 g, 150 mmol) in water (20 ml). After the addition, the solution was stirred 1 hour at 0˚C, 1 hour at room temperature and concentrated in vacuo below 50˚C leaving oil with precipitate. This residue was extracted with acetone (4 x 15 ml), acetone extracts were evaporated in vacuo, water (20 ml) was added and evaporated in vacuo below 50˚C. The residue was dissolved in water (30 ml) and pH was adjusted to 4.6 by aq. ammonia (2M) at 0˚C. The solution was evaporated to dryness in vacuo below 50˚C and the solid residue was triturated with ice-cold water (2 x 30 ml). After drying in vacuo (24 hours, room temperature) the yield of 2-bromo-3-(5-imidazolyl)propionic acid monohydrate **2** was (6.20 g, 52%).

Methyl 2-bromo-3-(5-imidazolyl)propanoate (**3**)[2b]

Through a stirred solution of 2-bromo-3-(5-imidazolyl)propionic acid monohydrate **2** (5 g, 21.1 mmol) in methanol (75 ml) kept at 10˚C was bubbled dry HCl during 2 hours. Then the solution was evaporated in vacuo below 50˚C, the resulting oil was dissolved in aq. $NaHCO_3$ (1M, 75 ml) and extracted with chloroform (3 X 50 ml). The extracts were dried over $Na_2SO_4$, filtered and evaporated in vacuo below 50˚C to yield methyl 2-bromo-3-(5-imidazolyl)propanoate **3** as a slightly yellow oil (4.87g, 99%).

Methyl 2-iodo-3-(5-imidazolyl)propanoate ( **4** )

A solution of NaI (3g, 20 mmol) in acetone (10 ml) was added to a solution of methyl 2-bromo-3-(5-imidazolyl) propanoate **3** (2.33 g, 10 mmol) in acetone (10 ml). Reaction mixture was well stirred and protected from a light. After 4h, reaction mixture was evaporated in vacuo to dryness, residue dissolved in water (15 ml) and extracted with ethyl acetate (3 x 15 ml). Combined extracts were washed with aq. $Na_2S_2O_3$ (1M, 5ml), dried over $Na_2SO_4$, and evaporated in vacuo at room temperature to give methyl 2-iodo-3-(5-imidazolyl)propanoate **4** as slightly yellow oil (2.80 g, 100%).

Results and Discussion

MscL mutant G22C was labelled with 2-bromo-3-(5-imidazolyl)propionic acid monohydrate (BI) or methyl 2-iodo-3-(5-imidazolyl)propanoate (IMI) for three days and products were analysed using ESI-MS. The product of the incubation with BI showed a mass identical to the calculated mass of unlabeled MscL mutant G22C (data not shown). The product of the incubation with IMI showed a mass calculated for the MscL mutant G22C with an expected additional mass of 153 Da, indicative of proper labelling (Fig. 13). Additionally, no unlabeled protein was observed after labelling with IMI under the described conditions and no doubly labelled subunits were observed, indicating that labelling conditions are optimal for IMI. The absence of attachment with BI, which is less hydrophobic than IMI, is consistent with the observations in example 2, Fig. 11 where cysteine reactivity relates to the hydrophobicity of the label.

IMI labelled MscL mutant G22C in spheroplast were analysed using patch clamp to characterize the channel properties as shown in Fig. 14.

Patch clamp experiments on spheroplast allows quantitation of the tension sensitivity because of the presence of an internal control, which is the mechanosensitive channel of small conductance (MscS). By labelling the MscL mutant G22C with IMI the ratio of tension sensitivity of MscL over MscS is significantly decreasing from 2.33 to 1.48. This result indicates that the IMI labelling effectively makes the channel protein open at a lower membrane tension at pH 6. Additionally, calcein efflux assays showed that the channel still has a tendency to stay open at pH 7.0 and 8.0.(Data not shown). For most clinical applications it is important that the channel remains closed at pH values of 7.4. Therefore another compound with a lower pKa was designed and synthesized, see example 4B.

**Example 4B**

The pKa of the IMI group attached to the MscL mutant G22C controlles the gating of the MscL channel and therefore the drug release in response to the pH. To manipulate this pH sensitivity of the drug delivery vehicle, several other pH sensitive compounds were designed (Fig. 15). The pKa's of these compounds are very diverse. These different pKa's allows fine-tuning of the drug release profile to the specific clinical application.

Materials and Methods

MscL mutant G22C was overexpressed, purified, labelled, and membrane reconstituted as described in example 2 and 4A. Synthesis of one of the substituents described in Fig. 15 is described below.

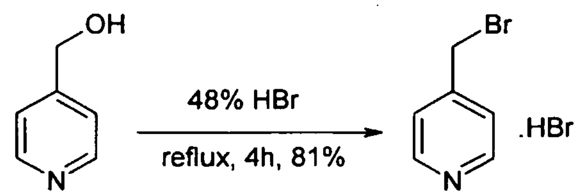


1

4-(bromomethyl)pyridine hydrobromide ( **1** )[1]

4-pyridinylmethanol (2 g, 18.3 mmol) was dissolved in aq. HBr (48%, 20 ml), solution was refluxed for 4 hours and concentrated in vacuo. The semisolid material was triturated with absolute ethanol (10 ml), cooled to 0°C, filtered and washed with another portion of ice cooled absolute ethanol (10 ml). After drying in vacuo, the yield of 4-(bromomethyl) pyridine hydrobromide 1 was (3.67, 81%).

Results and Discussion

Labeling was optimised for this pyridine compound, 4-(bromomethyl)pyridine hydrobromide (BP), to MscL mutant G22C and ESI-MS showed that all channel proteins were labelled (data not shown). Patch clamp was used to characterize the effect of this label on the channel gating properties.

Upon labelling with BP, the MscL mutant G22C channel shows a pH dependent change (figure 16). At pH 7.2 the BP labelled channel behaves as an unlabelled channel by exhibiting the same type of conductance preference and dwell times. However, when the channel is analysed at pH 5.2 it prefers not to open completely but only to subconducting states and the dwell times get shorter. Comparing the behaviour of BP labelled protein at pH 5.2 to both unlabelled protein at pH 5.2 and labelled one at pH 7.2 indicates that the channel opens normally at high pH values but at lower pH values starts to open more readily with shorter dwell times. The behaviour of the BP labelled channel at low pH is very similar to the MTSET labelled channel (Fig. 11), whereas at higher pH values the BP labelled channel behaves as unlabelled channel protein. Therefore it can be concluded that This BP labelled MscL mutant G22C will release drugs comparable as the MTSET induced release of calceine or insulin (examples 2 and 8, respectively) at low pH, whereas at higher pH values the channel is tightly closed ensuring little or no release of these substances.

## Example 4C

Instead of compounds described in examples 2, 3, 4A, and 4B, photo reactive compounds can be designed to react with MscL mutant G22C and respond to the absorption of light by changing the local charge or hydrophobicity. An example of such a photo reactive molecule is 4-{2-[5-(2-Bromo-acetyl)-2-methyl-thlophen-3-yl]-cyclopent-1-enyl}-5-methyl-thiophene-2-carboxylic acid (DTCP1), was designed and synthesized to reversibly switch conformation after light absorption of specific wavelengths (Fig. 17).

Materials and Methods

MscL mutant G22C was overexpressed, purified, labelled, and membrane reconstituted as described in example 2 and 4A

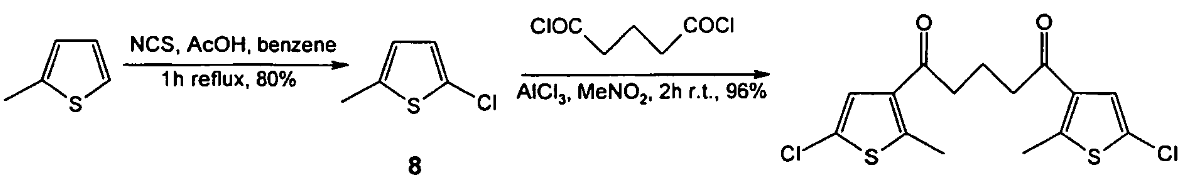


8

9

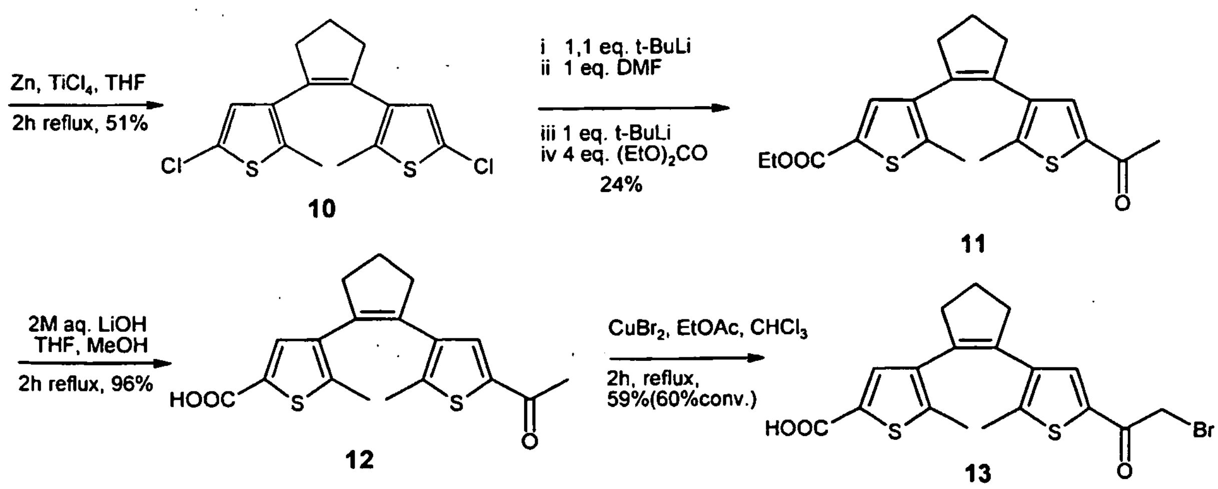


2-chloro-5-methylthiophene ( 8 )[4]

Suspension of N-chlorosuccinimide (75.9 g, 0.568 mol) and 2-mehylthiophene (50 ml, 50.7 g, 0.516 mol) in a mixture of benzene (200 ml) and acetic acid (200 ml) was stirred 30 minutes at room temperature and then 1 hour at reflux temperature. Cooled mixture was poured into aq. NaOH (3 M, 150 ml), organic phase washed with NaOH (3M, 3 x 150 ml), dried over $Na_2SO_4$ and evaporated in vacuo. Slightly yellow liquid product was further purified by vacuum distillation (19 mm, 55"C) to give
Colourless liquid of 2-chloro-5-methylthiophene **8** (55 g, 80.3%).

1,5-bis(5'-chloro-2'-methylthien-3'-yl)pentadione ( **9** )[4]

To a solution of 2-chloro-5-methylthiophene **8** (32.3 ml, 39.8 g, 0.3 mol) and glutaryl dichloride (19.2 ml, 25.4 g, 0.15 mol) in nitromethane (300 ml) was added at 0°C under vigorous stirring $AlCl_3$ (48 g, 0.36 mol) in several portions. After 2 hours stirring at room temperature ice-cold water (150 ml) was added carefully and extracted with diethyl ether (3 x 150 ml). Combined ether extracts were washed with water (100 ml), dried over $Na_2SO_4$ and evaporated in vacuo to yield a brown tar (52 g, 96%). This crude 1,5-bis(5'-chloro-2'-methylthien-3'-yl)pentadione **9** was used further without purification.

1,2-bis(5'-chloro-2'-methylthien-3'-yl)cyclopentene ( **10** )[4]

To a Zn dust (10 g, 0.153 mol) suspension in dry THF (200 ml) in a three-neck flask under nitrogen was slowly added $TiCl_4$ (24.8 ml, 42.9 g, 0.226 mol) through a glass syringe and resulting mixture was refluxed for 45 minues. Then the flask was cooled in an ice bath and crude 1,5-bis(5'-chloro-2'-methylthien-3'-yl)pentadione **9** (27.4 g, 75.9 mmol) was added. After refluxing for 2 hours, reaction was quenched with aq. $K_2CO_3$ (10%, 200 ml) and extracted with diethyl ether (4 x 80 ml). Combined organic extracts were washed with water (100 ml), dried over $Na_2SO_4$ and evaporated in vacuo. After column chromatography on silica-gel (petroleum ether 40-60) 1,2-bis(5'-chloro-2'-methylthien-3'-yl)cyclopentene **10** was obtained as a white solid (12.6 g, 50%).

ethyl 4-[2-(5-acetyl-2-methyl-3-thienyl)-1-cyclopenten-1-yl]-5-methyl-2-thiophenecarboxylate ( **11** )

To a 1,2-bis(5'-chloro-2'-methylthien-3'-yl)cyclopentene **10** (700 mg, 2.13 mmol) in diethyl ether (50 ml) was at 0°C added t-BuLi (1.5M in pentane, 1.7 ml). After 10 min. the cooling bath was removed and reaction mixture stirred for next 50 min at room temperature. Then N,N-dimethylacetamide (0.2 ml, 195 mg, 2.23 mmol) was added at 0°C, stirred at this temperature for 10 min. and 50 min at room temperature. Again t-BuLi (1.5 M in pentane, 1.4 ml) was added at 0°C stirred at this temperature for 10 min. and 50 min. at room temperature and finaly was added diethyl carbonate (1 ml, 957 mg, 8.25 mmol) at 0°C stirred at this temperature for 10 min. and 50 min at room temperature. Reaction was then quenched with aq. HCl (1M, 20 ml), organic layer separated and water layer extracted with diethyl ether (3 x 20 ml). Combined organic layers were washed with saturated aq. $NaHCO_3$ (10 ml), dried over $Na_2SO_4$ and evaporated in vacuo. After column chromatography on silica-gel (hexane : ethyl acetate / 9 : 1) ethyl 4-[2-(5-acetyl-2-methyl-3-thienyl)-1-cyclopenten-1-yl]-5-methyl-2-thiophenecarboxylate **11** is obtained (192 mg, 24%)

4-[2-(5-acetyl-2-methyl-3-thienyl)-1-cyclopenten-1-yl]-5-methyl-2-thiophenecarboxylic acid ( **12** )

To a solution of ethyl 4-[2-(5-acetyl-2-methyl-3-thienyl)-1-cyclopenten-1-yl]-5-methyl-2-thiophenecarboxylate **11** (114 mg, 0.315 mmol) in mixture of THF (3 ml) and methanol (1 ml) was added aq. LiOH (2 M, 0.6 ml) and the mixture was refluxed for 24 hours. Then aq. HCl (1 M, 10 ml) was added and extracted with ethyl acetate (3 x 10 ml). Organic extracts were washed with water (5 ml), dried over $Na_2SO_4$ and evaporated in vacuo. After column chromatography on silica-gel (hexane : ethyl acetate / 1 : 1, then $CH_2Cl_2$ : methanol /9 : 1) 4-[2-(5-acetyl-2-methyl-3-thienyl)-1-cyclopenten-1-yl]-5-methyl-2-thiophenecarboxylic acid **12** was obtained (101 mg, 92%)

4-{2-[5-(2-bromoacetyl)-2-methyl-3-thienyl]-1-cyclopenten-1-yl}-5-methyl-2-thiophenecarboxylic acid ( **13** )

To a boiling suspension of finely grounded $CuBr_2$ (130 mg, 0.581 mmol) in ethyl acetate (2 ml) was with vigorous stirring added solution of 4-[2-(5-acetyl-2-methyl-3-thienyl)-1-cyclopenten-1-yl]-5-methyl-2-thiophenecarboxylic acid **12** (101 mg, 0.292 mmol) in chloroform (2 ml). After 2 hours of reflux the mixture was filtered and evaporated. After column chromatography on silica-gel ($CH_2Cl_2$ : methanol/99 : 1) 4-{2-[5-(2-bromoacetyl)-2-methyl-3-thienyl]-1-cyclopenten-1-yl}-5-methyl-2-thiophenecarboxylic acid **13** was isolated (73 mg, 59%) together with starting material (40 mg, 40%).

Nuclear Magnetic Resonance and spectroscopic analysis (data not shown) indicated DTCP1 was chemically and functionally correct as shown in Fig. 17.

Results and Discussion

DTCP1 was designed to specifically react with the free sulfhydryl group of a single cysteine at position 22 of MscL (G22C-MscL). Position 22 in the MscL channel was chosen for its involvement in the gating mechanism of the channel. A conjugation protocol was developed and the products were analyzed employing electrospray ionization mass spectrometry (ESI-MS) and absorption spectroscopy. ESI-MS indicated that the mass of all MscL subunits increased with 344 Da, a mass increase expected for a conjugation of DTCP1 to a sulfhydryl group of MscL as shown in Fig. 18. The two photo-isomers of DTCP1 exhibit different absorption spectra in UV region Fig. 19. This difference was used to monitor the switching of DTCP1 after conjugation to MscL and reconstitution of the detergent solubilized G22C-MscL-DTCP1 conjugate into DOPC:DOPS (90:10, mol/mol) containing lipid bilayer Fig. 20 (due to light scattering by liposomes only substracted spectra before and after irradiation can be shown)

As can be seen from Fig. 20 conjugation and reconstitution into lipid bilayer has no effect on switching of DTCP1. To prove reversibility and reproducibility of switching, we repeteadly irradiated this system with 313 nm UV light to achieve closed form and with light with wavelenght longer than 460 nm to return back to open form while monitoring at 535 nm (absorption maximum of closed form) Fig. 21.

These data shows that we have synthesized an organic molecule (DTCP1) and this molecule can be conjugated to a specific site in the MscL channel, known to alter the gating properties of the channel, while maintaining the desired photochemical properties.

**Example 4D**

The DTCP1 molecule (example 4C) contains free carboxylic group in order to modify hydrophobicity of the pore of MscL. To enhance the hydrophilic properties of the synthesised molecule, we prepared spiropyran derivative SP1 Fig 22, which after UV irradiation changes into highly charged merocyanine form.

Materials and Methods

MscL mutant G22C was overexpressed, purified, labelled, and membrane reconstituted as described in example 2 and 4A, except labelling on column was 30 min. instead of 3 days.

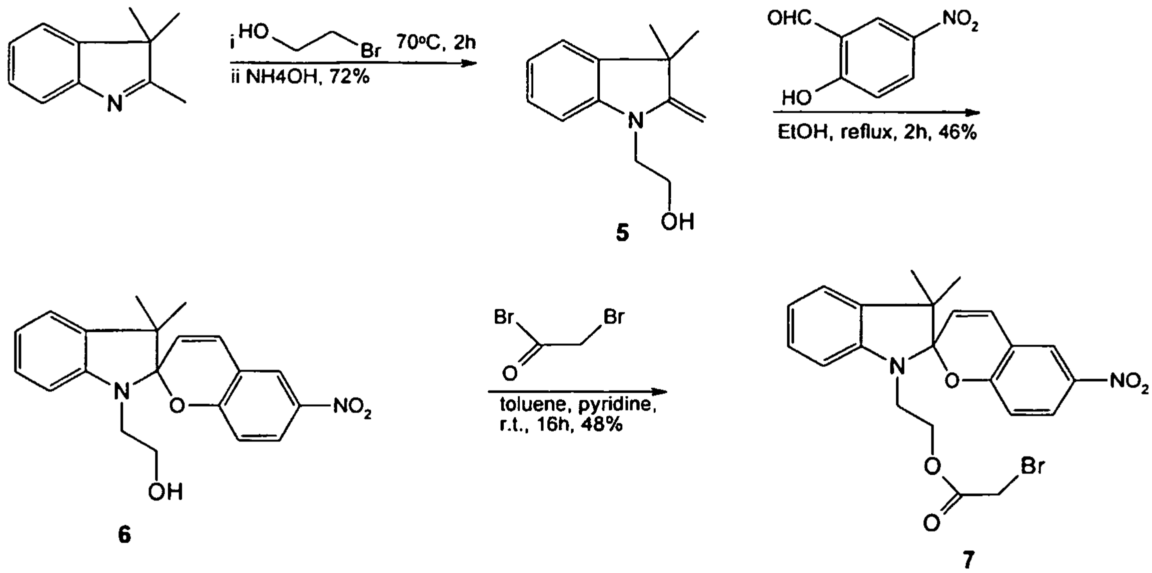


5 6 7

2-(3,3-dimethyl-2-methylene-2,3-dihydro-1*H*-indol-1-yl)-1-ethanol ( **5** )[3]

The mixture of 2,3,3-trimethyl-3*H*-indole (5 g, 31.4 mmol) and 2-bromoethanol (2.22 ml, 3.92 g, 31.4 mmol) was heated with stirring at 70˚C for 2 hours, then cooled to room temperature and washed with aq. ammonia (25%, 25 ml). Separated yellow oil was extracted with diethyl ether, dried over $Na_2SO_4$ and evaporated to give 2-(3,3-dimethyl-2-methylene-2,3-dihydro-1*H*-indol-1-yl)-1-ethanol 5 as an oil (4.57 g, 72%)

2-(3,3-dimethyl-6-nitrospiro[2*H*-1-benzopyran-2,2'indoline])-1-ethanol (**6**)[3]

The solution of 2-(3,3-dimethyl-2-methylene-2,3-dihydro-1*H*-indol-1-yl)-1-ethanol 5 (2 g, 9.8 mmol) and 2-hydroxy-5-nitrobenzaldehyde (1.64 g, 9.8 mmol) in ethanol (50 ml) was refluxed for 2 hour. After filtration, the product was recrystalised from ethanol. Yield of pure 2-(3,3-dimethyl-6-nitrospiro[2*H*-1-benzopyran-2,2'indoline])-1-ethanol **6** was (1.6 g, 46%)

2-(3,3-dimethyl-6-nitrospiro[2*H*-1-benzopyran-2,2'indoline])ethyl 2-bromoacetate ( **7** )

The solution of 2-(3,3-dimethyl-6-nitrospiro[2*H*-1-benzopyran-2,2'indoline])-1-ethanol **6** (1 g, 2.84 mmol), bromoacetyl bromide (0.37 ml, 0.86 g, 4.26 mmol) and pyridine (0.35 ml, 0.34 g, 4.26 mmol) in toluene (10 ml) was stirred at room temperature for 16 hours. Then water (10 ml) was added and extracted with diethyl ether (3 x 10 ml). Organic extracts were dried over $Na_2SO_4$, filtered and evaporated in vacuo. After chromatography on silica-gel (hexane : ethyl acetate / 5 :1) oily product **7** (0.65 g, 48%) was obtained.

Results and Discussion

SP1 react specifically with free sulfhydryl group of cysteine at position 22 of MscL allowing us to specifically modify the channel protein. Fig. 23 shows the UV change of the SP1 conjugated to MscL after irradiation with 313 nm UV light. New peak at 550 nm belongs to merocyanine form of the molecule.

To show reversibility and reproducibility of switching, we repeatedly irradiated SP1 conjugated to protein at 313 nm UV light to achieve merocyanine form and with light with wavelength longer than 460 nm to return back to spiropyran form while monitoring at 550 nm (absorption maximum of closed form) Fig. 24.

Materials and methods

Starting materials were commercially available (Aldrich, Acros Chimica, Fluka) and were used without further purification. Diethyl ether and THF were distilled from Na. For column chromatography Aldrich silica gel Merck grade 9385 (230-400 mesh) was used.

Compounds **10 - 13** are light sensitive and were handled in dark, resp. using brown glassware.
All compounds were characterised using $^1$H NMR (Varian VXR-300 at 300 MHz, or Varian Gemini-200 at 200 MHz), $^{13}$C NMR (Varian VXR-300 at 75.4 MHz, or Varian Gemini-200 at 50.3 MHz), and mass analysis (MS-Jeol mass spectrometer).

1 Bixler, R.L., Niemann, C. J. Org. Chem., 1958, 23, 575
2

(a) Yankeelov, J.A.; Jolley, C.J. Biochemistry, 1972, 11, 159
(b) Maat, L.; Beyerman, H.C.; Noordam, A. Tetrahedron, 1979, 35, 273

3 Sakuragi, M.; Aoki, K.; Tamaki, T.; Ichimura, K. Bull. Chem. Soc. Jpn., 1990, 63, 74
4 Lucas, L.N.; Esch, J.; Kellog, R.M.; Feringa, B.L. Chem. Commun., 1998, 2313

**Example 5**

Title: Upon exposure to light, a photo reactive lipid alters its chain conformation, which induces a changed lateral pressure in the membrane to controlle the gating of the MscL channel.

The basic components of this drug delivery vehicle are a lipid membrane and the MscL channel protein. Controlled release of a drug from these vehicles can either be achieved by directly effecting the gating mechanism of the channel protein, or indirectly, by effecting the physical properties of the lipid bilayer, which subsequently controls the gating of the channel. This example shows the synthesis of photo reactive lipids, that when incorporated in liposomes, can affect the lateral pressure in these membranes, thereby controlling the gating of the MscL channel protein.

Photo reactive lipids were designed and synthesized to reversibly switch conformation upon radiation with light of an appropriate wavelength (fig 25).

Three different lipids with an azobenzene unit have been synthesized (J. M. Kuiper and J.B.F.N. Engberts. to be published).

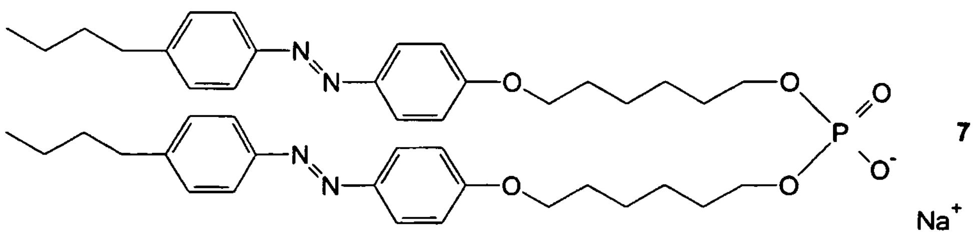

**7**

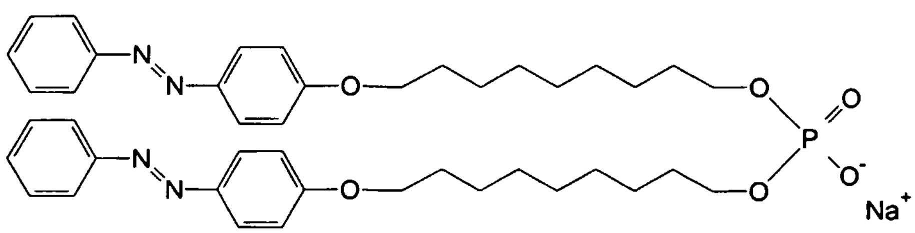

**6**

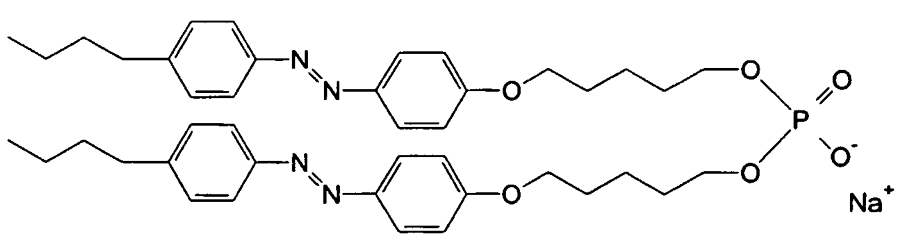

**8**

The synthesis of lipid 6 is described in the experimental section. The synthesis of **7** and **8** is similar.

Materials and Methods

Synthesis of **1**. To 4.0 g (20.16 mmol) of 4-phenylazophenol in 150 ml of acetone was added 4.52 g (20.16 mmol) 9-bromonona-1-ol, 5.56 g (40.32 mmol) of $K_2CO_3$ and a catalytic amount of KI. The mixture was refluxed for 5 days. The acetone was removed by evaporation under reduced pressure. Dichloromethane (500 ml) was added and the organic layer was washed three times a fresh layer of water. The organic layer was dried over $NaSO_4$, filtrated and evaporated under reduced pressure. The resulting yellow solid material was purified by crystallization from ethyl acetate (120 ml). Yellow crystals were obtained in 75% yield and the product was characterized by $^1$H and $^{13}$C NMR.

Synthesis of **2**. To 1.5 g (4.41 mmol) of 1 in 15 ml of dry dichloromethane under a nitrogen atmosphere was added slowly 238 μl (2.94 mmol) of pyridine and 128 μl (1.47 mmol) of $PCl_3$. The reaction was monitored by TLC(silica, ether) and additional portions of pyridine and $PCl_3$ (ratio 2:1) were added when the alcohol was still present. After the reaction was completed, the dichloromethane was washed twice with a saturated aqueous solution of NaCl. In case of very difficult separations the addition of some acid brought sometimes some relief. The organic layer was dried over $NaSO_4$, filtered and evaporated under reduced pressure. The resulting material was stirred overnight in hexane and the crystals were removed by filtration. These crystals were further purified by crystallization from ethanol. The hot solution of product in ethanol was filtrated. The crystallization took place at room temperature. The crystallization was repeated and pure yellow crystals were obtained in a 53% yield. The product was characterized by $^1$H, $^{31}$P and $^{13}$C NMR.

Synthesis of **5**. To 0.305 mg (0.42 mmol) of **2** in 15 ml of tetrachloromethane was added 234 μl (1.68 mmol) of triethylamine and 8 μl (±0.1 eq) of diisopropylethylamine. The mixture was stirred at room temperature for 19 days. The reaction time is much shorter (2-3 days); the conversion can easily be followed by $^{31}$P NMR. The volatile compounds were removed by evaporation under reduced pressure. To the resulting material (**3**) 0.6 ml acetic acid and 3 ml triethylamine was added. After two days of stirring at room temperature the reaction was completed. Again the volatile compounds were removed by evaporation under reduced pressure. The obtained crude product (4) was hydrolyzed by stirring in acidic water (pH=4-5, 100 ml) for 30 min. The resulting mixture was subjected to water/dichloroethane (100 ml) extraction. The organic layer was washed twice with a saturated aqueous solution of NaCl. With difficult separations the addition of some acid is advantageous. The organic layer was dried over $NaSO_4$, filtrated and evaporated under reduced pressure. The solid material was further purified by crystallization from ethanol. The hot solution of product in ethanol was filtrated. The solution was put away in a refrigerator overnight. The crystals were washed with cold ethanol. Yellow crystals were obtained in a 66% yield and the product (5) was characterized by $^1$H, $^{31}$P and $^{13}$C NMR.

Synthesis of **6**. To 0.1773 g (0.245 mmol) of **5** was added 1.80 g of a sodium ethoxide solution in ethanol (0.136 mmol/g). Extra dry ethanol was added and slowly the solution was warmed up until it became clear. After cooling down crystals were formed and the solution was put in the refrigerator. The crystals were washed with cold ethanol. Yellow crystals were obtained in an 89% yield and the product (**6**) was characterized by $^1$H, $^{31}$P and $^{13}$C NMR.

Preparation of the vesicles.

DSP/lipid **7** (95:5, mol/mol): The appropriate amounts of the lipids were solubilized in methanol. A thin film was created by evaporating the methanol under reduced pressure. Subsequently the film was kept under a high vacuum for at least one hour. Water was added and the mixture was stirred firmly for one hour at 85°C. At the end tipsonication was applied (3 times for 30 s). A clear solution was obtained.

DOPC/lipid **6** (95:5, mol/mol): The appropriate amounts of the lipids were solubilized in methanol. A thin film was created by evaporating the methanol under reduced pressure. Subsequently the film was kept under a high vacuum for at least one hour.

Water was added and the mixture was stirred firmly. The mixture was kept at 95°C for 15 minutes and after that the mixture was sonicated with a waterbad for a few minutes. A clear solution was obtained.

Results and Discussion

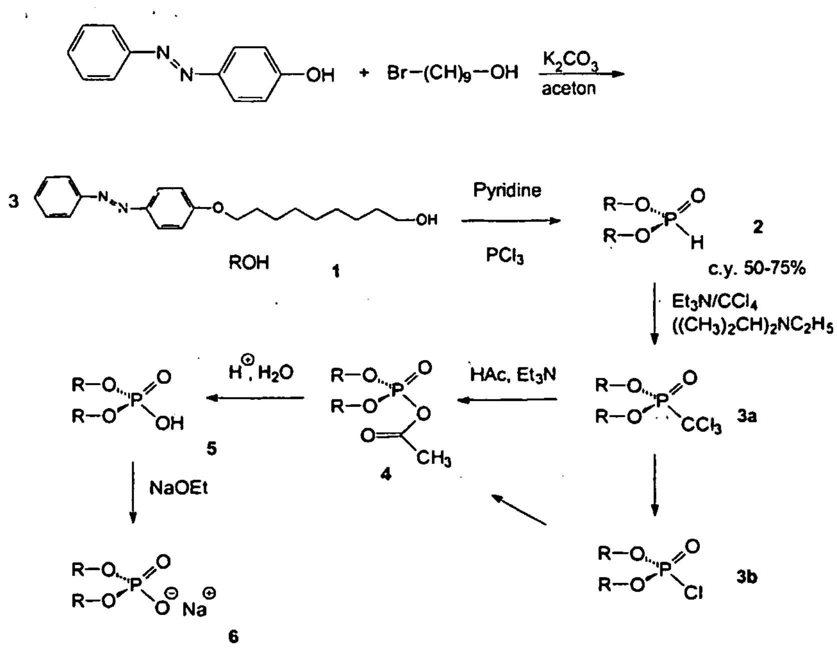


Scheme 2, Synthesis phosphates, see above.

Synthesis: A new synthetic route was used to synthesis the sodium phosphates (Scheme 2). Particulary the combination of the 3rd, 4th and 5th step is new. These are very mild steps and they can easily be followed by $^{31}P$ NMR. The 3rd, 4th and 5th step take place with a complete conversion. First with the use of $PCl_3$ and pyridine the phosphonate can be synthesized. Then with the use of a base and tetrachloromethane compound **3a** is obtained. This is called the Atherton Openshaw Todd reaction. **3a** can react further to **3b.**

After addition of acetic acid and base, a nucleophilic attack of acetic acid takes place at the phosphorus atom. After acid-catalyzed hydrolysis the phosphate acid is obtained which can be converted into the sodium salt with the use of sodium ethoxide.

Vesicle formation.

It was found that lipids **6-8** are not vesicle forming. This was confirmed by EM (electron microscopy, data not shown). Therefore the lipids were mixed with vesicle forming lipids (e.g. DOPC, DOP (sodium dioleyl phosphate) and DSP (sodium distearyl phosphate)). With a ratio of 95:5 for vesicle forming lipid and azobenzene containing lipid, stable vesicle solutions could be prepared. All mixtures were examined by EM.

UV/Vis spectroscopy and irradiation experiments.

In Fig. 26. the UV/Vis absorption spectra of a mixture of DSP and **7** are shown. The trans isomer was easily switched into the cis isomer upon irradiation with light of 365 nm. Also the back isomerization went smoothly. For a mixture of 95% DOPC and 5% lipid **6** the irradiation cycle was repeated several times. (Figure 27)

From the experiments it can be concluded that the isomerization cycle can be repeated several times without decomposition of the material. The trans azobenzene was subjected to irradiation (at 365 nm), for 30 second intervals, and the UV/Vis spectrum of the sample was taken between each irradiation cycle. (Figure 28) After 4 minutes of irradiation the UV/Vis spectrum did not change anymore which points to a maximal isomerization to the cis isomer. As can be seen from figure 28, isobestic points are observed. This means that there is only a transition from the trans isomer to the cis isomer and that are no side reactions.

**DSC experiments.**

The DSC graphs show that the phase transition temperature of the vesicles of DSP is changed if 5% of lipid **6** is added (Fig. 29). This indicates that the azobenzene containing lipids are incorporated into the vesicles. The broad transition indicates that a variety of domains of different lipids compositions are present.

The photo reactive lipids described above in combination with other lipids form liposomes and the physical properties of these liposomes can be altered upon irradiation. MscL channel or derivatives thereof can be reconstituted into these lipid membranes and become responsive to the cis trans switching of the photo reactive lipids resulting in controlled drug release.

**EXAMPLE 6A**

Title: The design of an animal model and the testing of MscL mediated drug release.

Besides triggered drug release (by pH, osmotic pressure and light), MscL containing liposomes can be used for sustained drug release. The rate of drug release can be controlled by the rate of channel gating, a property that can be manipulated by genetic or chemical modification.

The effect of drug formulation on the rate of drug release in-vivo was tested in the rat by external counting of radioactivity. The model is based on liposomal formulations that remain at the subcutaneous site of administration with an encapsulated model drug which, in case released, is rapidly removed from the subcutaneous site of injection and excreted into the urinary bladder.

Materials and Methods

Material

DOPC/DOPS (90: 10, mol/mol) liposomes with or without the MscL mutant G22C were used (protein to lipid ratio of 1:20, wt/wt). Radiolabeled mertiatide ($^{99m}$Technetium-MAG3) was chosen as model drug because of its rapid and exclusive excretion from the circulation into the urine (via active tubular secretion, 600 mL/min. in humans).

Sample preparation

Encapsulation of MAG3 in liposomes was performed by freezing/thawing three times followed by extrusion through a 400 nm filter. The free fraction of the compound was removed by G50 Sephadex column separation.

The normal liposomes were loaded with MAG3 in 0.9% NaCl and eluted on the G50 column with 25 mM HEPES pH 8, 150 mM NaCl. The G22C-MscL-liposomes were loaded with the model drug in 150 mM sucrose and 145 mM NaCl and eluted on the G50 column with 25 mM HEPES pH 8, 150 mM sucrose, 145 mM NaCl.

Kinetics of encapsulated model drugs administered subcutaneously

Male Wistar rats were anaesthetized using iso-flurane $O_2$/NO throughout the study. Liposome encapsulated MAG3 in a 0.5 ml volume was injected subcutaneously (in the neck) and accumulation of radioactivity in the urinary bladder constantly monitored by external counting using a gamma-camera (window: 140 keV, 250 keV width, 1 min time resolution). At the end of the study (after 40 min.), a 10 times higher dose of free MAG3 was administered subcutaneously or intravenously to measure the urinary excretion rates of those formulations.

Results and Discussion

Kinetics of free and encapsulated model drugs (Fig. 30)

Compared to intravenous administration of the free compound, the urinary excretion of MAG3 was slower after subcutaneous injection (50% in 3 and > 12 min., respectively).
Encapsulation in liposomes reduced the rate of urinary MAG3 excretion with a significant difference between the liposomes tested. Compared to the normal DOPC/DOPS liposomes, the G22C-Mscl containing liposomes released significantly more MAG3 (15% and 45% urinary MAG3 excretion in the first 30 min. after injection).

These results show that liposomes containg MscL mutant G22C exhibit release of the hydrophilic molecules MAG3. This MscL mediated release is significantly faster than MAG3 release from liposomes without MscL but slower compared to free MAG3 injected subcutaneously. Therefore, it can be concluded that the MscL channel modulates the transport of hydrophilic molecules in-vivo, and can be used as a drug delivery vehicle for sustained release.

**EXAMPLE 6B**

Example describes the use of MscL channels or derivatives thereof for sustained release of drugs. In examples 1,2,4, and 5 different examples are described to control the gating of the channel and thus the release of drugs. In one

example, pH is described as a signal to control the gating of the channel. This example describes a method to induce a temporary pH-reduction subcutaneously for the testing of pH sensitive MscL-mediated drug release.

Materials and Methods

pH-reduction subcutaneously

Male Wistar rats remained conscious throughout the study. The subcutaneous pH was constantly recorded using a micro-glass electrode. After stabilization of the pH, 0.5 ml MES buffer (pH 6.1) was injected approximately 3 mm from the pH-electrode. The effect of different molarities (50, 100 and 250 mM) of the MES buffer on the time-course of pH reduction was tested.

Results and Discussion

pH reduction subcutaneously

MES buffer is suitable to lower the pH in the subcutaneous tissue. The duration of pH reduction appeared to depend on the molarity of the buffer (figure 31). With 50 and 100 mM MES, the pH returned steadily to physiological pH (pH 7.4) within 10 min. By using 250 mM MES, pH remained below pH 6.5 for more than 30 min.

The subcutaneous tissue can temporarily be acidified by a MES buffer with the molarity of the buffer determining the duration of pH reduction. Short-lasting pH reductions allow to measure the effect of repeated gating and closing of the channel.

**EXAMPLE 6C**

The radioactive method, described in example 6A, is suitable to determine the rate of subcutaneous released drug administered in different formulations. Drawbacks are the unphysiological state of anesthesia and the limited period of time that can be measured (due to the short half-life of the radioactive label, the instability of the compound and the required anesthesia). Therefore, an alternative was developed. In this method, the release of a drug from different formulations subcutaneously, can be determined in conscious rats for a long period of times (days).

Materials and Methods

Material

DOPC/DOPS (90:10, mol/mol) and DOPC/DOPE liposomes (70:30, mol/mol) liposomes were tested. Iodothalamate (IOT) was chosen as model drug because of its rapid and exclusive excretion (via glomerular filtration, 1 $ml.min^{-1}$. 100 g rat) from the circulation into the urine.

Sample preparation

Encapsulation of IOT in liposomes was performed by freezing/thawing three times followed by extrusion through a 400 nm filter. The free fraction of the compound was removed by G50 Sephadex column separation.

The liposomes were loaded with IOT in an iso-osmotic solution (25 mM HEPES pH 7.4 and 145 mM NaCl) and eluted on the G50 column using the same buffer as eluens.

Kinetics of encapsulated model drugs administered subcutaneously

Male Wistar rats remained conscious throughout the study. To increase the time-resolution, the diuretic furosemide (10 mg/kg dose s.c) was given in the morning, 7 hours before administration of the sample. Urine was collected automatically with a 1 hour resolution. The concentration of IOT in the urine was measured by HPLC.

Results and Discussion

Kinetics of free and encapsulated model drugs.

After subcutaneous injection of free IOT, the first phase urinary excretion of the model drug was completed in the first couple hours after injection( figure 32). In contrast, the rate of excretion was strongly reduced by liposomal

encapsulation.

Both, the radioactive method (example 6A) and the present method are suitable to measure the stability of subcutaneous liposomal drug formulations. The radioactive method is more suitable for relatively fast releasing formulations whereas the last described method is more suitabable for the slower releasing formulations. These animal models can be used to monitor the controlled release of drugs from liposomal formulations containing MscL channels or derivatives thereof that respond to changes in pH, light of a specific wavelengths, changes in osmolality, or the addition of an activator such as MTSET or reduced gluthathione (described in previous examples).

Example 7

Title: $MscL^{Ll}$: Mechano sensitive channel of large conductance homologue found in *Lactococcus lactis* IL1403 (NCBI: 12725155)

Certain applications require MscL channels with specific characteristics. For this it is possible to use mutants or chemically modified MscL channels of *E.coli.* Alternatively homologues mechanosensitive channels from other organisms could be used. This example describes the cloning, overexpression, purification, membrane reconstitution, and functional characterization of a MscL homologue found in *Lactococcus lactis.* An additional advantage of this system is the significantly higher overexpression of the channel protein, and the MscL channel protein originates, and is overexpressed, in a GRAS organism.

Material and Methods

$MscL^{Ll}$ Expression, Purification and reconstitution

The gene of $MscL^{Ll}$ was taken from the GRAS organism *Lactococcus lactis* IL1403 and cloned with a 6-histidine tag into an overexpression vector. *Lactococcus lactis* NZ9000 cells containing the plamid $pNZ8020MscL^{Ll}6H$ carrying the MscL-6Histidine construct was grown to $OD_{600}$ of approximately 1 in 3L M17(Difco) medium supplemented with 10mM argenine and 0.5% galactose and induced with 0.5ng/ml (final concentration) Nisin for 3h. The cells were harvested and washed by centrifugation (10min 6,000 x g) in 50mM Tris-HCl pH 7.3 buffer. After incubation for 30min at 30˚C with 10mg/ml lysozyme, $MgSO_4$ was added to the cell suspension to a final concentration of 10mM and DNase and Rnase were added to 0.1mg/ml ruptured by two fold passage through a French Pressure cell (15kPsi *L. lactis*). The cell-debris and cell membranes were separated by centrifugation (10min 11,000 x g) after addition of 15mM Na-EDTA pH 7.0. The membranes, contained in the supernatant, were collected by ultra-centrifugation (1h 150,000 x g) and resuspended in 3ml (total protein content: 20mg/ml) in 50mM Tris-HCl pH 7.3 and stored at -80˚C until further use.

Before purification 1volume of membranes was solubilised with 9volumes of 50mM $Na_2HPO_4$.$NaHPO_4$, 300mM NaCl, 10mM imidazole at pH 8.0 (buffer A) containing 3% n-octyl β-glucoside. The extract was cleared by ultra-centrifugation (20min 150,000 x g) and mixed with 1(bed)volume $Ni^{2+}$-NTA agarose beads (pre-equilibrated with buffer A + 1% n-octyl β-glucoside) and gently rotated for 30min at 4˚. The mixed column material was then poured into a Bio-spin column (Bio-Rad) and washed with 20volumes buffer A + 1% n-octyl β-glucoside. The protein was eluted with buffer A + 1% n-octyl β-glucoside and increasing amounts of L-Histidine (1vol 50mM, 1vol 100mM, 2x 1vol 200mM). Protein concentration was detemined according to Schaffner and Weissmann [1]. Further analysis was done on a SDS-15% polyacrylamide gel followed by staining with Coomassie Brilliant Blue or transferral to PVDF membranes by semi-dry electroforetic blotting for immunodetection with anti-His antibodys (Amerham Pharmacia Biotech). Immunodetection was performed with an alkaline phosphatase conjugated secondary antibody as recomended by the manifacturer (Sigma).

The purified protein was reconstituted a mixture of the following lipids: 1,2-Dioleoyl-sn-Glycero-3-Phosphocholine (avanti 850375) and 1,2-Dioleoyl-sn-Glycero-3-Phospho-L-serine (avanti 810225) 9:1 w/w or Dioleoyl-sn-Glycero-3-Phosphocholine and Cholesterol (avanti 700000) 8:2 mol:mol. Before reconstitution the lipids were washed and mixed in chloroform (20mg/ml) and dried under $N_2$ gas. The dried lipids were then resuspended in 50mM Kpi buffer pH 7.0 to a final concentration of 20mg/ml. The suspension was then sonicated using a tip sonicator (8 cycles 15s on 45s off, intensity of 4μm (peak to peak)). The formed liposome solution was then completely solubilised using n-octyl β-glucoside and the purified protein was added (1:1000, 1:500 or 1:50 w/w protein/lipid). Proteoliposomes were then formed by dialysing the lipid-protein mixture for 3 days at 4˚C against 500volumes 50mM Kpi pH 7.0 without any detergent, using a 3,500Da MWCO Spectrum spectrapor dialysis-membrane. After the first night incubation 0.5g of polystyrene beads (Bio-Beads SM2™) were added for extra detergent removal.

Freeze fracture Electron microscopy

Freeze fracture electron microscopy of reconstituted $MscL^{Ll}$ was performed as described elsewere [2].

Electrophysiological characterization of $MscL^{Ll}$

Electrophysiological characterization was essentially performed as described by Blount et al [3]. Giant spheroplasts of *E. coli* PB104 (MscL negative) containing the plasmid pB10bMscL$^{Ll}$6H (for overexpression of $MscL^{Ll}$) were generated: cells were grown to $OD_{600}$ of 0.5 diluted 10 fold and grown in the presence of 60μg/ml cephalexin (preventing septation, but not cellgrowth) and 1.3mM IPTG. When the cells had formed non- septated filamentous snakes of 50-150μm they were harvested at 5,000 x g. The pellet was resuspended in 1/10$^{th}$ of the original volume of 0.8M sucrose. Cell outer-membranes (peptidoglycan) were digested with lysozyme (200μg/ml) in the presence of DNase (50μg/ml) in 50mM Tris-HCl, 6mM Na-EDTA at pH 7.2 for 2-5 minutes. The reaction is stopped when sufficient giant spheroplasts have formed by the addition of 8mM $MgCl_2$ (final concentration). Spheroplasts were enriched by spinning on a 0.8M sucrose cushion.

Alternatively patches were studied from liposomes with reconstituted $MscL^{Ll}$. Proteoliposomes were centrifuged at 200,000 x g, resuspended to 100mg/ml in 10mM MOPS pH 7.2, 5% ethylene glycol and dried over-night for 4h on glass slides in a desiccator at 4˚C. Rehydration of the lipids to 100mg/ml was performed in liposome patch buffer (200mM KCl, 0.1M EDTA, $10^{-2}$mM $CaCl_2$ and 5mM HEPES pH 7.2). The proteoliposomes were then loaded into the sample well containing patch buffer with 20mM $MgCl_2$ caused the lipid sample to form large unilammelar blisters, which were patched.

Patches were examined at room temperature, with symmetrical solutions for pipette and bath. The buffer is composed of 200mM KCl, 0.1M EDTA, $10^{-2}$mM $CaCl_2$ and 5mM HEPES pH 7.2. For spheroplasts 90mM of $MgCl_2$ was added, for proteoliposomes 20mM. Recordings of currents trough open channels were performed at +/-20mV. Data on pressure and current were acquired at a sampling rate of 30kHz with a 10kHz filtration and analysed using PCLAMP8 software. Pressure was measured using a piezoelectric pressure transducer (Micro-switch)(World Precision Instruments PM01).

In vitro release profiles of a model drug from proteoliposomes

As described in example 1.

Results and Discussion

$MscL^{Ll}$ Expression, Purification and reconstitution

This means that expression levels in the membrane were around 5% of total membrane protein. The purification of $MscL^{Ll}$ was to apparent homogeneity in a single step using nickel chelate affinity chromatography as shown in figure 33 lane c. Per ml of vesicles about 1mg of protein could be obtained at an estimated purity of 95% based on SDS-PAGE and Coomassie Brilliant Blue staining. The band also showed up very clearly after immunological detection on a PVDF membrane (data not shown). The amount of protein for reconstitution was determined experimentally. For patch-clamp experiments a 1: 1000 w/w protein/lipid ratio was found to be useful whereas for the calcein release assay a 1:500 ratio seemed to give the clearest results.

Freeze fracture Electron microscopy

Figure 34 shows an electron micrograph of freeze-fractured proteoliposomes. As can be seen the $MscL^{Ll}$ protein was indeed inserted into the lipid bilayer.

Electrophysiological characterization of $MscL^{Ll}$

Figure 35 shows a typical trace of the $MscL^{Ll}$ in *E. coli* spheroplasts. The channel openings are indicated as an upward current as a result of the applied pressure. Both $MscS^{Ec}$ and $MscL^{Ll}$ channels are visible in this patch enabling a sensitivity comparison to $MscL^{Ec}$. This showed that $MscL^{Ll}$ in *E. coli* cells opens at higher pressures than $MscL^{Ec}$. The ratio of pressures for opening MscL/MscS are 2.4 for $MscL^{Ec}$ and 2.8 for $MscL^{Ll}$. Figure 36 gives information on pressure sensitivity, open dwell time and conductance of $MscL^{Ll}$. Which are all comparable to the values found for $MseL^{Ec}$. Figure 37 shows traces of $MscL^{Ll}$ reconstituted into different lipid compositions. It can be seen that the initial full openings occur at different pressures in the different liposome compositions.

In vitro release profiles of a model drug from proteoliposomes

Fig.38 shows the release of calcein in response to an osmotic shock in proteoliposomes containing $MscL^{Ll}$. The results of patch clamp and the calcein release assay show that this MscL homologue can be used to deliver

substances from liposomes as described for MscL from *E.coli* and derivatives thereof.

[1]. W. Schaffner and C. Weissmann. A rapid, sensitive, and specific method for the determination of protein in dilute solution. Anal.Biochem. 56 (2):502-514, 1973.

[2]. R. H. Friesen, J. Knol, and B. Poolman. Quaternary structure of the lactose transport protein of Streptococcus thermophilus in the detergent-solubilized and membrane-reconstituted state. J.Biol.Chem. 275 (43):33527-33535, 2000.

[3]. P. Blount, S. I. Sukharev, P. C. Moe, B. Martinac, and C. Kung. Mechanosensitive channels of bacteria. Methods Enzymol. 294:458-482, 1999.

**Example 8**

Title: Controlled release of Insulin from liposomes containing MscL Mutant G22C.

The present invention provides a method for obtaining controlled release of hydrophilic drugs from liposomes. For practical reasons, either calcein release or ion fluxes are monitored to functionally characterize the delivery system. This example shows that the observed principles in the previous examples also apply to therapeutically relevant hydrophilic molecules. Additionally, the applied filter-binding assay can be used to test the controlled release of many different substances from these delivery vehicles.

Materials and Methods

DOPC:DOPS (9:1 mol/mol) liposomes containing the G22C MscL were prepared as described in example 2. Insulin and FITC were obtained from Sigma (St. Louis, MO, USA). Insulin (23 mg) was reacted with a fourfold molar ratio of FITC in 0.1 N borate buffer pH 9.0 for 60 min. The pH was lowered to 7.5 with 0.1 N boric acid and the solution was extensively dialyzed, using dialysis membrane with molecular weight cutoff of 2,000 Da, for 96 hours against water at 4 ˚C with frequent water changes. Absorption spectra of the dialysed sample was used to quantify the protein concentration and the stoichiometry of labelling. Concentrations of fluoresceine and insulin were both 0.1mM. The labelled insulin was encapsulated by three freeze thaw cycles, followed by extrusion through a 400 nm polycarbonate membrane. The proteoliposomes containing labelled insulin were separated from free labelled insulin by using sephadex G-50 column chromatography equilibrated with 145 mM NaCl, 300 mM Sucrose, 25 mM Tris.HCl and 1 mM EDTA pH 8.0.

Proteoliposomes were prepared as described in example 2 and MTSET is used for opening of the MscL mutant G22C channels. Samples were taken at different time points and triton was added as a control for maximum fluorescence (100 %). Samples were filtered over a 450 nm Cellulose Nitrate filter (Schleicher & Schuell BA85). The filtrate of 2 ml was retained and the fluorescence of 200 μl of each filtrate was monitored in a fl600 platereader (Bio-Tek). All experiments were performed in triplo.

Results and Discussion

Figure 39 shows the release of FITC-insulin through MscL mutant G22C upon activation with 1 mM MTSET. The difference between filtered and unfiltered conditions is the amount of FITC-insulin encapsulated in the proteoliposomes. The fluorescence of the unfiltered condition with and without Triton X-100 indicates that the concentration of FITC-insulin in the proteoliposomes exhibit self-quenching. With and without MTSET are control conditions for the effect of MTSET on the FITC-insulin efflux, to show that FITC-insulin efflux is indeed MscL mediated.

The mass of FITC-insulin is approximately 6,100 Da and considerably higher compared to calcein. Therefore, this examples shows the applicabillity of this delivery sytem for therapeutic macromolecules molecules. This filter assay can also be used for monitoring the controlled release of other labelled drug molecules from proteoliposomes.

FIGURE LEGENDS

Figure 1. SDS-PAGE stained with Coomassie Brilliant Blue (molecular weight markers indicated in kDa on the left of lane A, and purified detergent-solubilized MscL in lane B), and the Western blot (lane C).

Figure 2. Electrospray ionization mass spectrometry of G22C-MscL-6His and its MTSES conjugate. (solid line)

Spectrum of G22C- MscL-6His. Not all peptides that are present in the sample are indicated in the spectrum. (broken line) Spectrum of MTSES conjugated G22C-MscL-6His. The masses are indicated at the peaks and show that all proteins are conjugated

Figure 3. Equilibrium centrifugation on sucrose gradients of proteoliposomes. 6His-MscL purified with Triton X-100 and incorporated in liposomes titrated with 4.0 mM Triton X-100 (Rsat; open squares) and liposomes titrated with 10.0 mM Triton X-100 (Rsol; closed squares). After centrifugation, the gradients were fractionated (0.5 mL) and assayed for the presence of lipids and protein. All protein, as determined by Western blotting as shown in the inset, is shown to be associated with the lipids, as determined by measuring fluorescence (AU) of $R_{18}$.

Figure 4. Freeze-fracture image of proteoliposome showing the MscL channel protein as a transmembrane vesicle (white box).

Figure 5. Patch-clamp recordings of channel activities at -20 mV from MscL reconstituted into liposomes of different lipid compositions as indicated in the figures. Pressure in the pipette, relative to atmospheric, is shown in the lower traces, and recording of the current through a patch of membrane excised from a blister is shown in the upper traces.

Figure 6. Pressure dependence of the MscL channel reconstituted in liposomes of different lipid composition. Open probability in the patch of a membrane with a lipid composition of PC: PS, 90:10 m/m (A) and PC: PE, 70:30, m/m (B) versus the applied pressure. Smooth curves are Boltzman fits.

Figure 7. Calcein efflux from liposomes with (closed circles) and without (closed squares) MscL as a function of a decrease in Osmolality of the external medium. A small volume (typically 20 μL) containing (proteo)liposomes in iso-osmotic buffer, is rapidly diluted with buffer of decreasing osmolality and calcein release was determined by dividing the fluorescence at 100 sec after dilution by the total fluorescence obtained after Triton X-100 lysis.

Figure 8. Calcein release under iso-osmotic condition mediated by conjugated G22C-MscL-6His. Calcein release of MTSES conjugated G22C-MscL-6His channel protein reconstituted into liposomes (PC:Chol, 60:40, m/m) (closed circles). Liposomes with the same lipid composition and sample treatment as above but without MscL (closed squares).

Figure 9. (not being part of the invention as claimed) Effect of 5 mol% DGPE-PEG(2000) on the calcein release from liposomes (PC:Chol, 60:40, m/m). Calcein release from PC:Chol:DGPE-PEG(2000) liposomes in the presence of buffer (closed triangles), rat plasma (closed circles), human plasma (closed squares). Calcein release from liposomes without DGPE-PEG(2000) (closed diamond).

Fig. 10. Patch clamp recordings of MscL channel activities at +20 mV in spheroplasts. Pressure in the pipette, relative to atmospheric, is shown in the lower traces (A and B), and recordings of the current through a cell attached patch of a spheroplast are shown in the upper traces (A and B). Fig. A shows results of MscL mutant G22C in spheroplast before MTSET attachment, and Fig. B is the same as A but after MTSET attachment. Buffer: 200mMKCl, 90 mM MgCl2,10mMCaCl2, 5 mM HEPES, pH 6.0. Figures C and D show the histograms of the conductivity preferences of A and B, respectively.

Fig. 11. Calcein efflux from liposomes (DOPC:DOPS, 90:10, mol/mol) with MscL mutant G22C (protein to lipid, 1: 20 wt/wt). 1 mM MTSET, 2.5 mM MTSEA, and 10 mM MTSES was added at time indicated by the arrow.

Fig. 12 Calcein efflux from liposomes (DOPC:Cholesterol, 80:20, mol/mol) with MscL mutant G22C (closed squares) and without MscL mutant G22C (closed circles).

Fig.13. ESI-MS analysis of the IMI conjugation to a single cysteine mutant of MscL at position 22. Unconjugated G22C-MscL with expected mass of 15697 Da (closed squares). IMI conjugated G22C-MscL with a 156 Da mass increase (closed triangles).

Figure 14. Patch clamp recordings of imidazole coupled and uncoupled MscL mutant G22C channels as shown in B and A, respectively. 5μl of G22C spheroplasts were incubated with IMI (2 mM final concentration) or with patch buffer overnight at 4˚C. The next day, currents through cell attached patches held at +20 mV were recorded for unlabelled (Fig. A) and labelled (Fig.B) proteins and histograms showing the conductance states of each recording is given in Fig. C and Fig. D, respectively.

Fig. 15. Different pKa's of substituents for MscL mutant G22C labels.

Figure 16 Patch clamp recordings of patches excised from proteoliposomes containing BP coupled and uncoupled MscL mutant G22C channels. Fig. A shows the labelled channel behaviour at pH 7.2 and the histogram showing the conductance levels is given in Fig B. Fig C and E shows the behaviour of unlabelled MscL channel at pH 5.2, respectively. Fig. D and F shows that of the labelled channel at pH 5.2 and their conductance histograms are given in Fig. G for the unlabelled and H for the BP labelled MscL channel, respectively. Measurements were performed with +20 mV constant voltage.

Fig. 17. Structure of DTCP1 in the open state (A) and in the closed state (B). The molecule can reversibly isomerize depending on the wavelength of the absorbed light.

Fig. 18. ESI-MS analysis of the DTCP1 conjugation to a single cysteine mutant of MscL at position 22. Unconjugated G22C-MscL with expected mass of 15697 Da (closed squares). DTCP1 conjugated G22C-MscL with a 344 Da mass increase (closed triangles).

Fig. 19. Absorption spectra of DTCP1. Open isomer A has a maximum at 260 nm and no absorbance at higher vawelenghts than 400 nm, closed isomer B has very distinct peak vith maximum at 535 nm. Gray line shows substacted spectra of open and closed isomer.

Fig. 20. Substracted spectra of open and closed isomer of DTCP1 after conjugation to G22C-MscL and reconstitution in DOPC:DOPS (90:10, mol/mol) lipid bilayer

Fig. 21. Four switching cycles of DTCP1 conjugated to MscL and reconstituted in lipid bilayer.

Fig. 22. Photochromic molecule SP1 in its spiropyran form (left) and merocyanine zwitterionic form (right).

Fig 23. Absorption spectrum of SP1 conjugated to MscL in spiropyran form SP1 and after irrediation in highly charged merocyanine form MC1

Fig 24. Reversible switching between spiropyran (SP) and merocyanine (MC) form by alternating irradiation with UV and visible light.

Fig. 25. Structure of sodium di(C4azo-O-C6)-phosphate in the trans and cis conformation

Fig. 26. UV/Vis spectra of sodium di(C4azobenzene-O-C6)-phosphate (7) in the trans and cis state. The molar ratio of DSP to sodium di(C4azobenzene-O-C6)-phosphate is 95:5. Concentration of sodium di(C4azobenzene-O-C6)-phosphate is 12,5 pM.

Fig. 27. Repeated cycles of the isomerization of lipid 6 in a vesicle which composes of 95% DOPC and 5% lipid 6. For the trans configuration the absorbance at 349 nm is given and for the cis configuration the absorbance at 313 nm is given.

Fig. 28. UV/Vis spectra of of lipid 6 in a vesicle which composes of 95% DOPC and 5% lipid 6. The times indicated are the irradiation times. The sample was irradiated with 365 nm light.

Fig. 29. DSC graphs of pure DSP and a mixture of DSP and sodium di(azbezeneo-O-C9)-phosphate (molar ratio 95/5).

Fig. 30.. Urinary excretion of MAG3 after subcutaneous or intravenous injection. Free MAG3 intravenously (open circle, right y-axis), free MAG3 subcutaneously (closed circle), MAG3 in 'empty' liposomes subcutaneously (open squares), MAG3 in G22C-MscL containing liposomes subcutaneously (closed squares).

Fig. 31 Subcutaneous pH-reduction. MES buffer (0.5 ml, pH 6.1) of different molarities was injected subcutaneously in conscious rats.

Fig. 32 Urinary excretion of IOT after subcutaneous injection. Free IOT (open squares), IOT in DOPC/PS liposomes (closed squares), IOT in DOPC/PE liposomes (closed triangles)

Fig.33. SDS-PAGE gel stained with Coomassie Brilliant Blue. Lane A: vesicles containing the overexpressed protein, lane B: molecular weight marker, lane C: purified protein

Fig. 34. Freeze-fracture image of proteoliposome showing the MscL channel protein as a transmembrane particle (white box).

Fig. 35. A typical trace of channel activity of $MscL^{Ll}$ in $MscL^{Ec-}$/$MscS^{Ec+}$ *E. coli* cells. The upper trace shows the current across the membrane due to channel activity. Flow of current is shown upward in all traces. From left to right, in time, first two channels of small conductivity open (also shown in enlarged left panel) and later the opening of a single $MscL^{Ll}$ is shown (also shown in enlarged left panel). The lower trace indicates the pressure applied to the membrane. The panels show enlargements of the upper trace.

Fig. 36. Top panel shows the dependence of opening chance of the $MscL^{Ec}$ channel on the applied pressure in the pipette. The sigmoidal curve shows that no channels open at 0mmHg pressure and that all channels are open all the time at 90mmHg. Centre panel shows the time channels spend in the open state. The bars indicate the distribution of opening times of the *L. lactis* MscL (<0.1ms and 0.7ms). Unfortunately resolution of the traces doesn't allow analysis on a shorter time scale. Bottom panel shows relationship between voltage and current through the channel. The slope of this graph is the conductivity of the channel, which is 2.5nS.

Fig. 37. Electrophysiological analysis of $MscL^{Ll}$ in left panel: PC:Cholesterol 8:2 mol/mol, right panel: PC:PS 9:1. Protein:lipid ratio in both cases is 1:1000

Fig.38. Calcein release from PC:PS (9:1) proteoliposomes containing $MscL^{Ll}$ (protein:lipid 1:500) or not containing any protein after dilution of the isoosmotic buffer, with dH2O to indicated dillutions. It can be seen that the protein containing liposomes release more calcein than the liposomes without protein. This is $MscL^{Ll}$ mediated efflux of the calcein.

Fig. 39. Efflux of FITC-insulin under different conditions from DOPC:DOPS (9:1, mol/mol) liposomes containing MscL mutant G22C. Not filtered (200 μl proteoliposomes); not filtered after triton (200 μl proteoliposomes with Triton X-100); filtered (200 μl after filtration);5' or 10' + MTSET (200 μl proteoliposomes incubated with 1 mM MTSET for 5' or 10' followed by filtration; 10' - MTSET (200 μl proteoliposomes incubated 10' without MTSET followed by filtration; Triton (200 μl proteoliposomes with Triton X-100 followed by filtration).

REFERENCES

1. Arkin, I.T., Sukharev, S.I., Blount, P., Kung, C., and Brünger A.T. (1998) Biochim. Biophys. Acta 1369, 131-140.
2. Delcour, A.H., Martinac, B., Adler, J., and Kung, C. (1989) Biophys. J. 56, 631-636.
3. Blount, P., Sukharev, S.I., Moe, P.C., Schroeder, M.J., Guy, H.R., and Kung, C. (1996) EMBO J. 15, 4798-4805
4. Van Montfort, B.A., Cana, B., Duurkens, R., Godovac-Zimmermann, J., and Robillard, G.T. submitted
5. Hirel, A.U., Schmitter, M.J., Dessen, P., Fayat, G., and Blanquet, S. (1989) Proc. Natl. Acad. Sci. U. S.A. 86, 8247-8251
6. Knol, J., Sjollema, and Poolman, B. (1998) Biochemistry 37, 16410-16415
7. Friesen, R.H.E., Knol, J., Poolman, B. (2000) J. Biol. Chem. 43, 33527-33535
8. Moe, P.C., Blount, P. and Kung, C. (1998) Mol. Microbiol. 28, 583-592
9. Yoshimura, K., Batiza, A., Schroeder, M., Blount P., and Kung, C. (1999) Biophys. J. 77, 1960-1972
10. Song, X., Perlstein, J., and Whitten, D.G. (1997) J. Am. Chem. Soc. 119, 9144-9159
11. Blount, P., Sukharev, S.I., Schroeder, M.J., Nagle, S.K., and Kung, C. (1996) Proc. Natl. Acad. Sci USA 93, 11652-11657
12. Mayer, L.D., Hope, M.J., and Cullis, P.R. (1986) Biochim. Biophys. Acta 858, 161-168
13. Martinac, B., Adler, J., and Kung, C. (1990) Nature 348, 261-263
14. Ou, X., Blount, P., Hoffman, R.J., and Kung C. (1998) Proc. Natl. Acad. Sci. USA 95, 11471-11475
15. Damen J., Regts, J., and Scherphof G. (1981) Biochim. Bioph. Acta 665, 538-545
16. Yoshimura, K, Batiza and Kung, C. (2001) Biophysical Journal (80) 2198-2206.

## Claims

1. A composition for use as medicament, the composition comprising a lipid vesicle comprising a mechanosensitive proteinaceous channel and a small hydrophilic molecule, wherein said lipid vesicle and/or said proteinaceous channel is formulated such that said proteinaceous channel is in the open state in the vicinity of a target cell, and wherein said small hydrophilic molecule is selected from the group consisting of peptides and biologically active molecules.

2. A composition according to claim 1, wherein said mechanosensitive proteinaceous channel is a mechanosensitive channel of large conductance (MscL).

3. A composition according to claim 2, wherein said MscL is a mutant MscL.

4. A composition according to any one of claims 1 to 3, wherein said small hydrophilic molecule is selected from the group consisting of cytostatic drugs and small therapeutic peptides.

5. A composition according to any one of the above claims, wherein said lipid vesicle comprises an asymmetrical bilayer.

6. A composition according to any one of the above claims, wherein said lipid vesicle comprises a light sensitive lipid and/or MscL.

7. A composition according to any one of the above claims, wherein said lipid vesicle comprises positively charged and/or neutral lipids.

8. A composition according to any of the above claims, wherein said lipid vesicle does not comprise a negatively charged lipid.

9. A composition according to any one of the above claims, wherein said lipid vesicle further comprises a non-channel protein.

10. A composition according to claim 9, wherein said non-channel protein is a binding molecule capable of binding to a binding partner on said target cell, thereby enabling at least a prolonged stay of said vesicle near said target cell.

11. A composition according to any one of claims 1 to 10, formulated and prepared for use in a human.

12. Composition according to claim 11, wherein the small hydrophilic molecule is a drug and wherein the composition is formulated for triggered or sustained drug release.

13. Use of a lipid vesicle comprising a mechanosensitive channel of large conductance (MscL), for the preparation of a composition comprising a small hydrophilic molecule selected from the group consisting of peptides and biologically active molecules, for controlling delivery of said small hydrophilic molecule to a target tissue in a body.

14. Use of a composition according to claim 6, and claims depending thereon, wherein said lipid vesicle comprises a light sensitive protein channel, for the manufacture of a medicament for patient-controlled treatment of pain or diabetes.

15. A composition comprising a lipid vesicle, the vesicle comprising a mechanosensitive proteinaceous channel and a small hydrophilic molecule, wherein said lipid vesicle and/or said proteinaceous channel is formulated such that said proteinaceous channel is in the open state in the vicinity of a target cell, and wherein said small hydrophilic molecule is selected from the group consisting of interleukins, diphtheria, diphtheria fragment, muramyl dipeptide, cis-4-hydroproline, cisplatin, cisplatin derivatives, cytosine arabinose, phosphonopeptides, β-glucuronidase, cytostatic drugs, small therapeutic proteins and peptides.

16. Use of a composition according to claim 15 for delivering a small hydrophilic molecule to a biological system for a non-medical purpose.

17. An *in vitro* method for delivering a small hydrophilic molecule to a cell, comprising loading a lipid vesicle with a small hydrophilic molecule selected from the group consisting of peptides and biologically active molecules, and administering said vesicle to fluid that is in contact with said cell, said vesicle further comprising a mechanosensitive

proteinaceous channel that in the open state allows passage of said small molecule to the exterior of said vesicle, said lipid vesicle and/or said proteinaceous channel formulated such that said channel is in the open state in the vicinity of said cell.

**18.** A method according to claim 17, wherein opening of said channel is triggered upon the availability of a signal, preferably wherein said signal is light, a chemical compound, an altered pH, an altered temperature and /or an alteration in the redox potential.

**19.** A method according to claim 18, wherein said signal is a pH is equal or less than 6.5.

**Patentansprüche**

**1.** Zusammensetzung zur Verwendung als Medikament, umfassend ein Lipidvesikel, das einen mechanosensitiven proteinartigen Kanal und ein kleines hydrophiles Molekül umfasst, wobei das Lipidvesikel und/oder der proteinartige Kanal so gestaltet ist/sind, dass der proteinartige Kanal in der Nachbarschaft einer Zielzelle in geöffnetem Zustand ist, und wobei das kleine hydrophile Molekül ausgewählt ist aus der Gruppe bestehend aus Peptiden und biologisch aktiven Molekülen.

**2.** Zusammensetzung nach Anspruch 1, wobei der mechanosensitive proteinartige Kanal ein mechanosensitiver Kanal mit hoher Leitfähigkeit (MscL) ist.

**3.** Zusammensetzung nach Anspruch 2, wobei der MscL ein mutierter MscL ist.

**4.** Zusammensetzung nach einem der Ansprüche 1 bis 3, wobei das kleine hydrophile Molekül ausgewählt ist aus der Gruppe bestehend aus cytostatischen Arzneistoffen und kleinen therapeutischen Peptiden.

**5.** Zusammensetzung nach einem der vorhergehenden Ansprüche, wobei das Lipidvesikel eine asymmetrische Doppelschicht umfasst.

**6.** Zusammenfassung nach einem der vorhergehenden Ansprüche, wobei das Lipidvesikel ein lichtsensitives Lipid und/oder MscL umfasst.

**7.** Zusammensetzung nach einem der vorhergehenden Ansprüche, wobei das Lipidvesikel positiv geladene und/oder neutrale Lipide umfasst.

**8.** Zusammensetzung nach einem der vorhergehenden Ansprüche, wobei das Lipidvesikel kein negativ geladenes Lipid umfasst.

**9.** Zusammensetzung nach einem der vorhergehenden Ansprüche, wobei das Lipidvesikel weiter ein Nicht-Kanalprotein umfasst.

**10.** Zusammensetzung nach Anspruch 9, wobei das Nicht-Kanalprotein ein Bindemolekül ist, das fähig ist, an einen Bindungspartner auf der Zielzelle zu binden, wobei mindestens ein verlängerter Verbleib des Vesikels in der Nähe der Zielzelle erreicht wird.

**11.** Zusammensetzung nach einem der Ansprüche 1 bis 10, formuliert und hergestellt zur Verwendung in einem Menschen.

**12.** Zusammensetzung nach Anspruch 11, wobei das kleine hydrophile Molekül ein Arzneistoff ist und wobei die Zusammensetzung für eine gezielt ausgelöste oder anhaltende Arzneistofffreisetzung formuliert ist.

**13.** Verwendung eines Lipidvesikels umfassend einen mechanosensitiven Kanal mit hoher Leitfähigkeit (MscL) zur Herstellung einer Zusammensetzung umfassend ein kleines hydrophiles Molekül ausgewählt aus der Gruppe bestehend aus Peptiden und biologisch aktiven Molekülen zum Kontrollieren der Zuführung des kleinen hydrophilen Moleküls zu einem Zielgewebe in einem Körper.

**14.** Verwendung einer Zusammensetzung nach Anspruch 6 und den davon abhängigen Ansprüchen, wobei das Lipidvesikel einen lichtempfindlichen Proteinkanal umfasst, für die Herstellung eines Medikaments zur patientenkontrollierten Behandlung von Schmerz oder Diabetes.

**15.** Zusammensetzung umfassend ein Lipidvesikel, wobei das Vesikel einen mechanosensitiven proteinartigen Kanal und ein kleines hydrophiles Molekül umfasst, wobei das Lipidvesikel und/oder der proteinartige Kanal so gestaltet ist/sind, dass der proteinartige Kanal in der Nachbarschaft einer Zielzelle in geöffnetem Zustand ist, und wobei das kleine hydrophile Molekül ausgewählt ist aus der Gruppe bestehend aus Interleukinen, Diphtherie, Diphtheriefragment, Muramyldipeptid, cis-4-Hydroxyprolin, Cisplatin, Cisplatinderivaten, Cytosinarabinose, Phosphonopeptiden, β-Glucuronidase, cytostatischen Arzneistoffen, kleinen therapeutischen Proteinen und Peptiden.

**16.** Verwendung einer Zusammensetzung nach Anspruch 15 zum Zuführen eines kleinen hydrophilen Moleküls zu einem biologischen System für einen nichtmedizinischen Zweck.

**17.** *In vitro*-Verfahren zum Zuführen eines kleinen hydrophilen Moleküls zu einer Zelle, umfassend das Beladen eines Lipidvesikels mit einem kleinen hydrophilen Molekül ausgewählt aus der Gruppe bestehend aus Peptiden und biologisch aktiven Molekülen, und Verabreichen des Vesikels an ein Fluid, das in Kontakt mit der Zelle ist, wobei das Vesikel weiter einen mechanosensitiven proteinartigen Kanal umfasst, der in geöffnetem Zustand den Durchtritt des kleinen Moleküls zur Außenseite des Vesikels erlaubt, wobei das Lipidvesikel und/oder der proteinartige Kanal so gestaltet ist, dass der Kanal in der Nachbarschaft der Zelle in geöffnetem Zustand ist.

**18.** Verfahren nach Anspruch 17, wobei die Öffnung des Kanals durch die Verfügbarkeit eines Signals ausgelöst wird, wobei das Signal vorzugsweise Licht, eine chemische Verbindung, ein geänderter pH-Wert, eine geänderte Temperatur und/oder eine Änderung im Redoxpotential ist.

**19.** Verfahren nach Anspruch 18, wobei das Signal ein pH-Wert ist, der gleich oder kleiner als 6,5 ist.

**Revendications**

**1.** Composition destinée à être utilisée comme médicament, la composition comprenant une vésicule lipidique qui comprend un canal protéinique mécanosensible et une petite molécule hydrophile, dans laquelle ladite vésicule lipidique et/ou ledit canal protéinique est formulé(e) de telle sorte que ledit canal protéinique se trouve à l'état ouvert au voisinage d'une cellule cible et dans laquelle ladite petite molécule hydrophile est choisie dans le groupe constitué par des peptides et des molécules biologiquement actives.

**2.** Composition selon la revendication 1, dans laquelle ledit canal protéinique mécanosensible est un canal mécanosensible à grande conductance (MscL).

**3.** Composition selon la revendication 2, dans laquelle ledit MscL est un MscL mutant.

**4.** Composition selon l'une quelconque des revendications 1 à 3, dans laquelle ladite petite molécule hydrophile est choisie dans le groupe constitué par des médicaments cytostatiques et des petits peptides thérapeutiques.

**5.** Composition selon l'une quelconque des revendications précédentes, dans laquelle ladite vésicule lipidique comprend une bicouche asymétrique.

**6.** Composition selon l'une quelconque des revendications précédentes, dans laquelle ladite vésicule lipidique comprend un lipide et/ou un MscL photosensible.

**7.** Composition selon l'une quelconque des revendications précédentes, dans laquelle ladite vésicule lipidique comprend des lipides chargés positivement et/ou neutres.

**8.** Composition selon l'une quelconque des revendications précédentes, dans laquelle ladite vésicule lipidique ne comprend pas de lipide chargé négativement.

**9.** Composition selon l'une quelconque des revendications précédentes, dans laquelle ladite vésicule lipidique comprend en plus une protéine non canalaire.

**10.** Composition selon la revendication 9, dans laquelle ladite protéine non canalaire est une molécule de liaison capable de se lier à un partenaire de liaison sur ladite cellule cible, permettant ainsi au moins un séjour prolongé de ladite vésicule au voisinage de ladite cellule cible.

**11.** Composition selon l'une quelconque des revendications 1 à 10, formulée et préparée pour une utilisation chez l'homme.

**12.** Composition selon la revendication 11, dans laquelle la petite molécule hydrophile est un médicament et dans laquelle la composition est formulée pour une libération de médicament déclenchée ou entretenue.

**13.** Utilisation d'une vésicule lipidique comprenant un canal mécanosensible à grande conductance (MscL) pour préparer une composition, comprenant une petite molécule hydrophile choisie dans le groupe constitué par des peptides et des molécules biologiquement actives, destinée à contrôler la délivrance de ladite petite molécule hydrophile dans un tissu cible dans un organisme.

**14.** Utilisation d'une composition selon la revendication 6 et les revendications dépendantes de celle-ci, dans laquelle ladite vésicule lipidique comprend un canal protéinique photosensible pour la fabrication d'un médicament destiné au traitement de la douleur ou du diabète contrôlé par le patient.

**15.** Composition comprenant une vésicule lipidique, la vésicule comprenant un canal protéinique mécanosensible et une petite molécule hydrophile, dans laquelle ladite vésicule lipidique et/ou ledit canal protéinique est formulé(e) de telle sorte que ledit canal protéinique se trouve à l'état ouvert au voisinage d'une cellule cible et dans laquelle ladite petite molécule hydrophile est choisie dans le groupe constitué par les interleukines, la toxine diphtérique, un fragment de toxine diphtérique, le dipeptide muramylique, la cis-4-hydroproline, le cisplatine, des dérivés du cisplatine, la cytosine arabinoside, des phosphonopeptides, la β-glucuronidase, des médicaments cytostatiques, des petites protéines thérapeutiques et des peptides.

**16.** Utilisation d'une composition selon la revendication 15 pour délivrer une petite molécule hydrophile dans un système biologique à des fins non médicales.

**17.** Procédé *in vitro* pour délivrer une petite molécule hydrophile dans une cellule, comprenant le chargement d'une vésicule lipidique avec une petite molécule hydrophile choisie dans le groupe constitué par des peptides et des molécules biologiquement actives, et administrer ladite vésicule dans un fluide qui est en contact avec ladite cellule, ladite vésicule comprenant en plus un canal protéinique mécanosensible qui, à l'état ouvert, permet le passage de ladite petite molécule à l'extérieur de ladite vésicule, ladite vésicule lipidique et/ou ledit canal protéinique étant formulé(e) de telle sorte que ledit canal se trouve à l'état ouvert au voisinage de ladite cellule.

**18.** Procédé selon la revendication 17, dans laquelle l'ouverture dudit canal est déclenchée par la présence d'un signal, ledit signal étant de préférence la lumière, un composé chimique, un pH modifié, une température modifiée et/ou une modification du potentiel oxydoréducteur.

**19.** Procédé selon la revendication 18, dans lequel ledit signal est un pH égal ou inférieur à 6,5.

Fig. 1

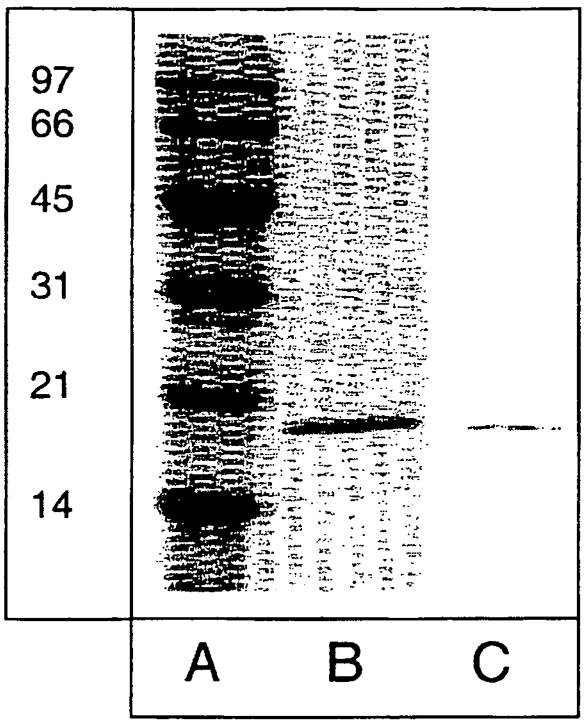

Fig. 2

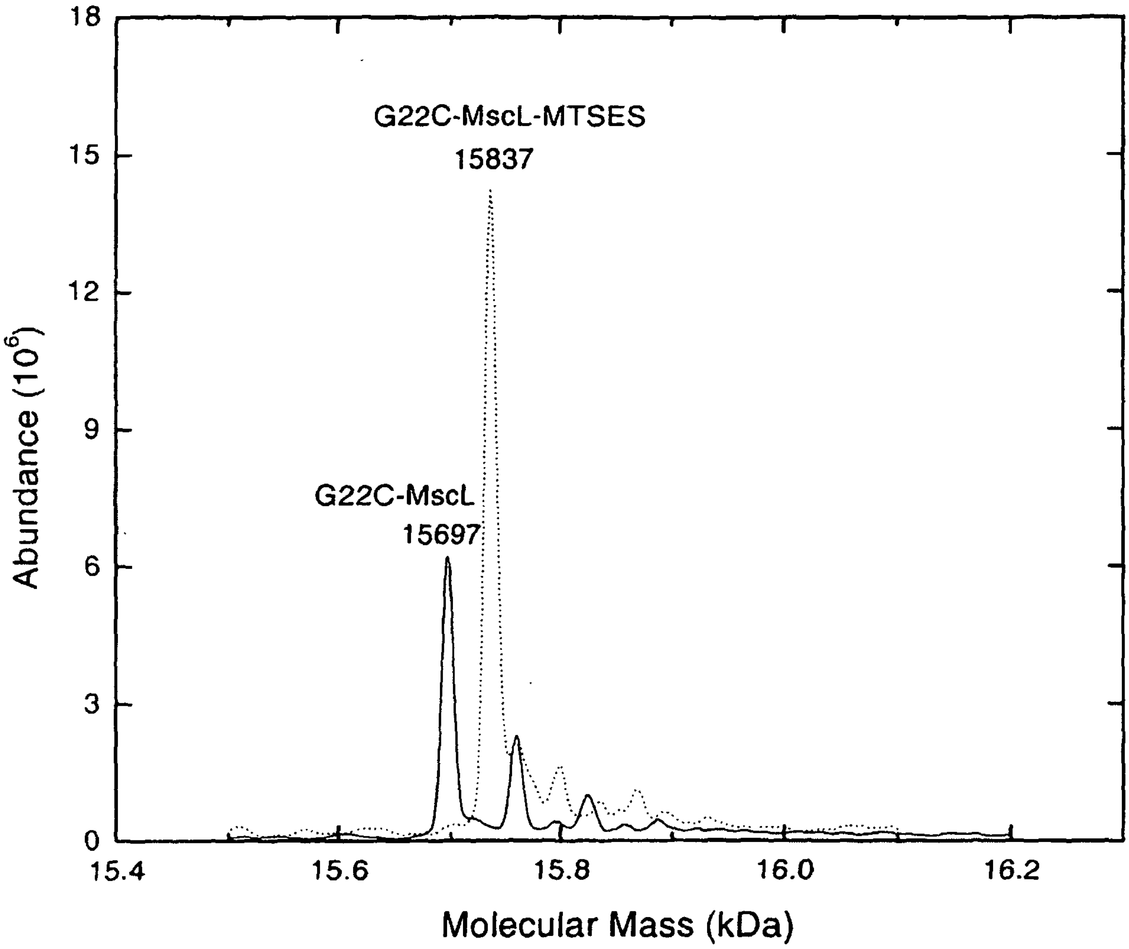
18
15
12
9
6
3
0
Abundance ($10^6$)
G22C-MscL-MTSES
15837
G22C-MscL
15697
15.4
15.6
15.8
16.0
16.2
Molecular Mass (kDa)

Fig. 3

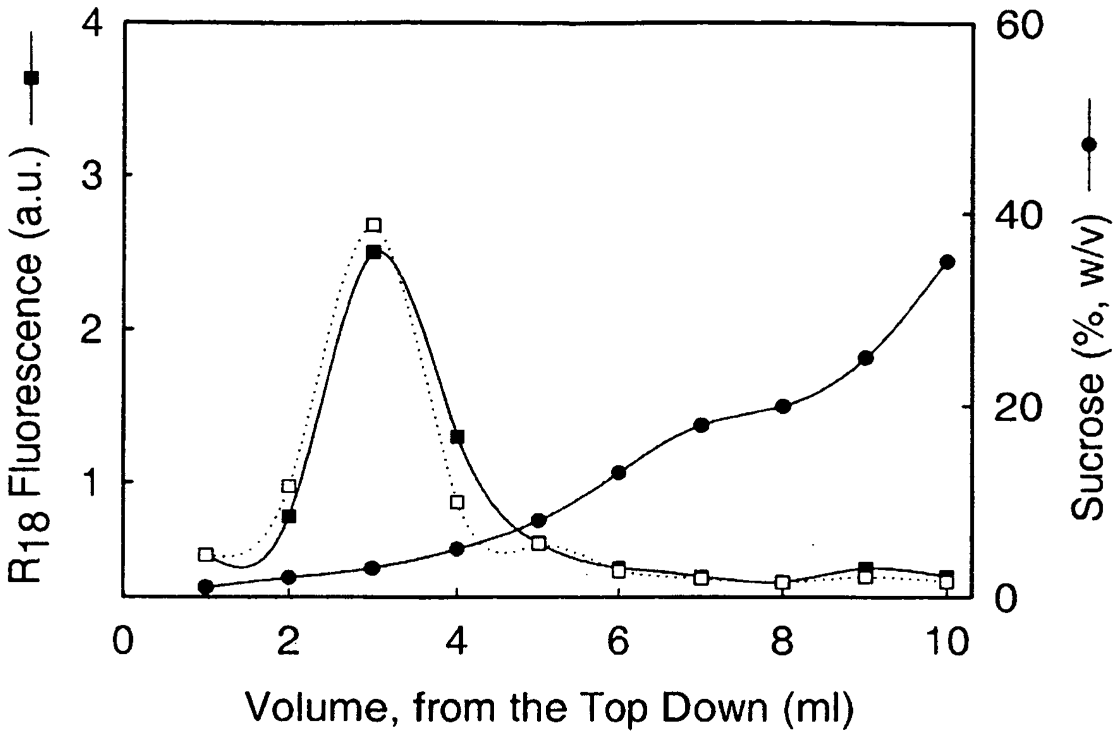
R18 Fluorescence (a.u.)
Sucrose (%, w/v)
Volume, from the Top Down (ml)
0
1
2
3
4
20
40
60
6
8
10

Fig. 4

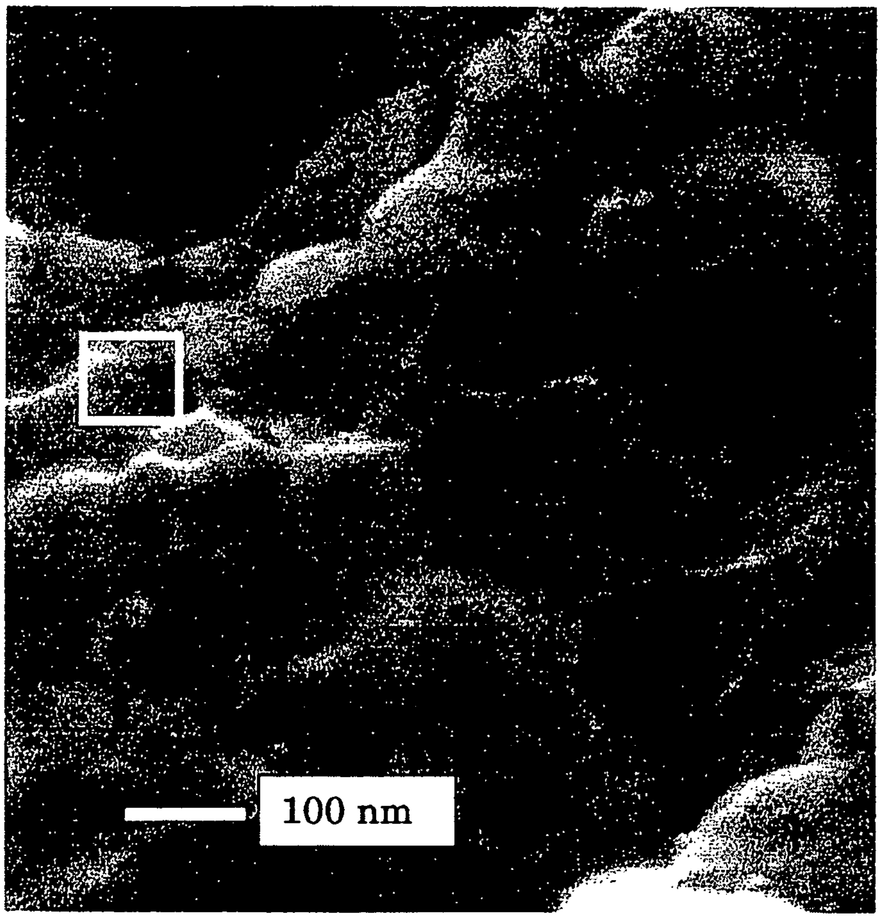

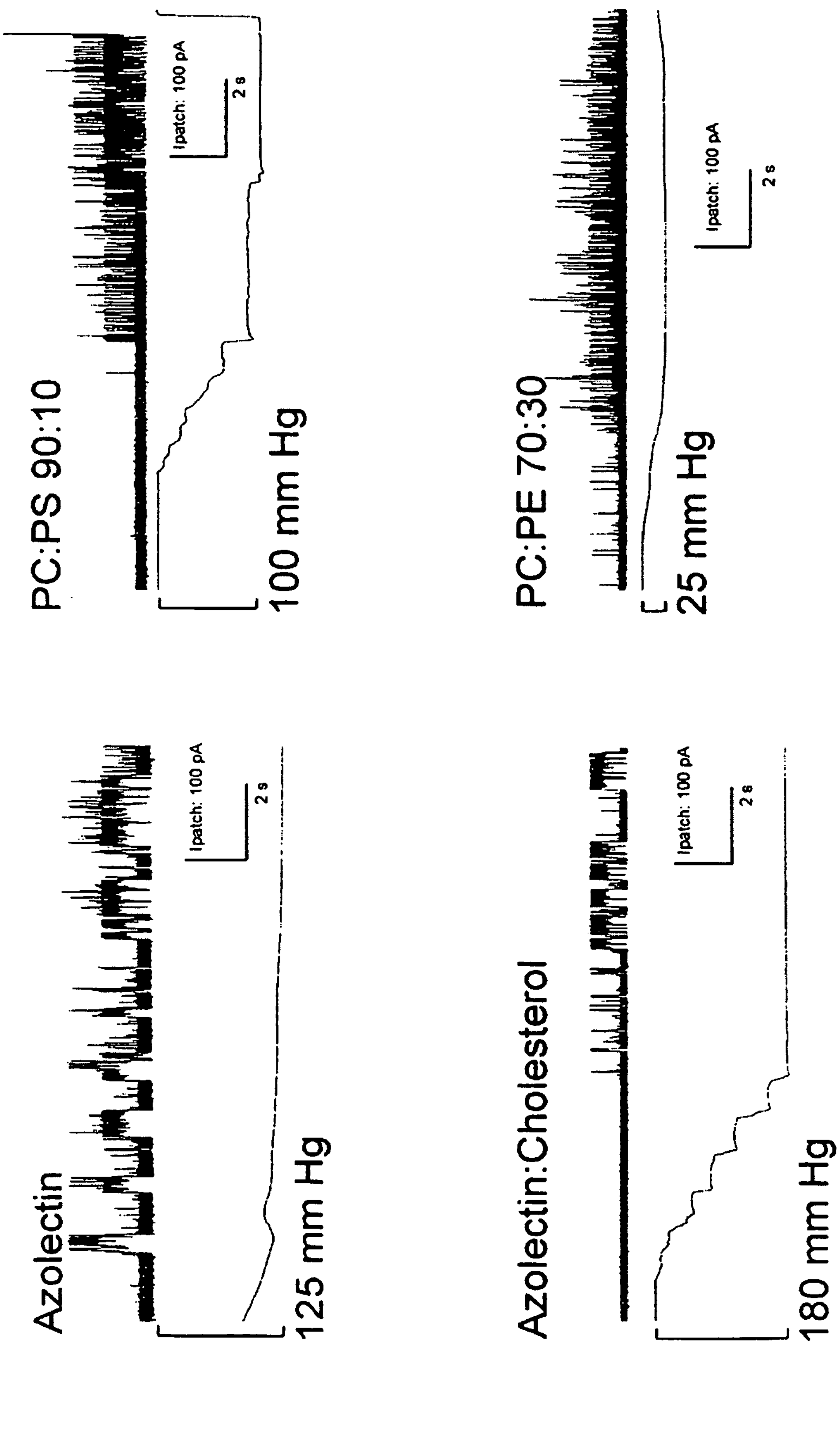
PC:PS 90:10
Ipatch: 100 pA
2 s
100 mm Hg
PC:PE 70:30
Ipatch: 100 pA
2 s
25 mm Hg
Azolectin
Ipatch: 100 pA
2 s
125 mm Hg
Azolectin:Cholesterol
Ipatch: 100 pA
2 s
180 mm Hg

Fig. 5

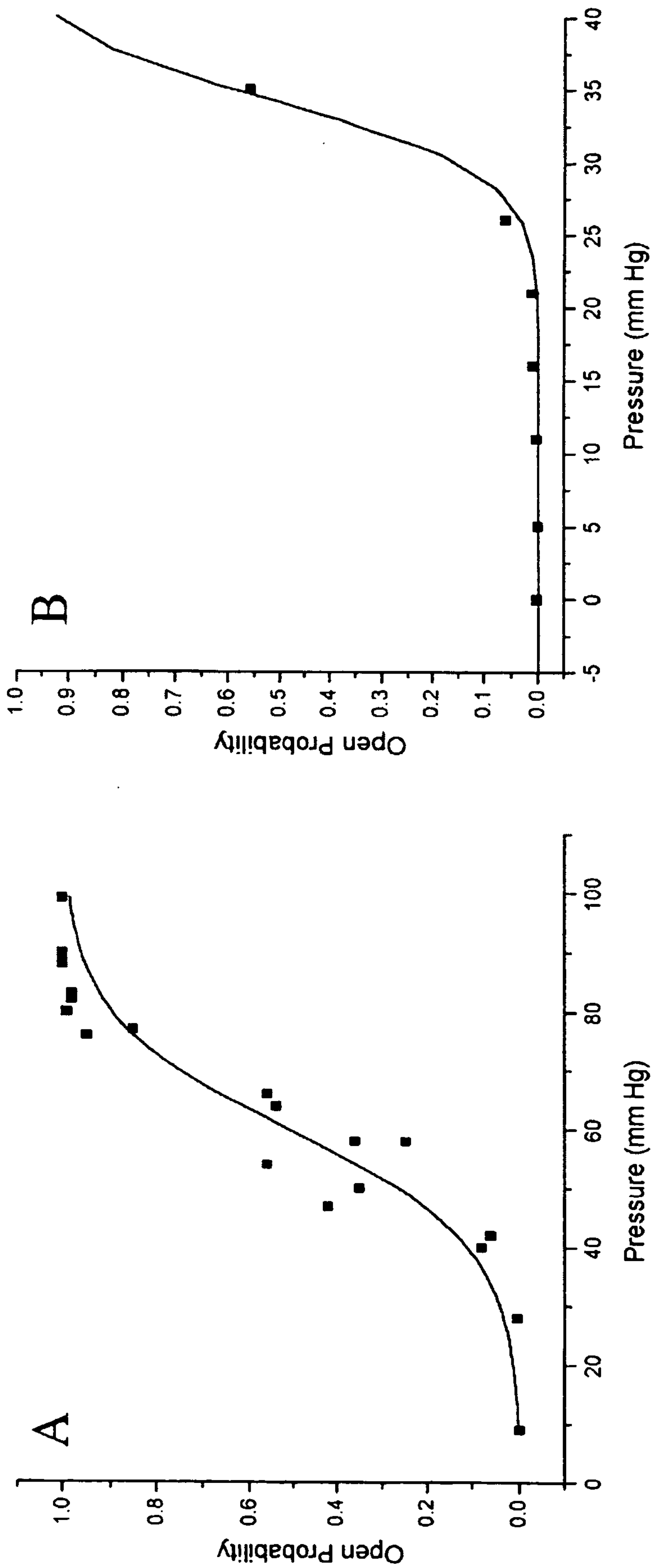
A
Open Probability
Pressure (mm Hg)
B
Open Probability
Pressure (mm Hg)

Fig. 6

Fig. 7

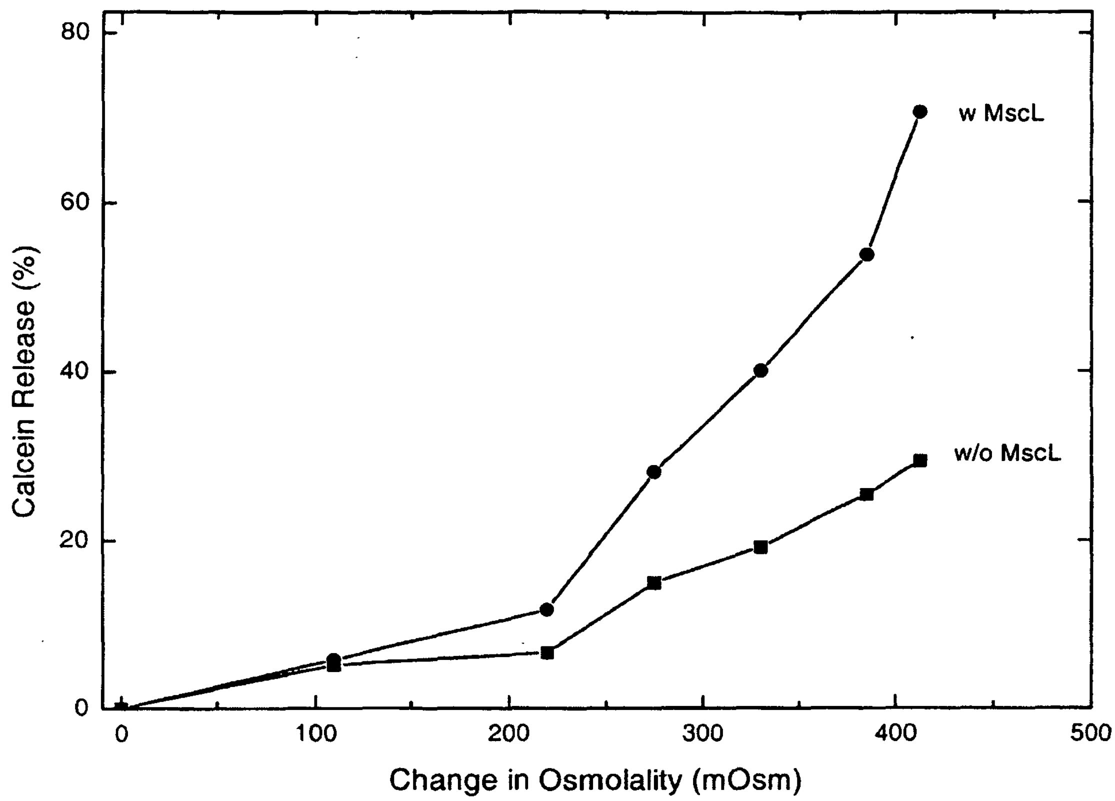
80
60
40
20
0
Calcein Release (%)
0
100
200
300
400
500
Change in Osmolality (mOsm)
w MscL
w/o MscL

Fig. 8

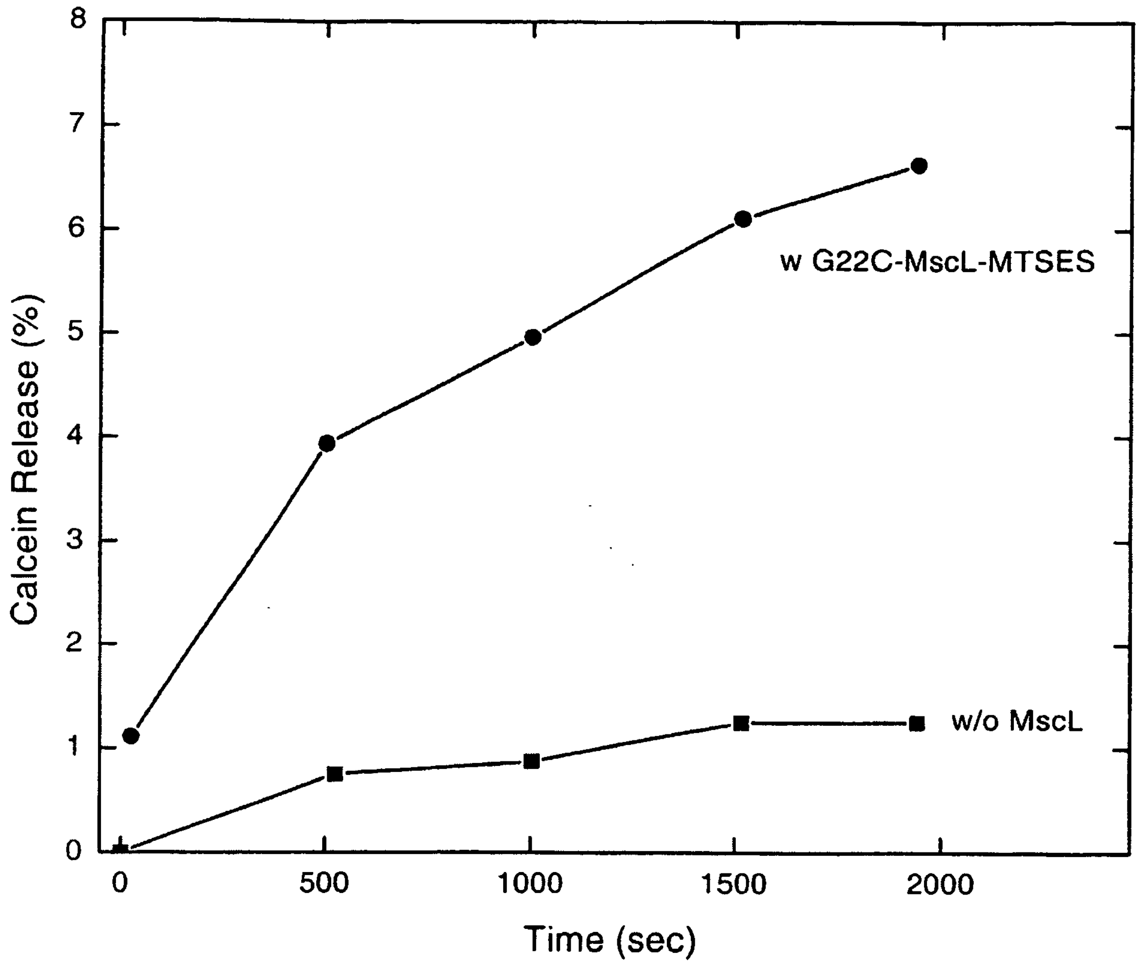
8
7
6
5
4
3
2
1
0
Calcein Release (%)
w G22C-MscL-MTSES
w/o MscL
0
500
1000
1500
2000
Time (sec)

Fig. 9

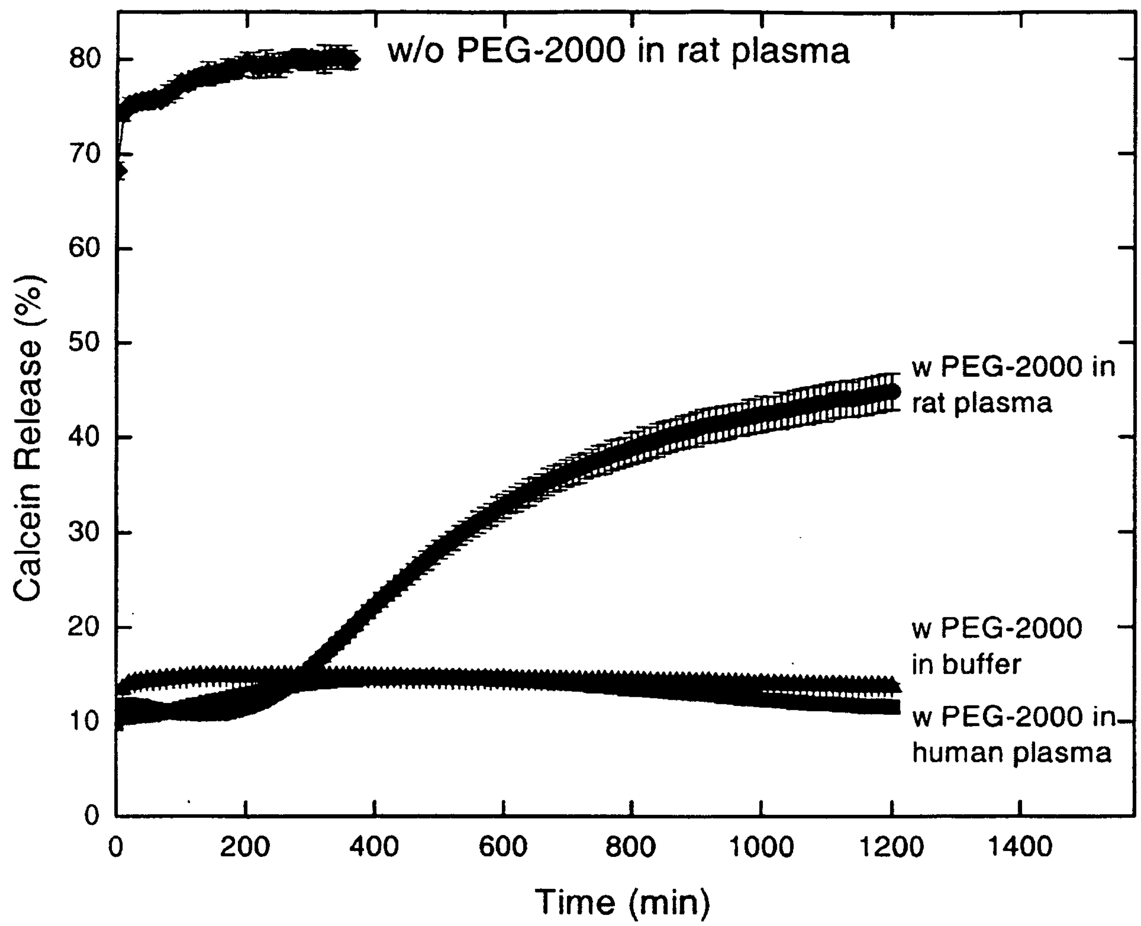
w/o PEG-2000 in rat plasma
w PEG-2000 in rat plasma
w PEG-2000 in buffer
w PEG-2000 in human plasma
Calcein Release (%)
Time (min)
0
10
20
30
40
50
60
70
80
0
200
400
600
800
1000
1200
1400

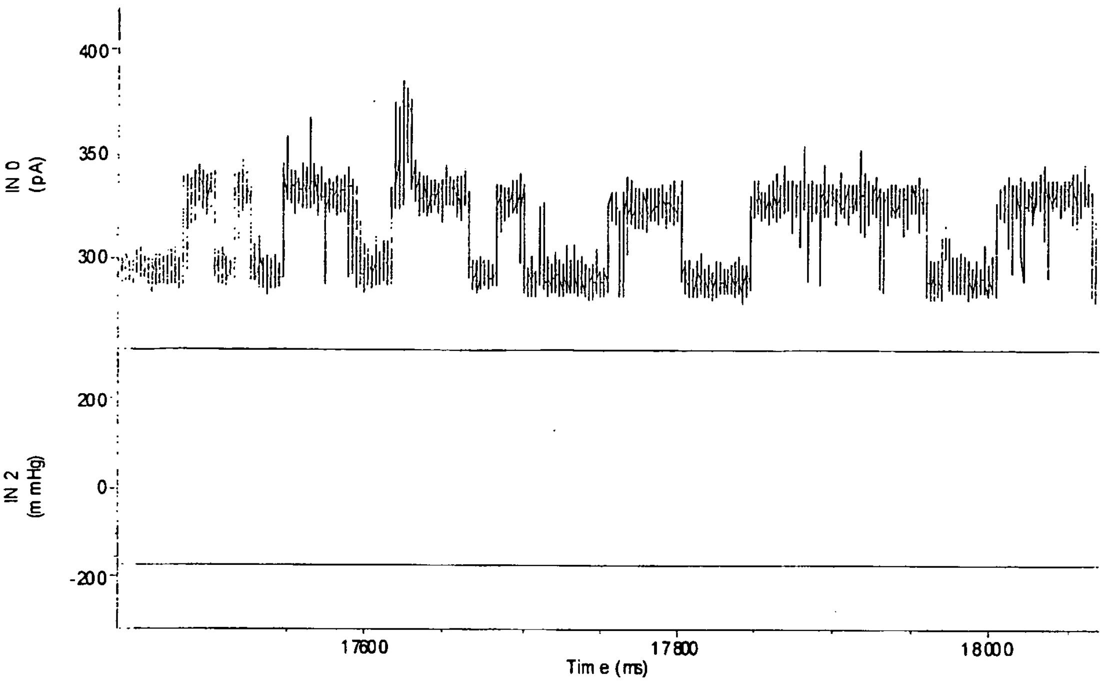


Fig. 10A

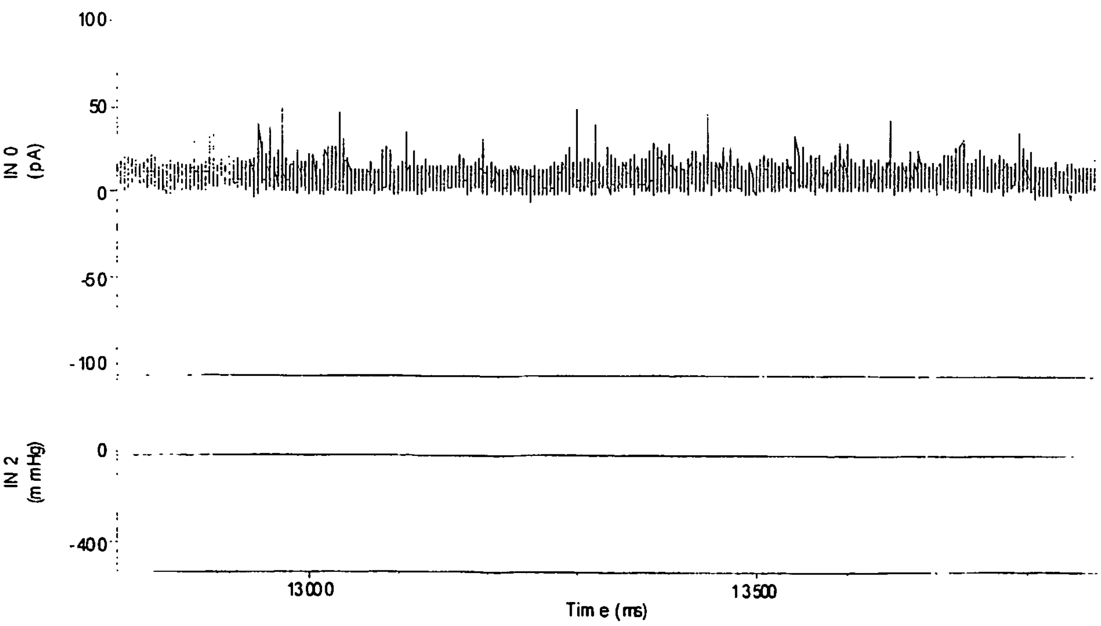


Fig. 10B

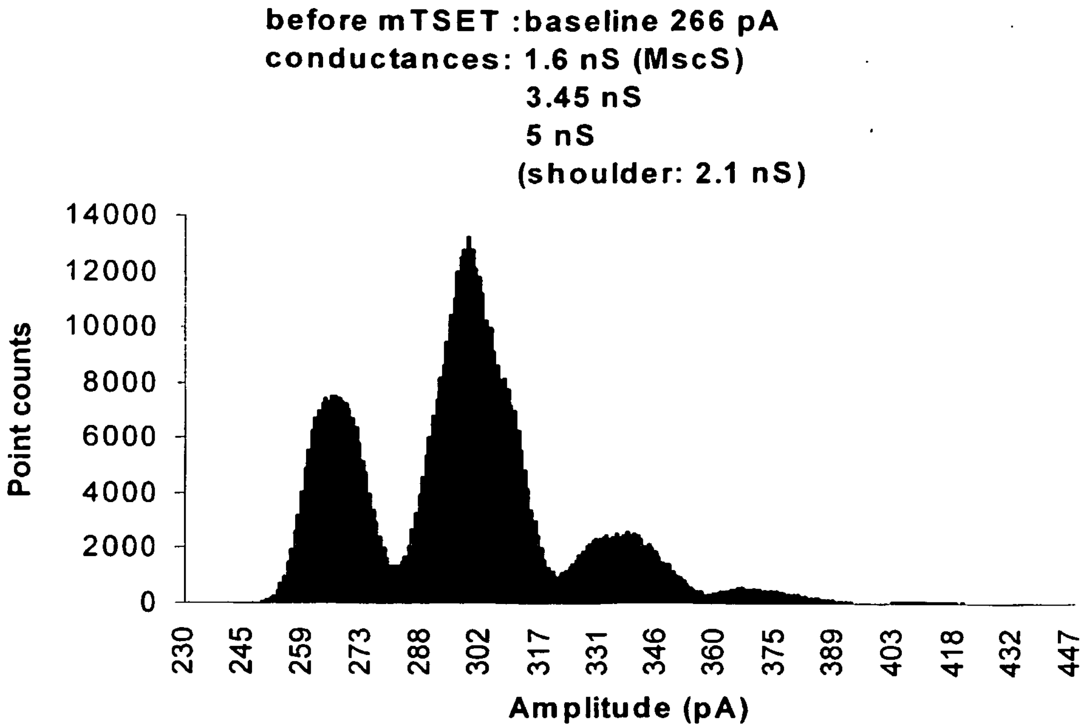


Fig. 10C

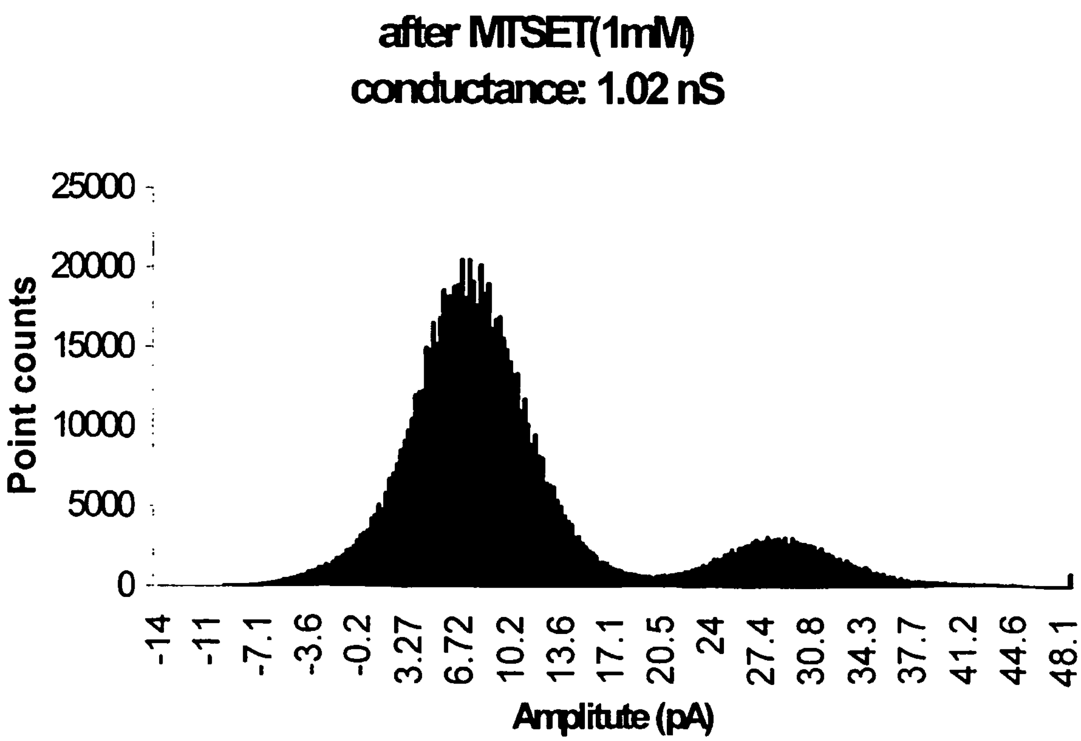


Fig. 10D

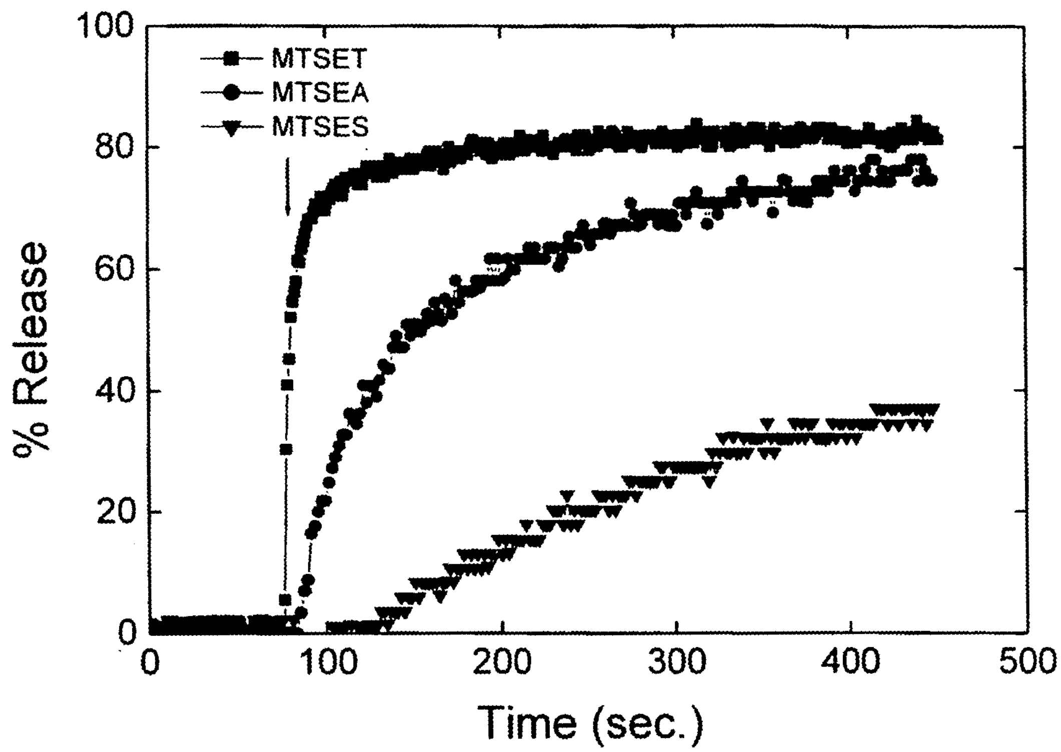
MTSET
MTSEA
MTSES
% Release
Time (sec.)
0
20
40
60
80
100
0
100
200
300
400
500

Fig. 11

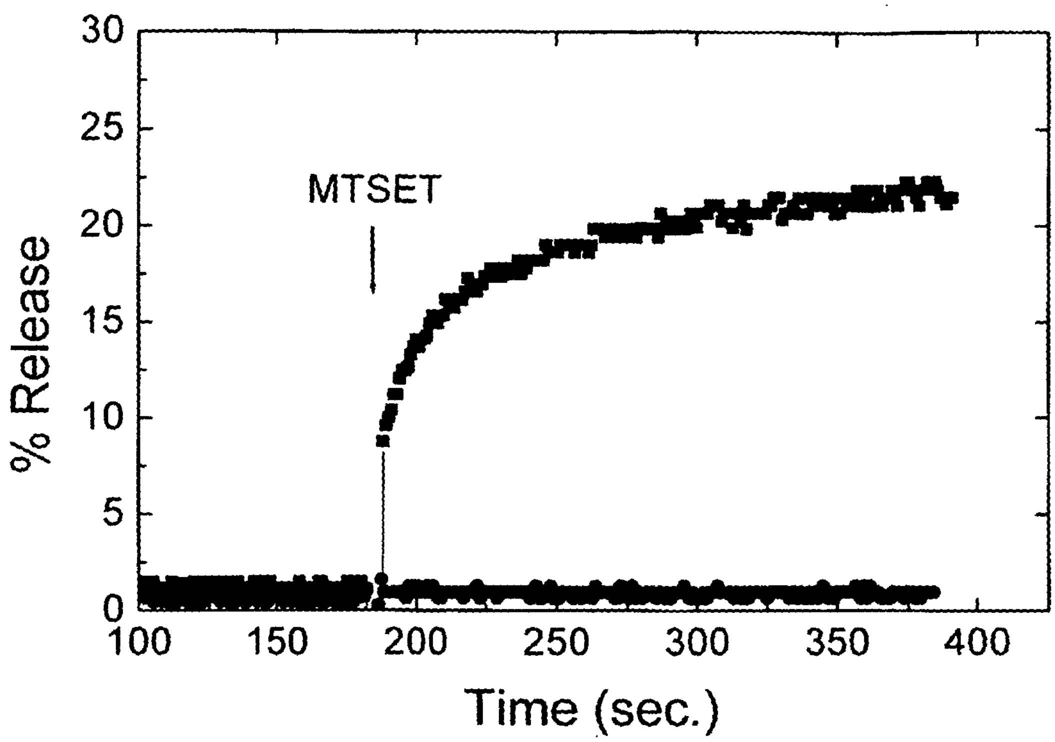

30
25
20
15
10
5
0
% Release
MTSET
100
150
200
250
300
350
400
Time (sec.)


Fig. 12

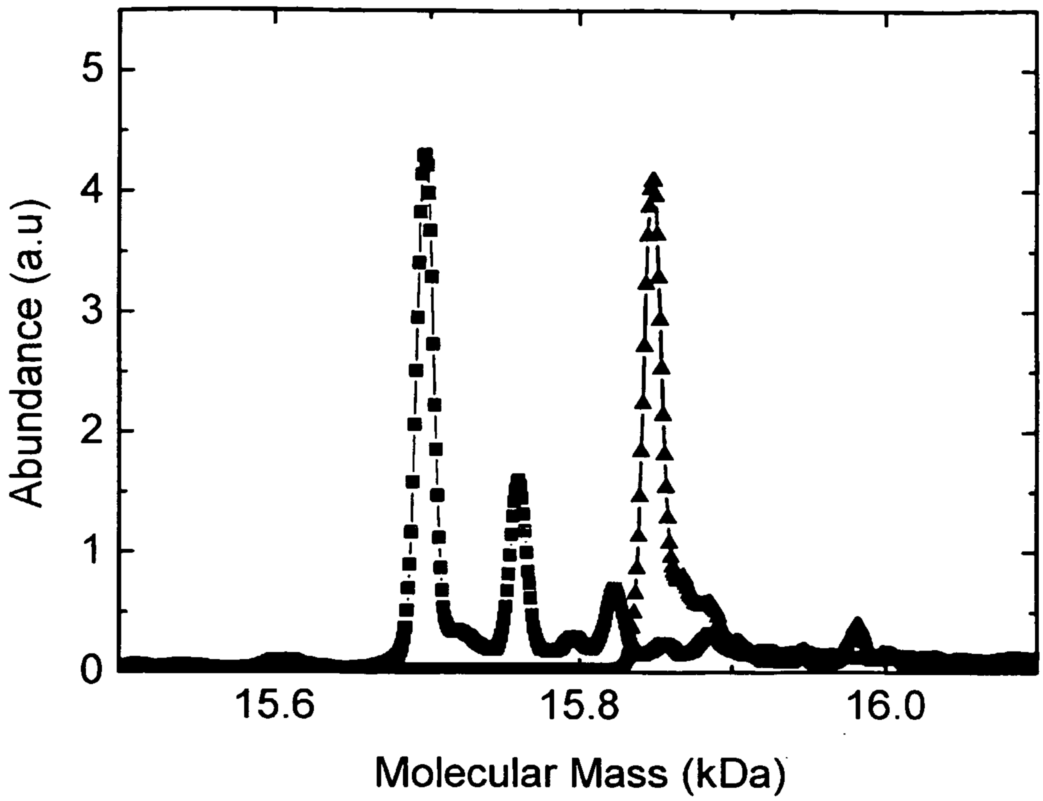


Fig. 13

Treshold tension= 2.33
(MscS-34.3mmHg and MscL= -84 mmHg)

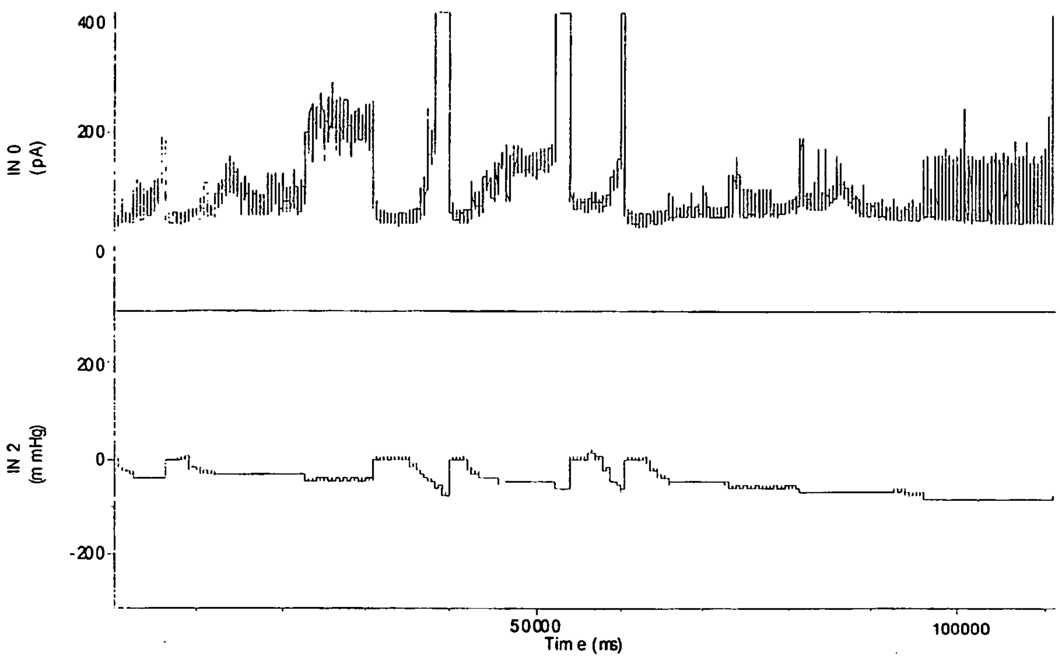


Fig. 14A

Treshold tension = 1.48
(MscS= -201mmHg and MscL= -297mmHg)

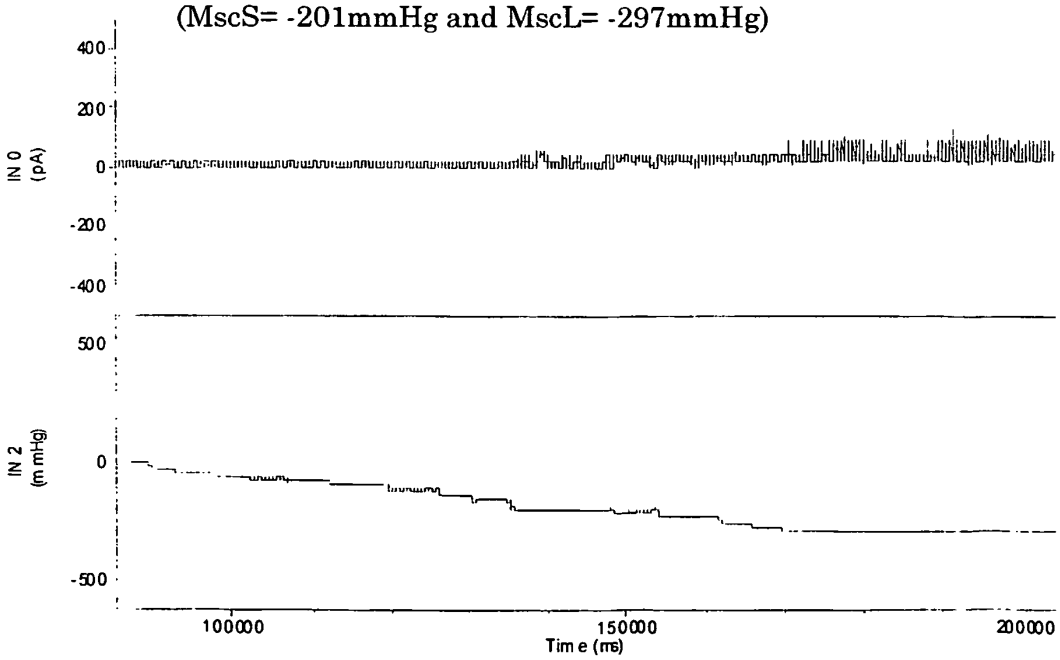


Fig. 14B

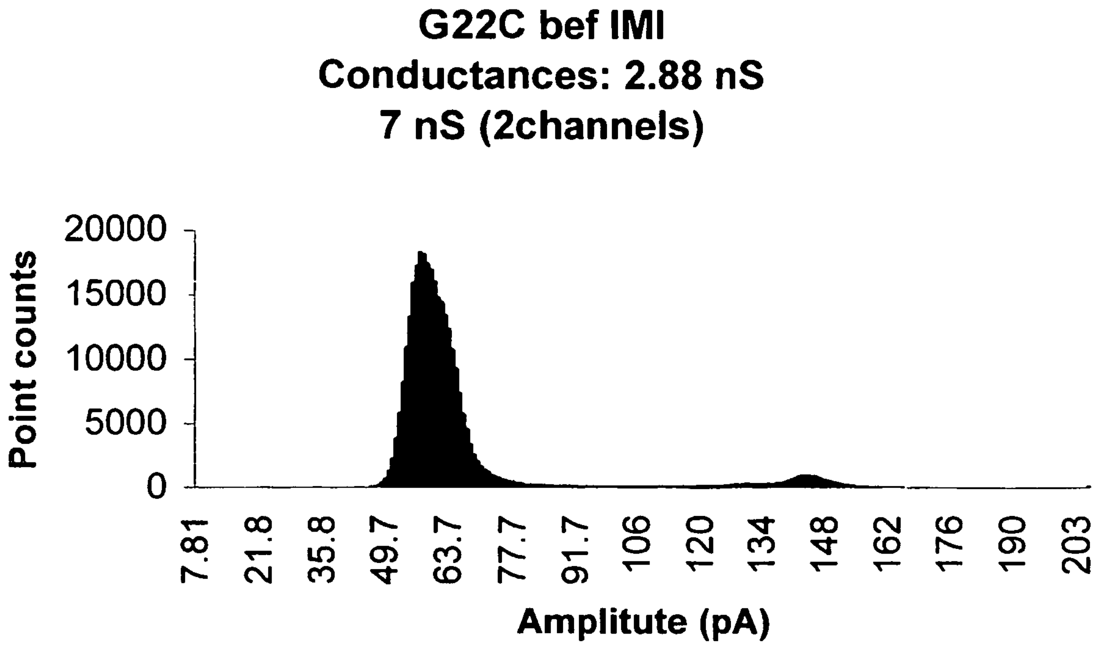


Fig. 14C

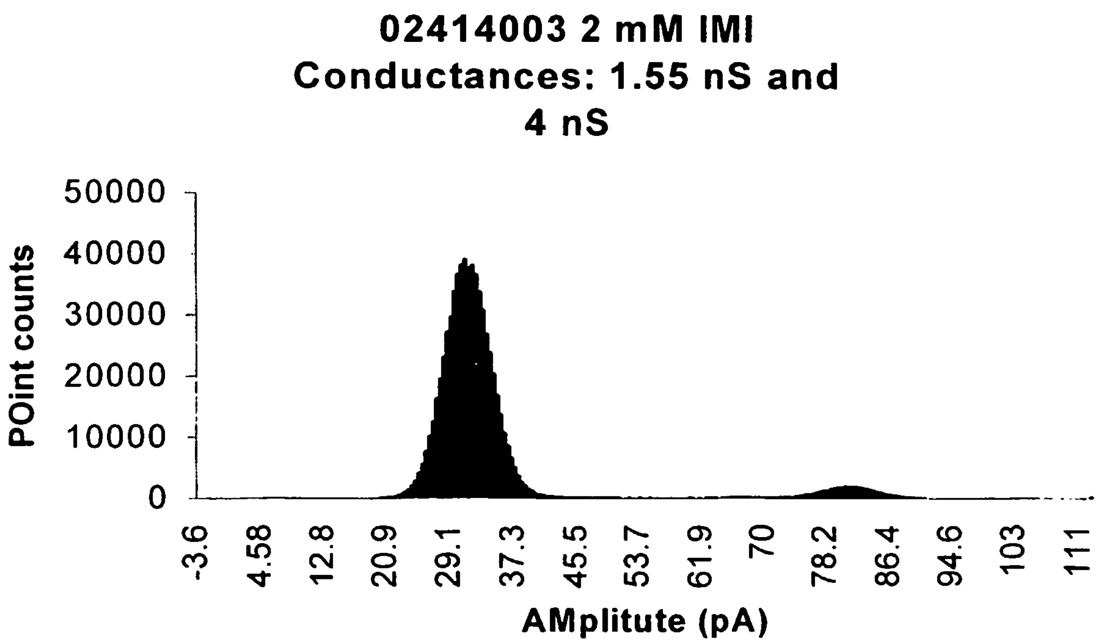


Fig. 14D

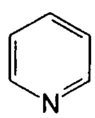

$pK_a$= 5.19

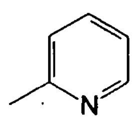

$pK_a$= 5.97

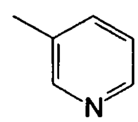

$pK_a$= 5.68

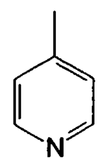

$pK_a$= 6.02

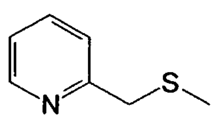

$pK_a$= 5.4

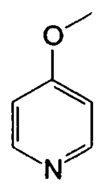

$pK_a$= 6.62

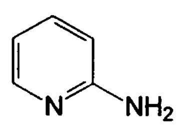

$pK_a$= 6.82

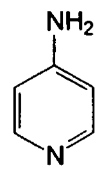

$pK_a$= 9.25

Fig. 15

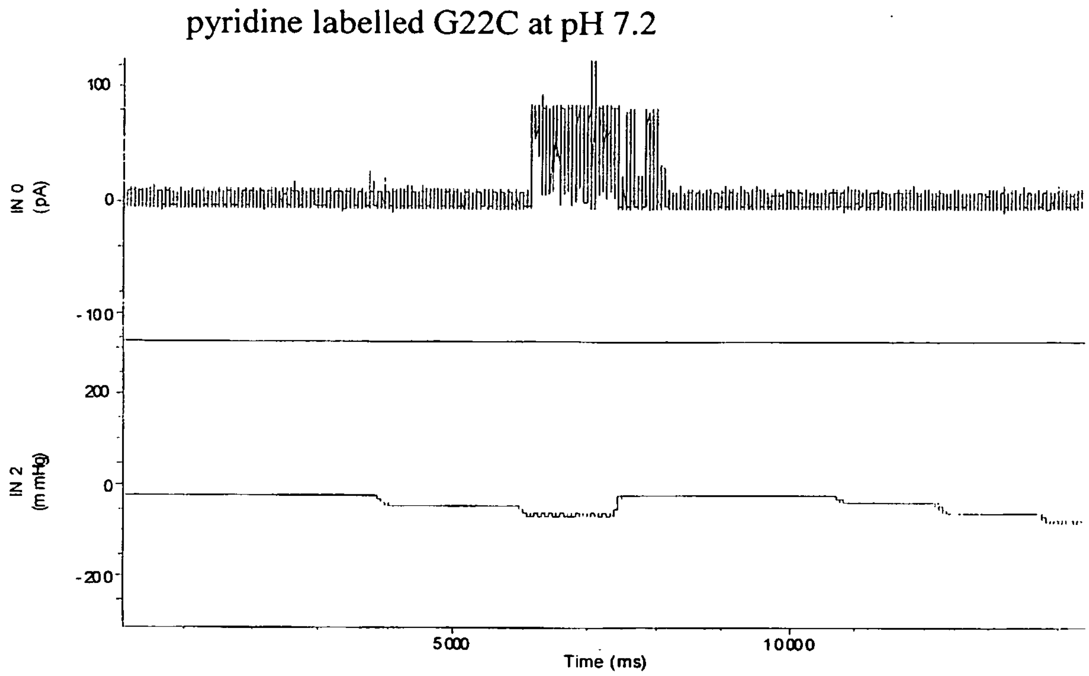


Fig. 16A

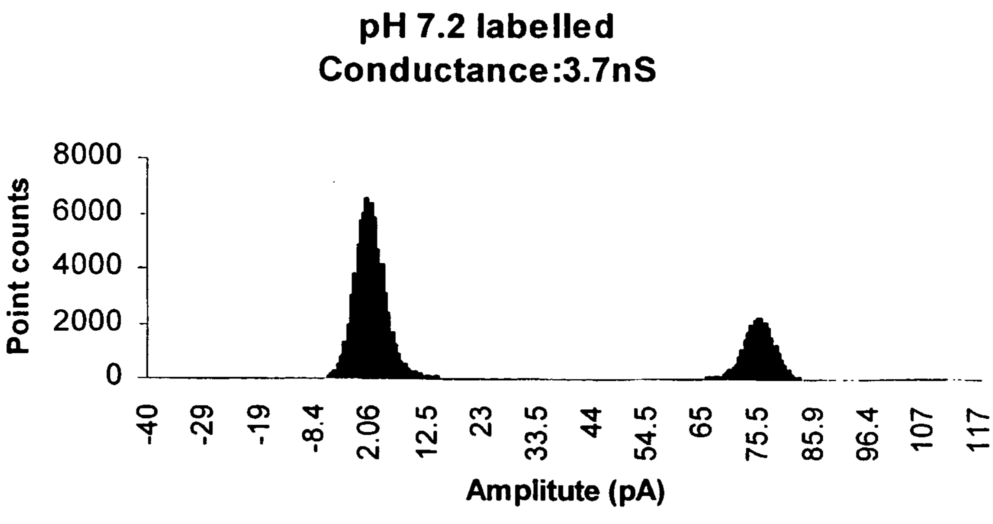


Fig. 16B

Unlabelled G22C, pH 5.2

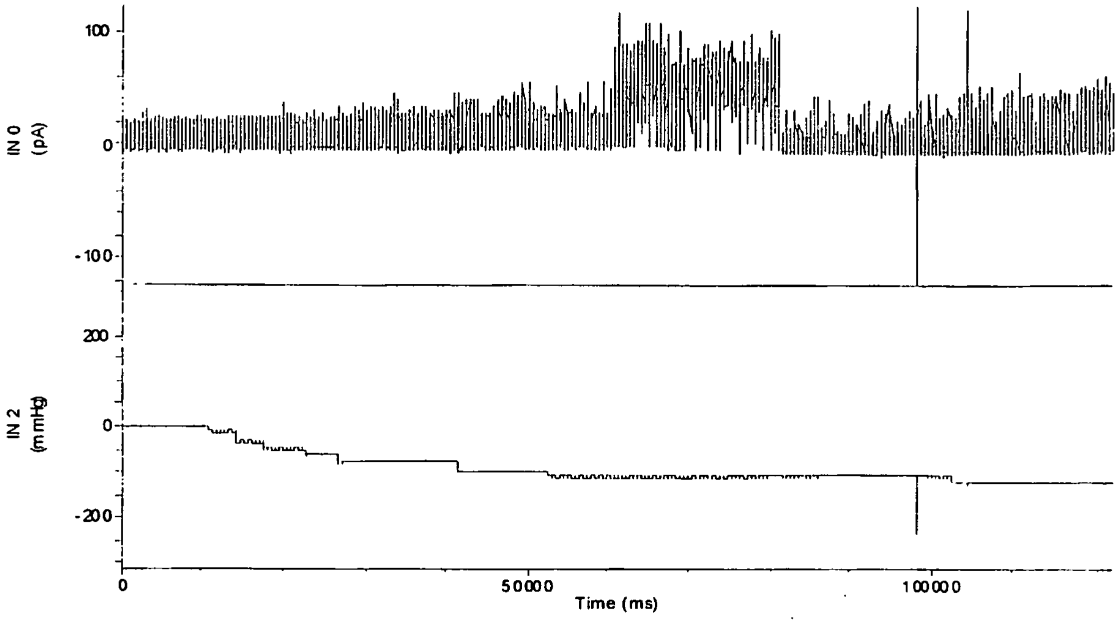


Fig. 16C

BP labelled G22C, pH 5.2

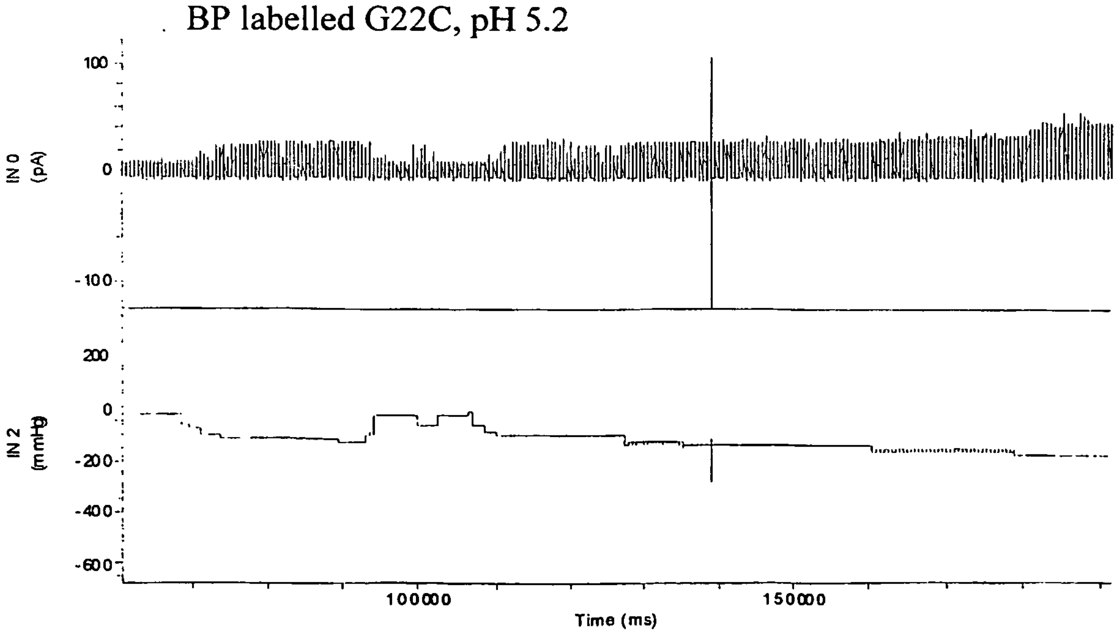


Fig. 16D

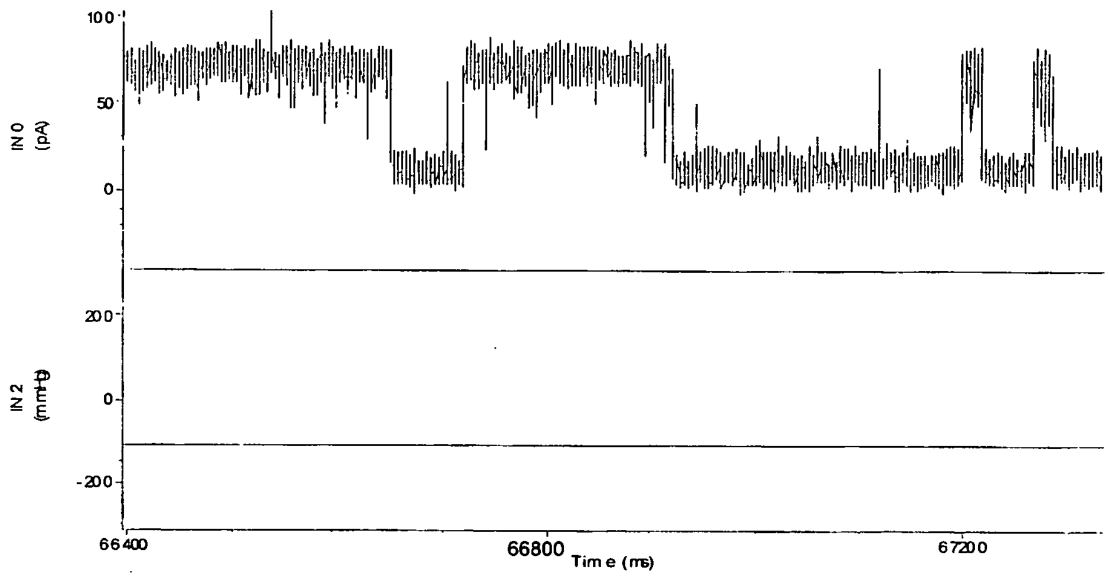


Fig. 16E

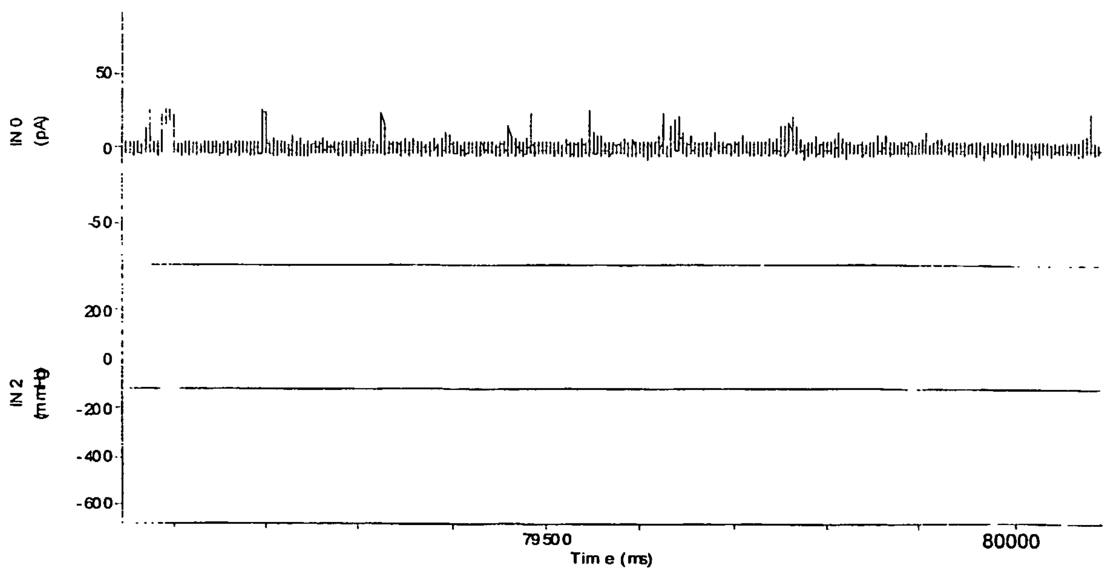


Fig. 16F

**unlabelled pH5.2**
**Conductances:**
**0.5 nS, 3.19 nS**

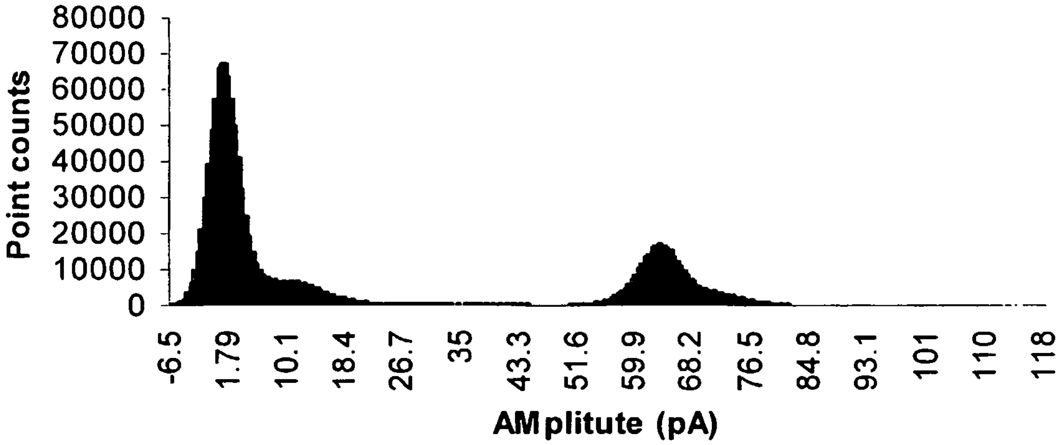


Fig. 16G

**pyridine lab. pH5.2**
**Conductances: 0.4 nS, 1.1 nS**

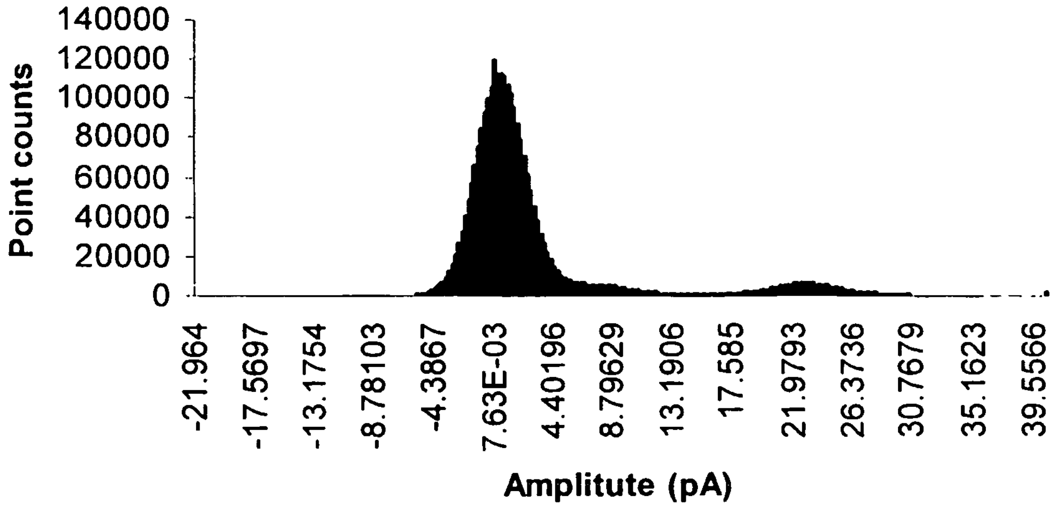


Fig. 16H

Fig. 17

A

B

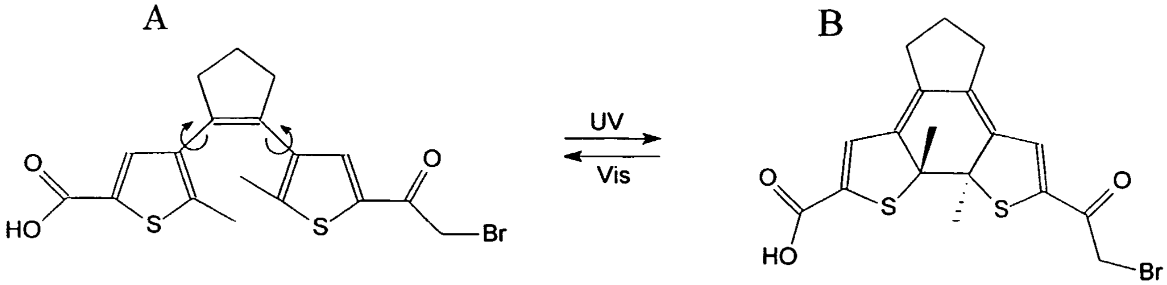

Fig. 18

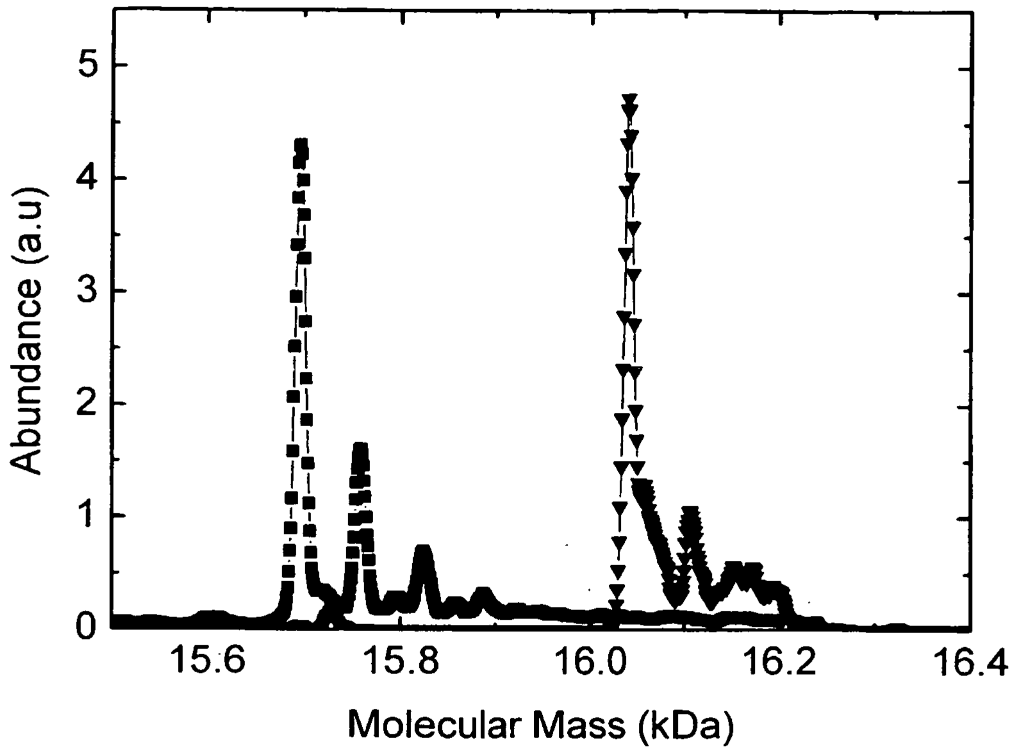

Fig. 19

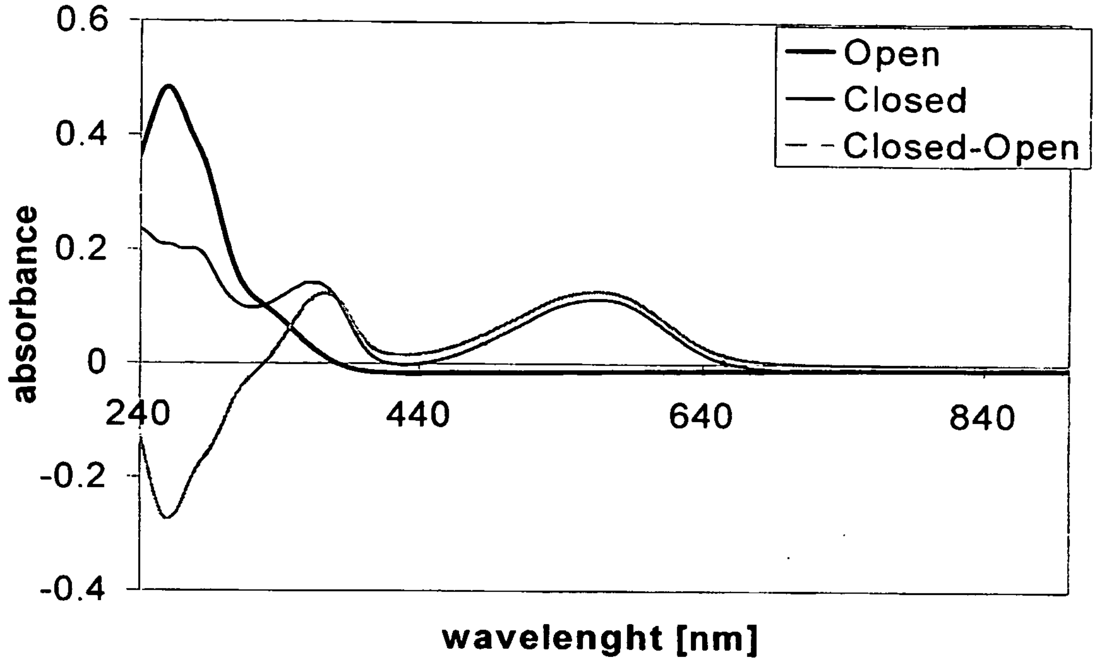
0.6
0.4
0.2
0
-0.2
-0.4
absorbance
240
440
640
840
wavelenght [nm]
Open
Closed
Closed-Open

Fig. 20

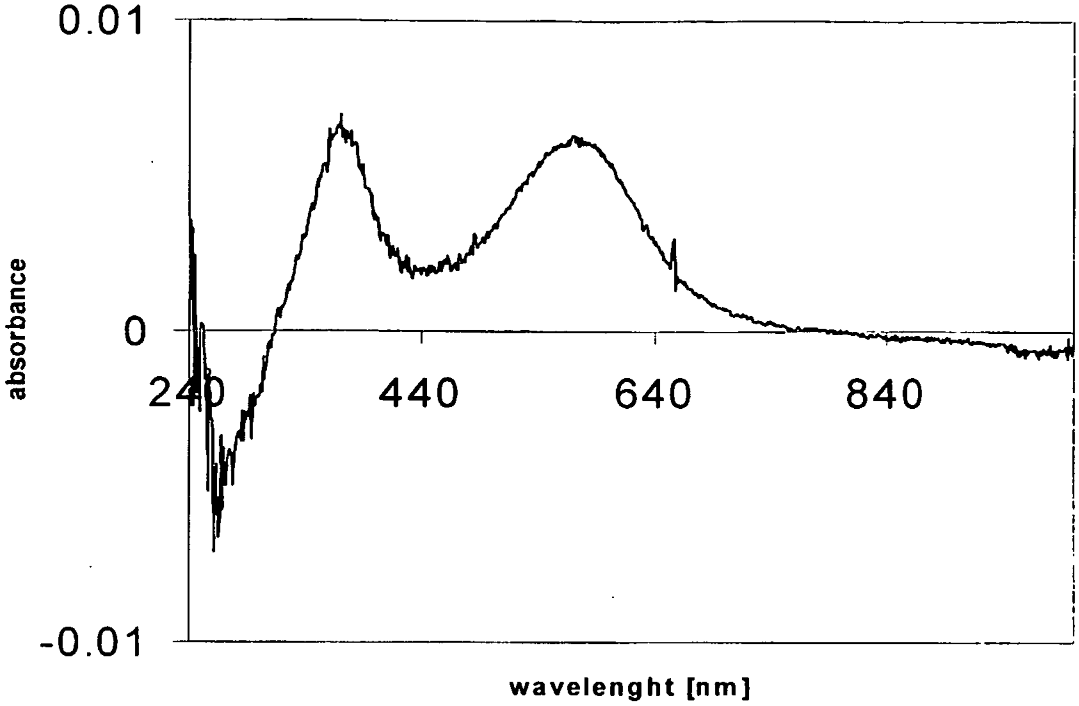

Fig. 21

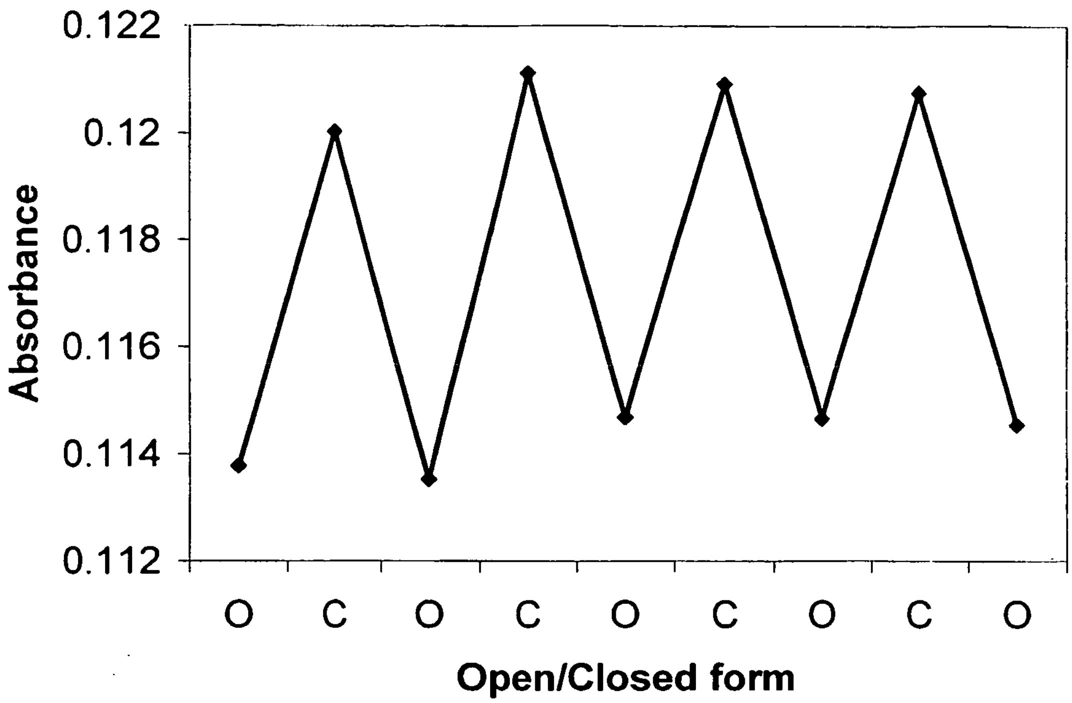
0.122
0.12
0.118
0.116
0.114
0.112
Absorbance
O C O C O C O C O
Open/Closed form

Fig. 22

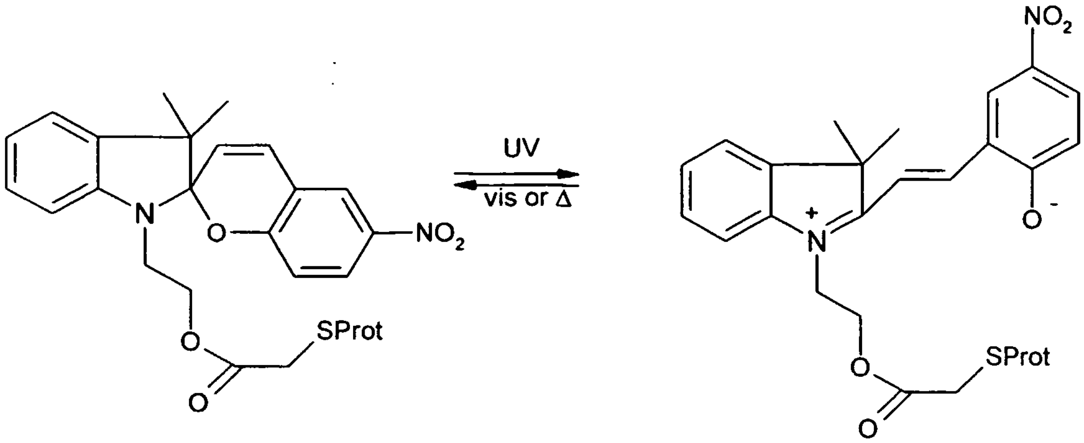

Fig. 23

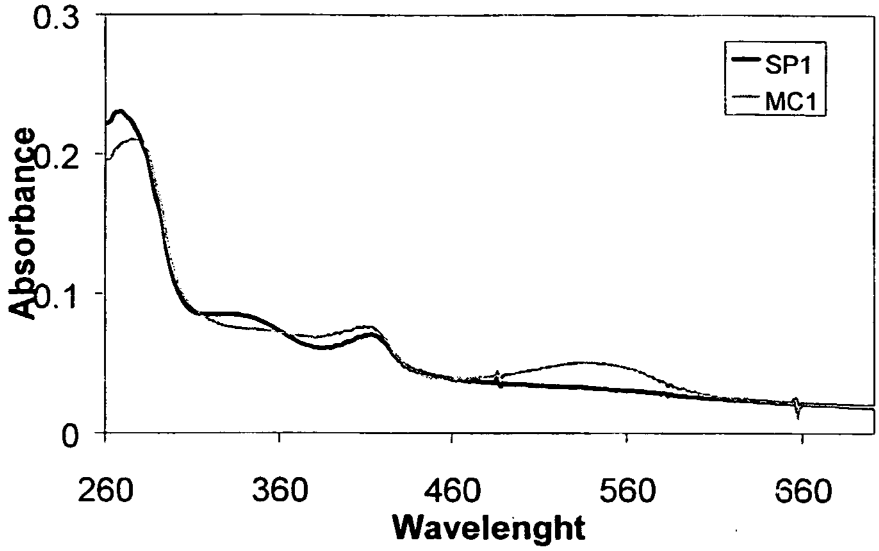

Fig. 24

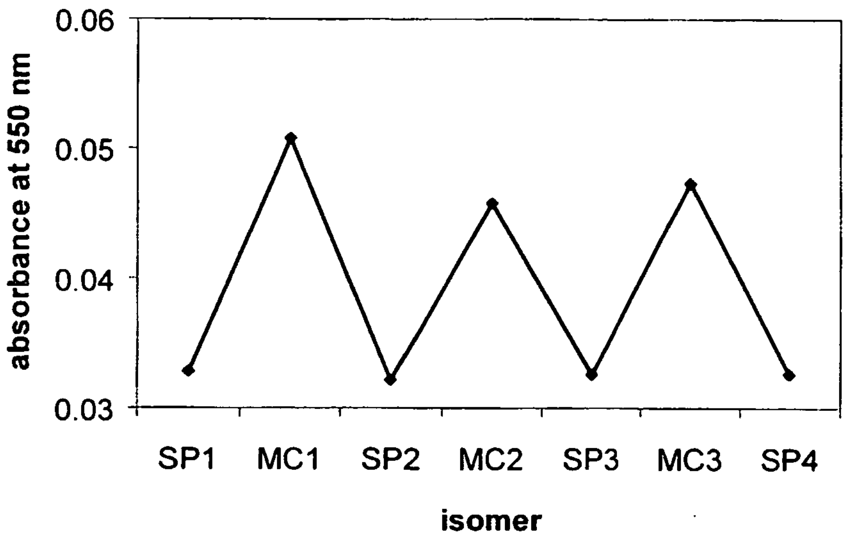
0.06
0.05
0.04
0.03
absorbance at 550 nm
SP1
MC1
SP2
MC2
SP3
MC3
SP4
isomer

Fig. 25

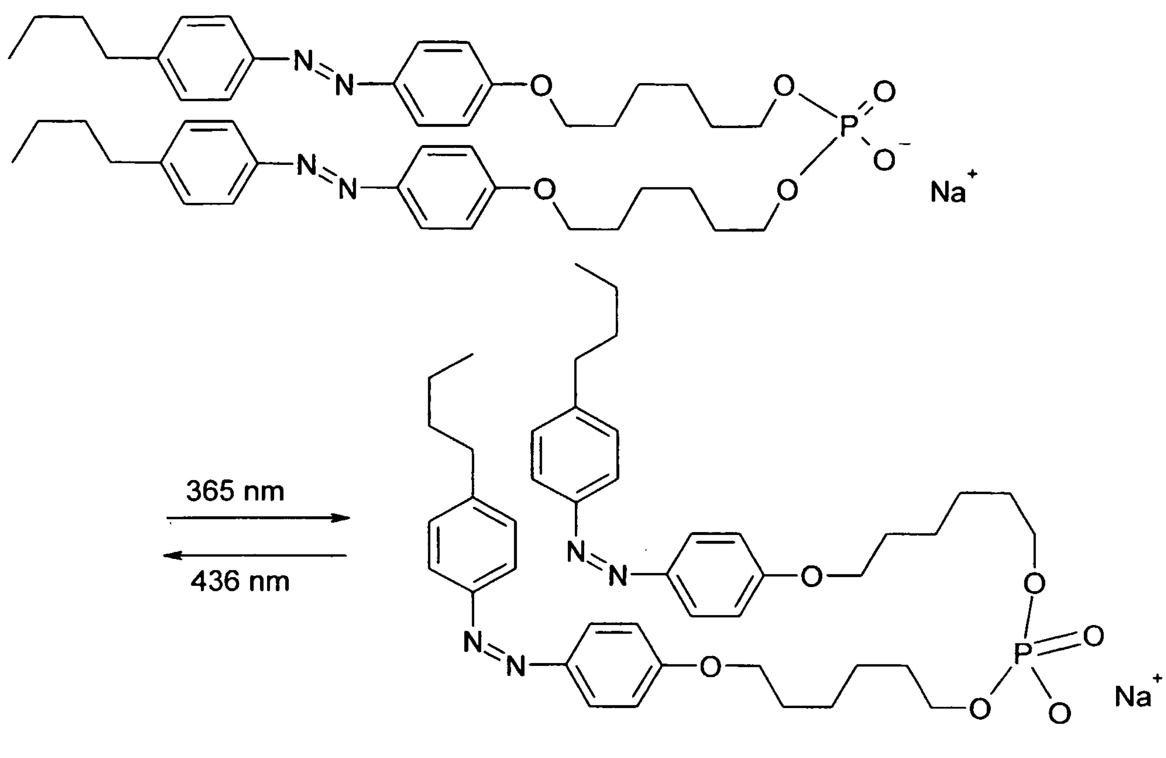

Fig. 26

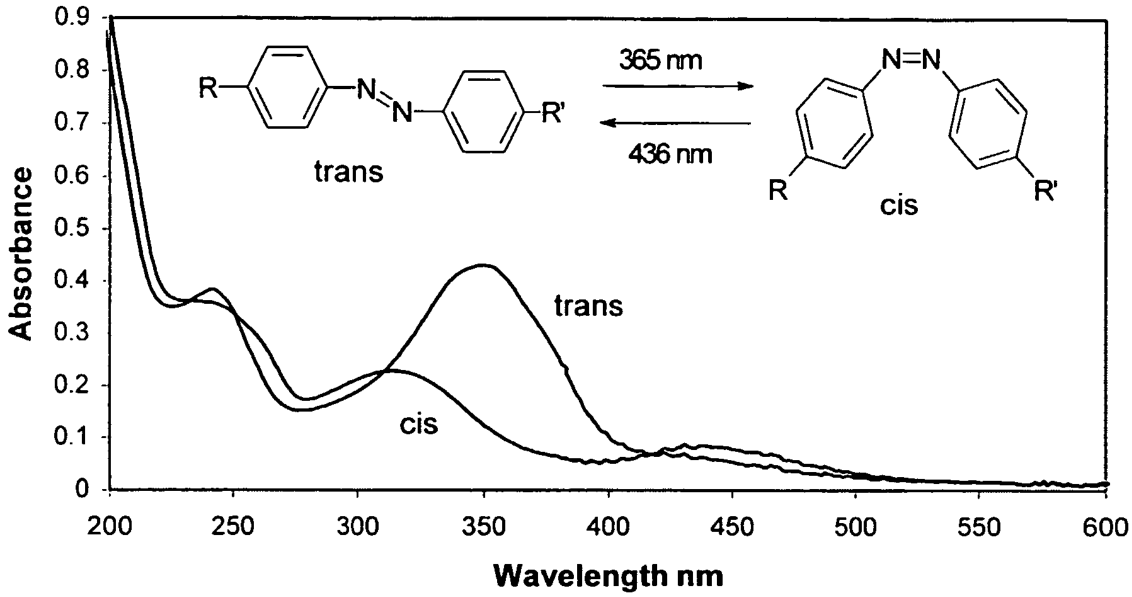
365 nm
436 nm
trans
cis
Absorbance
Wavelength nm
0.9
0.8
0.7
0.6
0.5
0.4
0.3
0.2
0.1
0
200
250
300
350
400
450
500
550
600

Fig. 27

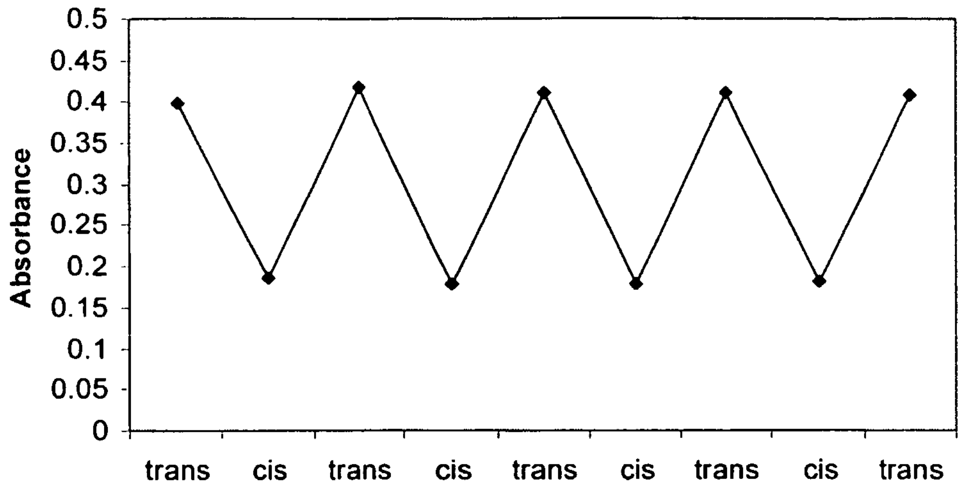
Absorbance
0.5
0.45
0.4
0.35
0.3
0.25
0.2
0.15
0.1
0.05
0
trans
cis
trans
cis
trans
cis
trans
cis
trans

Fig. 28

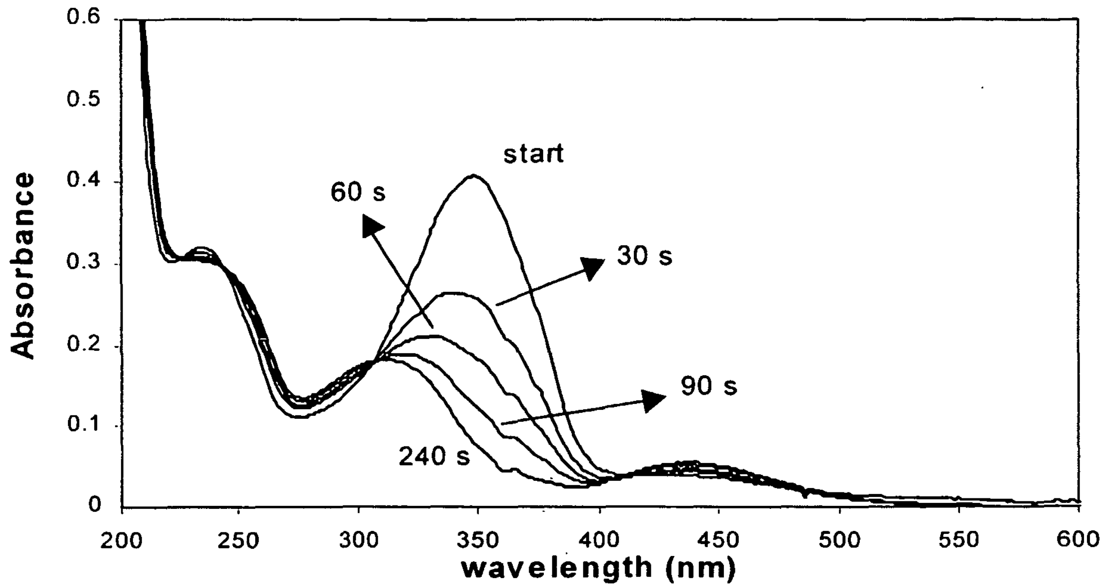
start
60 s
30 s
90 s
240 s
0.6
0.5
0.4
0.3
0.2
0.1
0
Absorbance
200
250
300
350
400
450
500
550
600
wavelength (nm)

Fig. 29

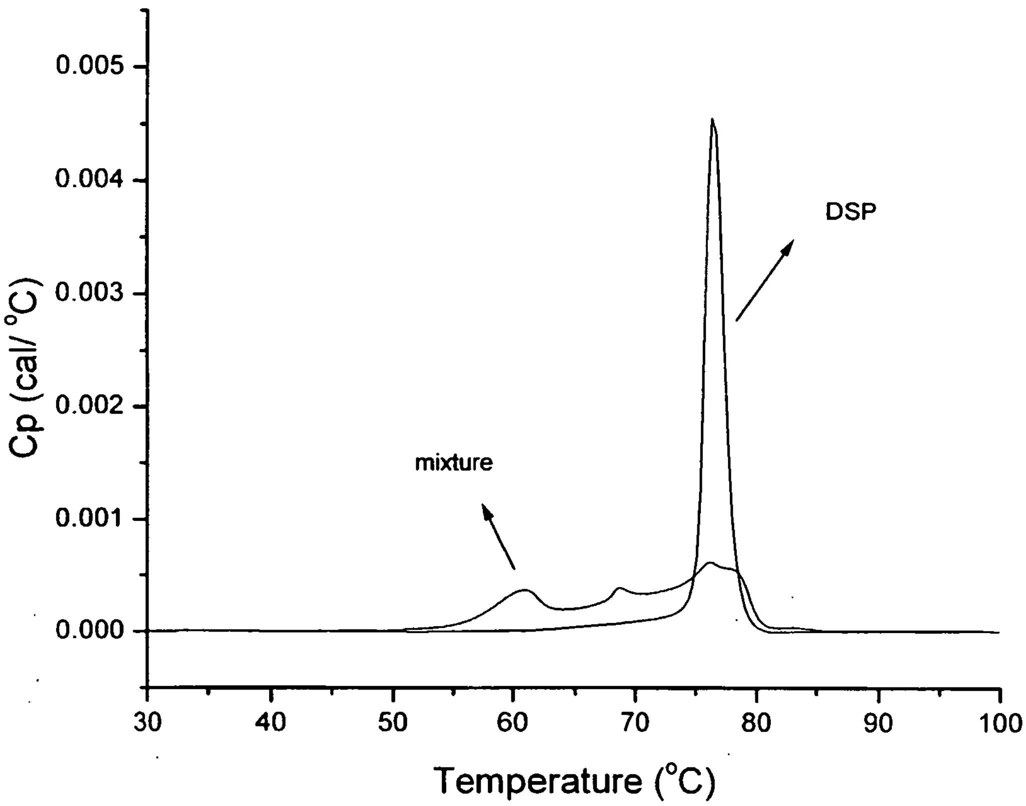
0.005
0.004
0.003
0.002
0.001
0.000
Cp (cal/ °C)
DSP
mixture
30
40
50
60
70
80
90
100
Temperature (°C)

Fig. 30

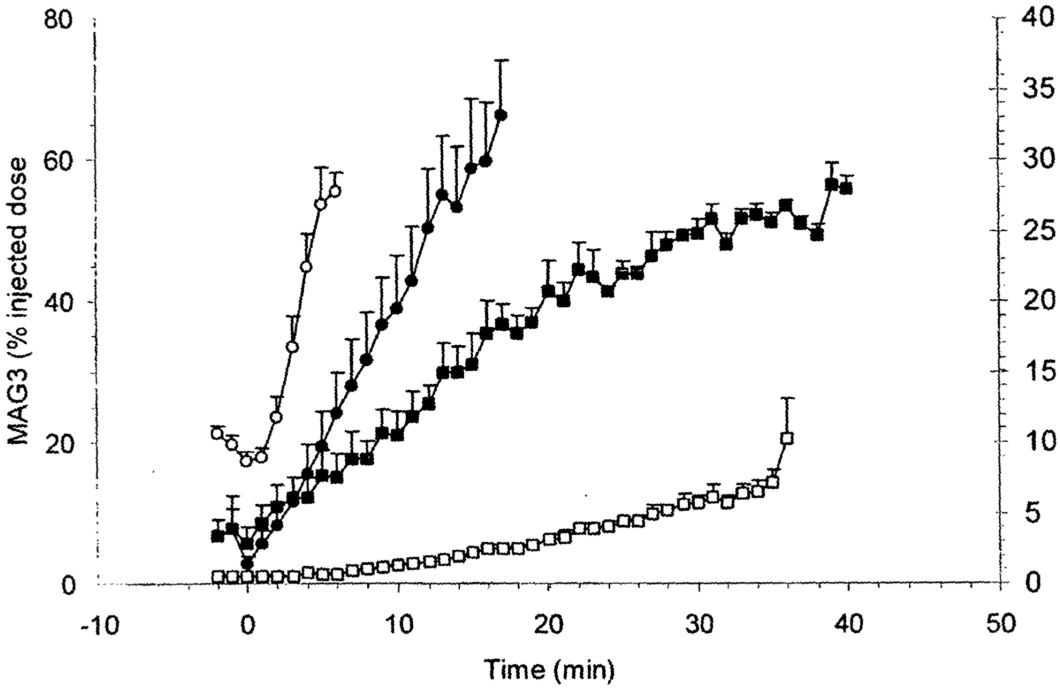
80
60
40
20
0
MAG3 (% injected dose
40
35
30
25
20
15
10
5
0
-10
0
10
20
30
40
50
Time (min)

Fig. 31

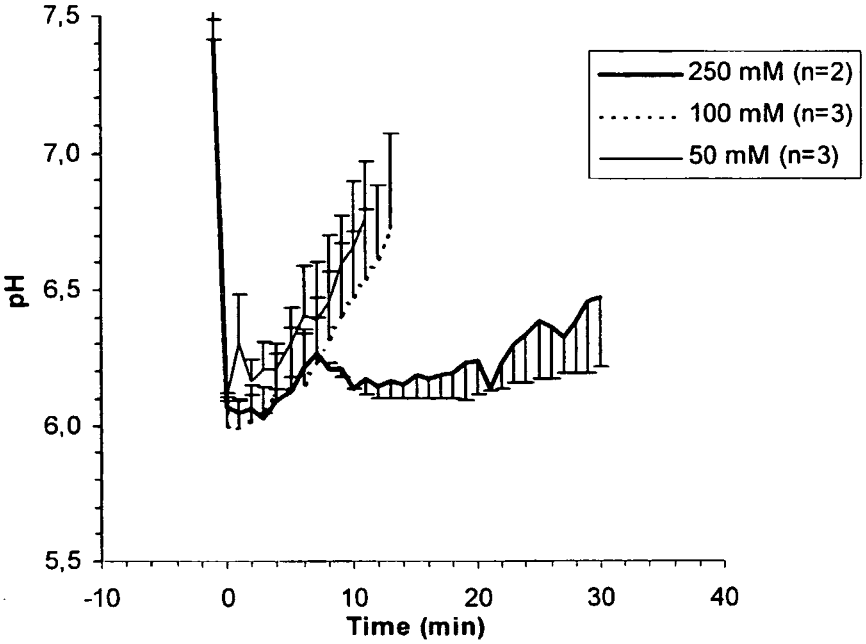
7,5
7,0
6,5
6,0
5,5
pH
-10
0
10
20
30
40
Time (min)
250 mM (n=2)
100 mM (n=3)
50 mM (n=3)

Fig. 32

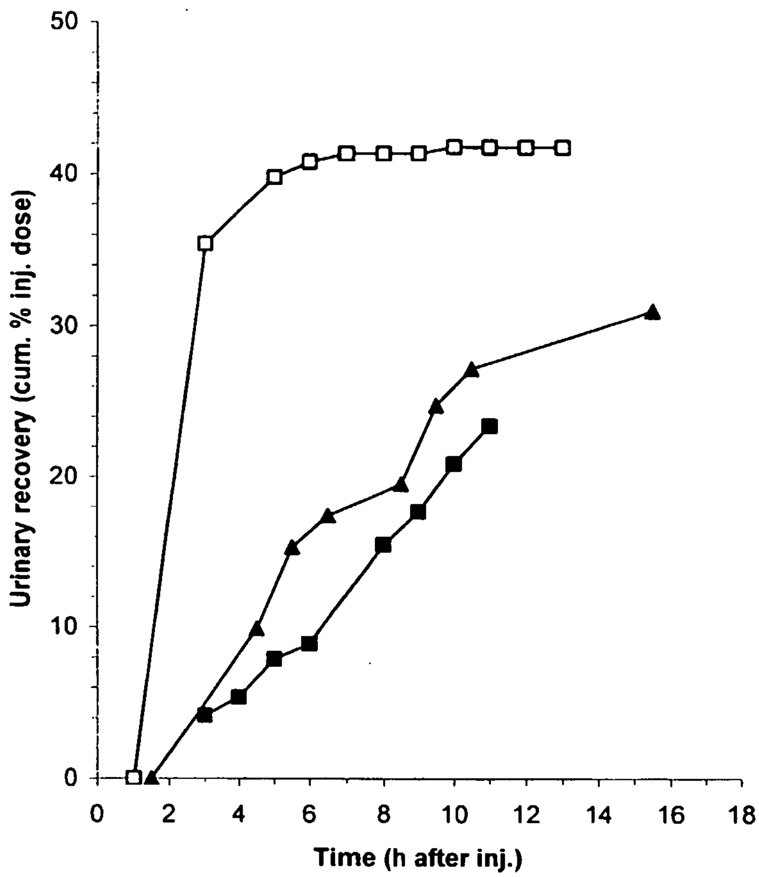
50
40
30
20
10
0
Urinary recovery (cum. % inj. dose)
0
2
4
6
8
10
12
14
16
18
Time (h after inj.)

Fig. 33

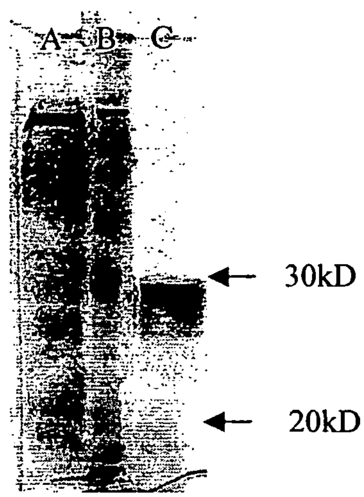

Fig. 34

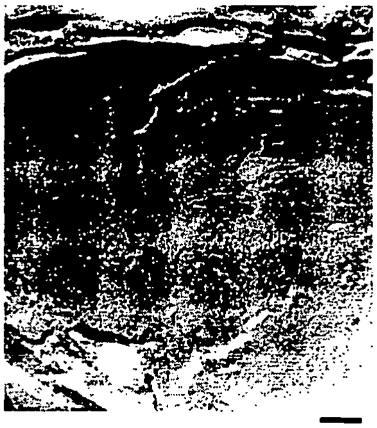


1:50 MscL

Fig. 35

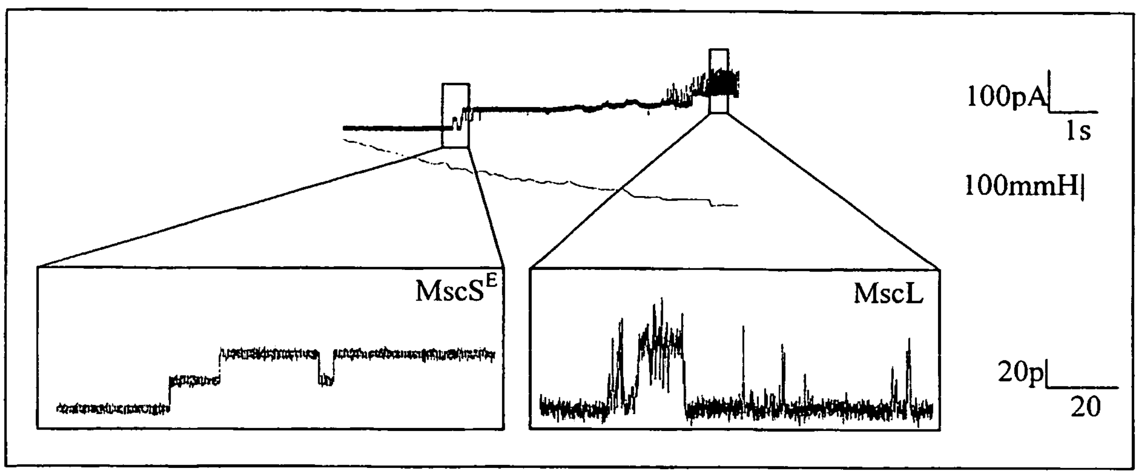

Fig. 36

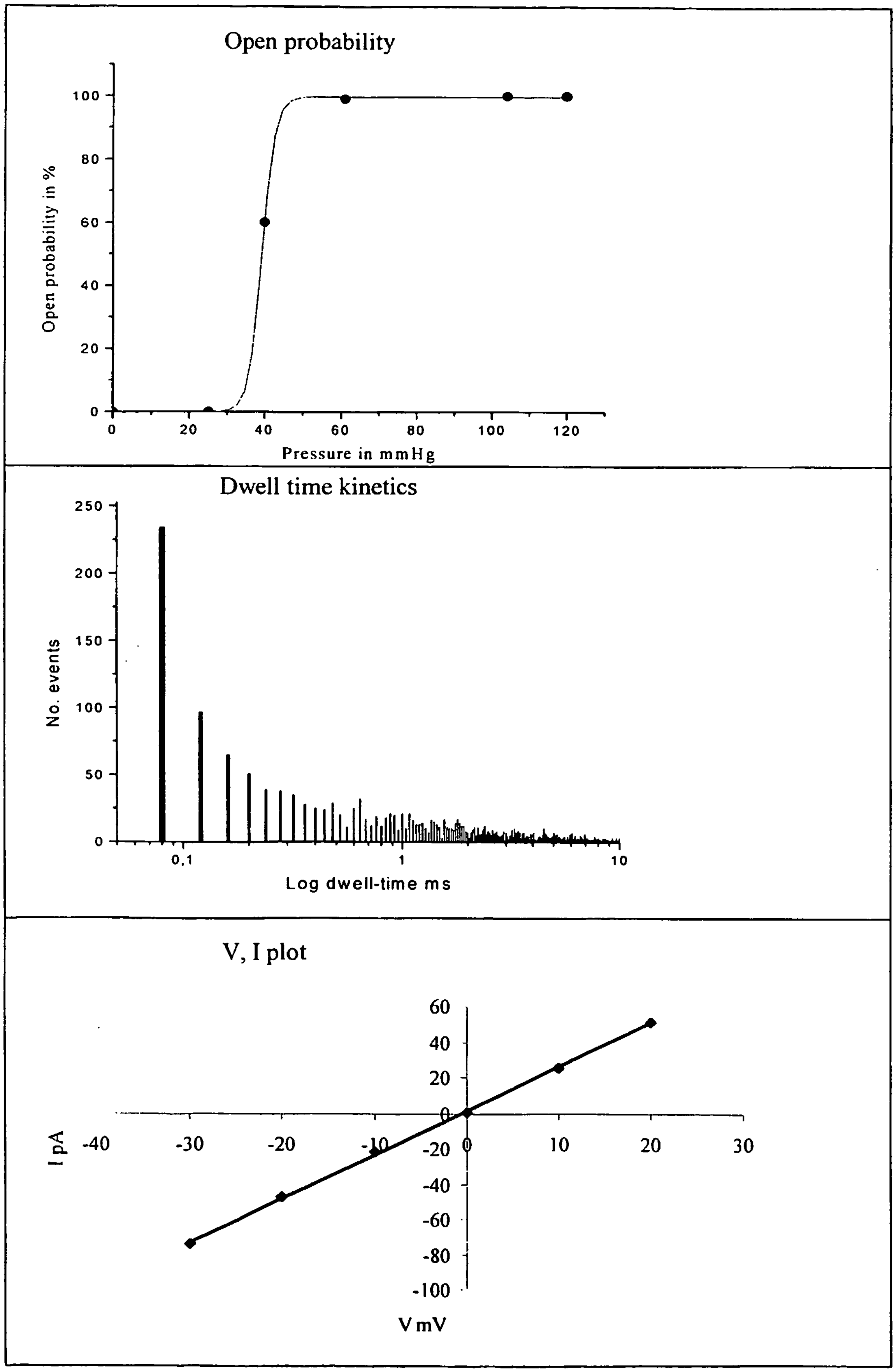
Open probability
Open probability in %
0
20
40
60
80
100
0
20
40
60
80
100
120
Pressure in mmHg
Dwell time kinetics
No. events
0
50
100
150
200
250
0,1
1
10
Log dwell-time ms
V, I plot
I pA
60
40
20
0
-20
-40
-60
-80
-100
-40
-30
-20
-10
0
10
20
30
V mV

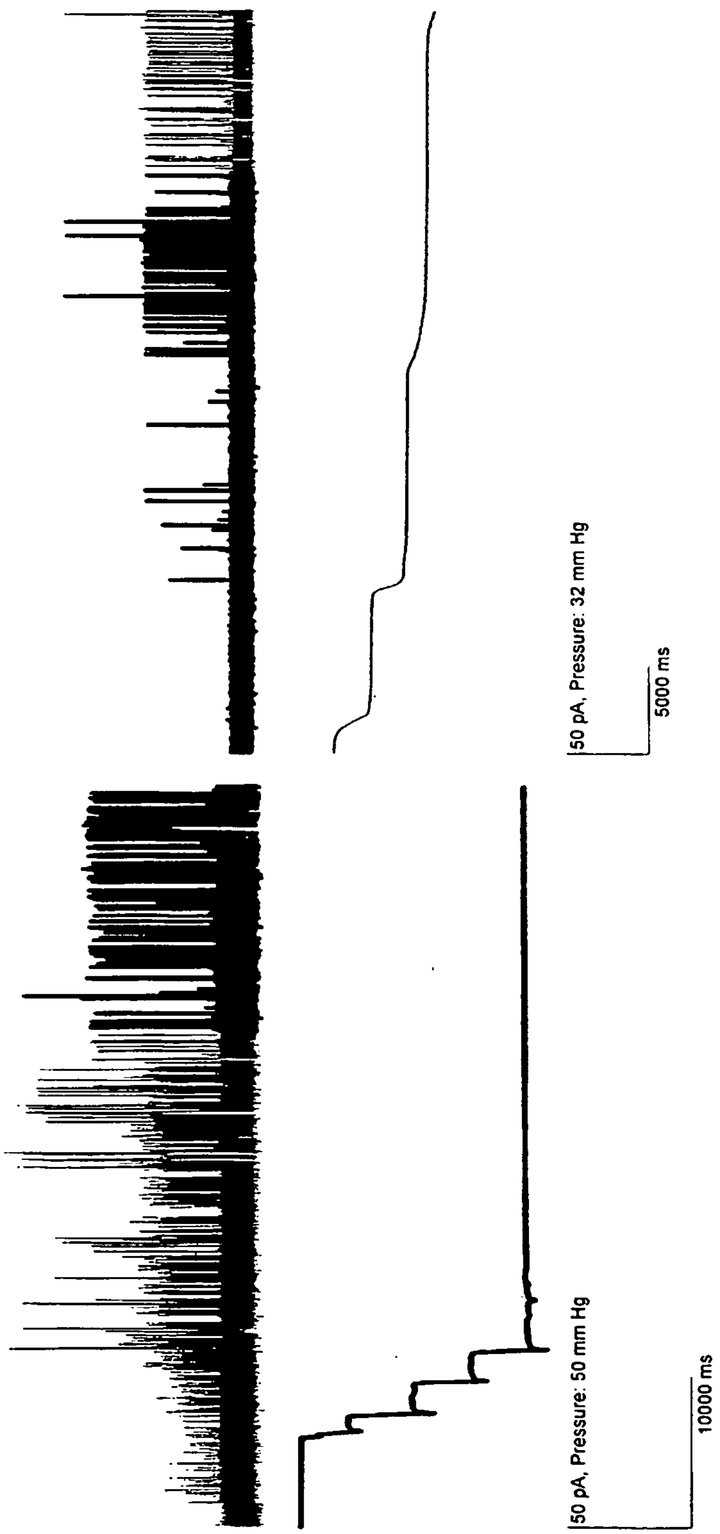


Fig. 37

Fig. 38

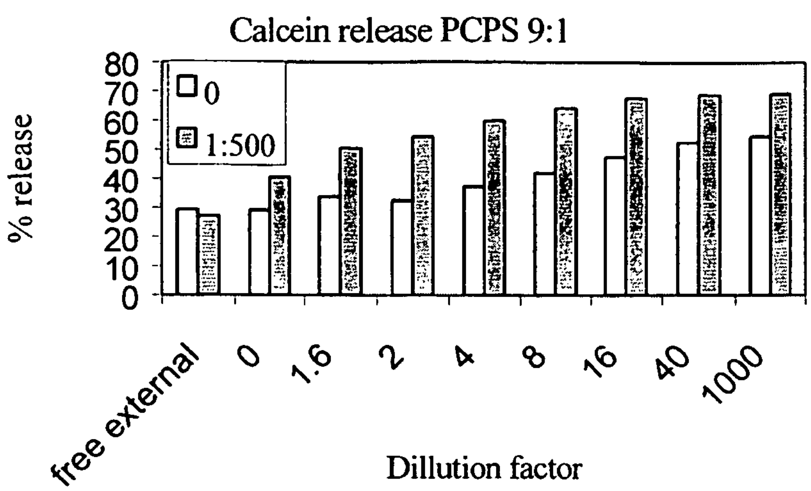
Calcein release PCPS 9:1
% release
80
70
60
50
40
30
20
10
0
0
1:500
free external
0
1.6
2
4
8
16
40
1000
Dillution factor

Fig. 39

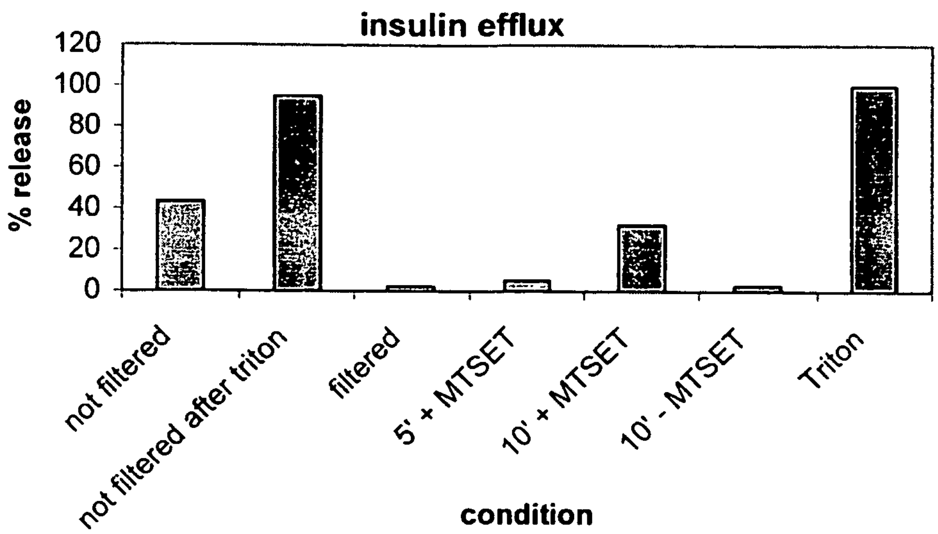
insulin efflux
120
100
80
60
40
20
0
% release
not filtered
not filtered after triton
filtered
5' + MTSET
10' + MTSET
10' - MTSET
Triton
condition

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9920252 A
- US 5820879 A

### Non-patent literature cited in the description


- **Shi.** *J Contr. Release,* 2002, vol. 80, 309
- **Drummond.** *Biochem. Biophys,* 2000, vol. 1463, 383
- **Coakley.** *J. Pancreas,* 2001, vol. 2, 294
- **Straubinger.** *Methods Enzymol,* 1993, vol. 221, 361
- **Friend.** *Aliment Pharmacol Ther,* 1998, vol. 12, 591
- **Blount, P. et al.** *Methods Enzymol.,* 1999, vol. 294, 458-482
- **Bixler, R.L. ; Niemann, C.** *J. Org. Chem.,* 1958, vol. 23, 575
- **Yankeelov, J.A. ; Jolley, C.J.** *Biochemistry,* 1972, vol. 11, 159
- **Maat, L. ; Beyerman, H.C. ; Noordam, A.** *Tetrahedron,* 1979, vol. 35, 273
- **Sakuragi, M. ; Aoki, K. ; Tamaki, T. ; Ichimura, K.** *Bull. Chem. Soc. Jpn,* 1990, vol. 63, 74
- **Lucas, L.N. ; Esch, J. ; Kellog, R.M. ; Feringa, B.L.** *Chem. Commun,* 1998, 2313
- **W. Schaffner ; C. Weissmann.** A rapid, sensitive, and specific method for the determination of protein in dilute solution. *Anal.Biochem.,* 1973, vol. 56 (2), 502-514
- **R. H. Friesen ; J. Knol ; B. Poolman.** Quaternary structure of the lactose transport protein of Streptococcus thermophilus in the detergent-solubilized and membrane-reconstituted state. *J.Biol.Chem.,* 2000, vol. 275 (43), 33527-33535
- **P. Blount ; S. I. Sukharev ; P. C. Moe ; B. Martinac ; C. Kung.** Mechanosensitive channels of bacteria. *Methods Enzymol.,* 1999, vol. 294, 458-482
- **Arkin, I.T. ; Sukharev, S.I. ; Blount, P. ; Kung, C. ; Brünger A.T.** *Biochim. Biophys. Acta,* 1998, vol. 1369, 131-140
- **Delcour, A.H. ; Martinac, B. ; Adler, J. ; Kung, C.** *Biophys. J.,* 1999, vol. 56, 631-636
- **Blount, P. ; Sukharev, S.I. ; Moe, P.C. ; Schroeder, M.J. ; Guy, H.R. ; Kung, C.** *EMBO J.,* 1996, vol. 15, 4798-4805
- **Hirel, A.U. ; Schmitter, M.J. ; Dessen, P. ; Fayat, G. ; Blanquet, S.** *Proc. Natl. Acad. Sci. U. S.A.,* 1989, vol. 86, 8247-8251
- **Knol, J., Sjollema ; Poolman, B.** *Biochemistry,* 1998, vol. 37, 16410-16415
- **Friesen, R.H.E. ; Knol, J. ; Poolman, B.** *J. Biol. Chem.,* 2000, vol. 43, 33527-33535
- **Moe, P.C. ; Blount, P. ; Kung, C.** *Mol. Microbiol.,* 1998, vol. 28, 583-592
- **Yoshimura, K. ; Batiza, A. ; Schroeder, M. ; Blount P. ; Kung, C.** *Biophys. J.,* 1999, vol. 77, 1960-1972
- **Song, X. ; Perlstein, J. ; Whitten, D.G.** *J. Am. Chem. Soc.,* 1997, vol. 119, 9144-9159
- **Blount, P. ; Sukharev, S.I. ; Schroeder, M.J. ; Nagle, S.K. ; Kung, C.** *Proc. Natl. Acad. Sci USA,* 1996, vol. 93, 11652-11657
- **Mayer, L.D. ; Hope, M.J. ; Cullis, P.R.** *Biochim. Biophys. Acta,* 1986, vol. 858, 161-168
- **Martinac, B. ; Adler, J. ; Kung, C.** *Nature,* 1990, vol. 348, 261-263
- **Ou, X. ; Blount, P. ; Hoffman, R.J. ; Kung C.** *Proc. Natl. Acad. Sci. USA,* 1998, vol. 95, 11471-11475
- **Damen J. ; Regts, J. ; Scherphof G.** *Biochim. Bioph. Acta,* 1981, vol. 665, 538-545
- **Yoshimura, K, Batiza ; Kung, C.** *Biophysical Journal,* 2001, 2198-2206